# EUROPEAN PATENT APPLICATION

(11) **EP 4 406 962 A1**
(43) Date of publication of application: **31.07.2024**
(21) Application number: 23834811.4
(22) Date of filing: 03.07.2023
(51) Int. Cl.: C07K 5/027, C07D 491/20, A61K 31/407, A61P 31/14, C07K 5/097

(54) **MACROCYCLIC PEPTIDOMIMETIC PROTEASE INHIBITOR AND USE THEREOF**

(30) Priority: 08.07.2022 CN 202210806189; 26.06.2023 CN 202310765969
(71) Applicant: Tencent Technology (Shenzhen) Company Limited, Shenzhen, Guangdong, 518057 (CN)
(72) Inventor: YU, Yang, Shenzhen, Guangdong 518057 (CN); XUE, Ding, Shenzhen, Guangdong 518057 (CN); LIU, Wei, Shenzhen, Guangdong 518057 (CN)
(74) Representative: Nederlandsch Octrooibureau
(86) International application number: PCT/CN2023/105562
(87) International publication number: WO 2024/008044

(57) **Abstract**

The present disclosure provides a compound. The compound is a compound shown in formula (I) or a stereoisomer, a tautomer, a nitrogen oxide, a solvate, a metabolite, a pharmaceutically acceptable salt, or a prodrug of the compound shown in formula (I). The compound shown in formula (I) can significantly inhibit M^{Pro} or inhibit coronavirus replication, and has excellent metabolic stability.

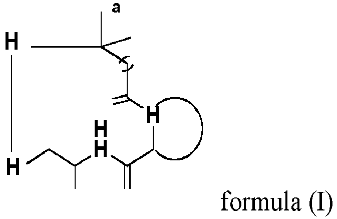

## Description

### FIELD OF THE TECHNOLOGY

The present disclosure relates to the field of medicine, and specifically, to a macrocyclic peptidomimetic protease inhibitor and use thereof.

### BACKGROUND OF THE DISCLOSURE

Coronavirus pneumonia (Covid-19), caused by the novel coronavirus SARS-Cov-2, leads to a global pandemic in year 2020, promoting scientists all over the world to actively participate in the development of drugs against the novel coronavirus. Small-molecule drugs are very promising in the treatment of the novel coronavirus because these drugs can be taken orally and are less affected by variants, with advantages in price and production capacity. At present, in addition to three international approved small-molecule drugs for the novel coronavirus: Merck's Molnupiravir based on an inhibitor targeting RdRp, Pfizer's Paxlovid, and Gilead's Remdesivir, Paxlovid in China has also been approved to be covered by national medical insurance. However, Nirmatrelvir, which has good efficacy, still needs to be combined with the CYP enzyme inhibitor Ritonavir, which still has a great risk on patients with other underlying diseases or even cannot be applied there-to.

Therefore, it is necessary to develop a medicine for monotherapy.

### SUMMARY

The present disclosure is made based on findings and knowledge of the following facts and issues:

Pfizer's molecule PF-07321332 is metabolized quickly in the body, and therefore Ritonavir is required to prolong the action of the molecule in the body. The macrocyclic peptidomimetic molecule provided in this application is a good solution to the problem of metabolic stability, and also has very good activity, which is very promising for future treatment of novel coronavirus pneumonia.

According to a first aspect of the present disclosure, the present disclosure provides a compound, being a compound shown in formula (I) or a stereoisomer, a tautomer, a nitrogen oxide, a solvate, a metabolite, a pharmaceutically acceptable salt, or a prodrug of the compound shown in formula (I), 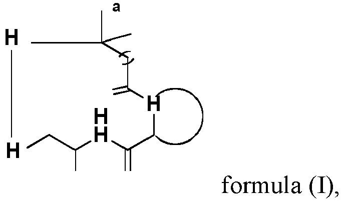
L₁ being a linker;
L₂ being -(CH₂)ₘCONR₁- or 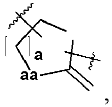 Y being C or N;
R₁ being H, F, Cl, Br, CN, NO₂, -OR^{b}, -NR^{c}R^{d}, or C₁₋₆ alkyl;
R₂ₐ and R_{2b} being each independently H, -C(=O)R^{a}, -C(=O)OR^{b}, -S(=O)₂R^{b}, - C(=O)NR^{c}R^{d}, -NC(=O)R^{c}R^{d}, -OR^{b}, -NR^{c}R^{d}, R^{b}0-C₁₋₄ alkylene, R^{d}R^{c}N-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, and (heteroaryl consisting of 5-10 atoms)-C₁₋₄ alkylene being each independently unsubstituted or substituted with one, two, three, or four substituents, the substituent being independently F, Cl, Br, CN, =O, -OR^{b}, -NR^{c}R^{d}, C₁₋₆ alkyl, C₁₋₆ haloalkyl, R^{b}0-C₁₋₄ alkylene, or R^{d}R^{c}N-C₁₋₄ alkylene, and R₂ₐ and R_{2b} being not all H;
ring A being optionally heterocyclyl consisting of 3-10 atoms or heteroaryl consisting of 5-10 atoms that is substituted with one or more R';
Z being cyano, aziridine, epoxypropane, trifluoromethyl ketone, aldehyde, - CH(O(C=O)R₃)SO₃Na, -CH(O(C=O)OR₃)SO₃Na, -CH(O(C=O)NHR₃)SO₃Na, - (C=O)(C=O)NHR₃, -(C=O)(P=O)(OR₃), -(C=O)COOR₃, acetoxymethyl ketone, Michael receptor, or substituted or unsubstituted heterocyclyl consisting of 3-6 atoms or heteroaryl consisting of 5-10 atoms, R₃ being H, F, Cl, Br, CN, NO₂, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene;
m being an integer between 1 and 6; n being 1 or 2; k being 0 or 1;
R' being independently H, F, Cl, Br, CN, NO₂, =O, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, heterocyclyl consisting of 3-12 atoms, C₆₋₁₀ aryl, or heteroaryl consisting of 5-10 atoms, C₃₋₈ cycloalkyl, heterocyclyl consisting of 3-12 atoms, C₆₋₁₀ aryl, or heteroaryl consisting of 5-10 atoms being independently unsubstituted or substituted with one, two, three, or four substituents, and the substituent being independently F, Cl, Br, CN, =O, -OR^{b}, -NR^{c}R^{d}, C₁₋₆ alkyl, C₁₋₆ haloalkyl, R^{b}0-C₁₋₄ alkylene, or R^{d}R^{c}N-C₁₋₄ alkylene; and
R^{a}, R^{b}, R^{c}, and R^{d} being each independently H, C₁₋₆ alkyl, C₁₋₆ haloalkyl, heterocyclyl consisting of 3-6 atoms, C₆₋₁₀ aryl, heteroaryl consisting of 5-10 atoms, or heterocycle consisting of 3-6 atoms that is formed by R^{c}, R^{d}, and nitrogen atoms linked to R^{c} and R^{d}, C₁₋₆ alkyl, heterocycle consisting of 3-6 atoms, C₆₋₁₀ aryl, and heteroaryl consisting of 5-10 atoms being each independently unsubstituted or substituted with one, two, three, or four substituents, and the substituent being independently F, Cl, CN, OH, NH₂, C₁₋₆ alkyl, C₁₋₆ haloalkyl, C₁₋₆ alkoxy, or C₁₋₆ alkylamino.

According to an embodiment of the present disclosure, the compound is a compound shown in formula (II) or formula (III) or a stereoisomer, a tautomer, a nitrogen oxide, a solvate, a metabolite, a pharmaceutically acceptable salt, or a prodrug of the compound shown in formula (II) or formula (III),

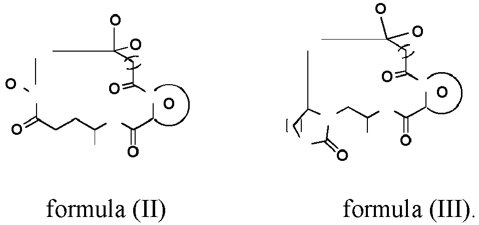

R₂ₐ, R_{2b}, k, R₁, n, z, and ring A are defined in the present disclosure as above.

According to an embodiment of the present disclosure, the compound is a compound shown in formula (IV) or formula (V) or a stereoisomer, a tautomer, a nitrogen oxide, a solvate, a metabolite, a pharmaceutically acceptable salt, or a prodrug of the compound shown in formula (IV) or formula (V),

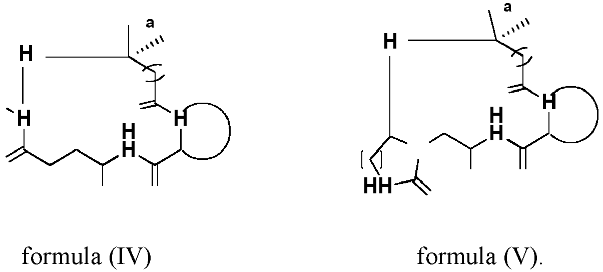

R₂ₐ, R_{2b}, k, R₁, n, z, and ring A are defined in the present disclosure as above.

According to an embodiment of the present disclosure, the compound is a compound shown in formula (VI) or formula (VII) or a stereoisomer, a tautomer, a nitrogen oxide, a solvate, a metabolite, a pharmaceutically acceptable salt, or a prodrug of the compound shown in formula (VI) or formula (VII),

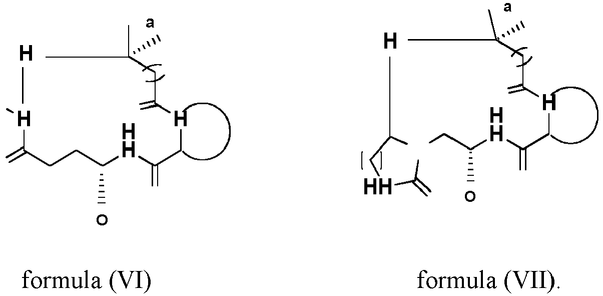

R₂ₐ, R_{2b}, k, R₁, n, z, and ring A are defined in the present disclosure as above.

According to an embodiment of the present disclosure, the compound is a compound shown in formula (VIII) or formula (IX) or a stereoisomer, a tautomer, a nitrogen oxide, a solvate, a metabolite, a pharmaceutically acceptable salt, or a prodrug of the compound shown in formula (VIII) or formula (IX),

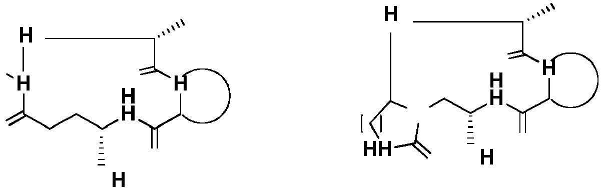

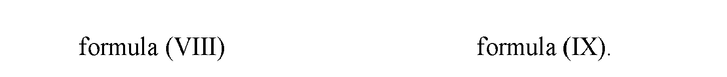

R₁, n, and ring A are defined in the present disclosure as above. R_{2b} is -C(=O)R^{a}, -C(=O)OR^{b}, -S(=O)₂R^{b}, -C(=O)NR^{c}R^{d}, -NC(=O)R^{c}R^{d}, -OR^{b}, -NR^{c}R^{d}, R^{b}0-C₁₋₄ alkylene, R^{d}R^{c}N-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, and (heteroaryl consisting of 5-10 atoms)-C₁₋₄ alkylene are each independently unsubstituted or substituted with one, two, three, or four substituents, and the substituent is independently F, Cl, Br, CN, =O, -OR^{b}, -NR^{c}R^{d}, C₁₋₆ alkyl, C₁₋₆ haloalkyl, R^{b}0-C₁₋₄ alkylene, or R^{d}R^{c}N-C₁₋₄ alkylene.

According to an embodiment of the present disclosure, the compound is a compound shown in formula (X) or formula (XI) or a stereoisomer, a tautomer, a nitrogen oxide, a solvate, a metabolite, a pharmaceutically acceptable salt, or a prodrug of the compound shown in formula (X) or formula (XI),

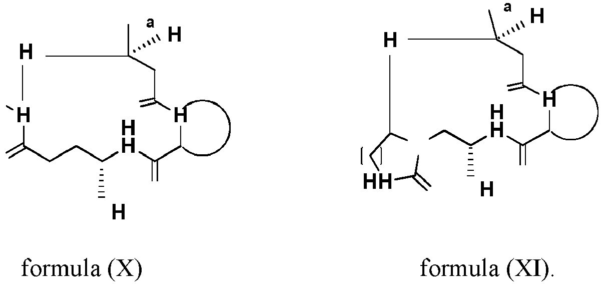

R₁, n, and ring A are defined in the present disclosure as above. R₂ₐ is -C(=O)R^{a}, -C(=O)OR^{b}, -S(=O)₂R^{b}, -C(=O)NR^{c}R^{d}, -NC(=O)R^{c}R^{d}, -OR^{b}, -NR^{c}R^{d}, R^{b}0-C₁₋₄ alkylene, R^{d}R^{c}N-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, and (heteroaryl consisting of 5-10 atoms)-C₁₋₄ alkylene are each independently unsubstituted or substituted with one, two, three, or four substituents, and the substituent is independently F, Cl, Br, CN, =O, -OR^{b}, -NR^{c}R^{d}, C₁₋₆ alkyl, C₁₋₆ haloalkyl, R^{b}0-C₁₋₄ alkylene, or R^{d}R^{c}N-C₁₋₄ alkylene.

According to an embodiment of the present disclosure, L₁ is a chain linker, and an atomic number of a main chain of the chain linker is 5, 6, 7, 8, 9, or 10, preferably, the atomic number of the main chain of the chain linker is 6.

According to an embodiment of the present disclosure, L₁ is optionally substituted -Rₑ-, -RₑCH(OH)R_{f}-, -RₑC(=O)R_{f}-, -RₑC(=O)OR_{f}-, -RₑOC(=O)R_{f}-, -RₑC(=O)SR_{f}-, -RₑSC(=O)R_{f}-, -RₑC(=O)N(R_{g})R_{f}-, -RₑN(R_{g})C(=O)R_{f}-, -RₑS(=O)₂N(R_{g})R_{f}-, - RₑN(R_{g})S(=O)₂R_{f}-, -RₑN(R_{g})C(=O)OR_{f}-, -RₑOC(=O)N(R_{g})R_{f}-, -RₑOR_{f}-, -RₑNR_{f}-, -RₑN=R_{f}-, - Rₑ-O-O-R_{f}-, -RₑSR_{f}-, -Rₑ-S-S-R_{f}-, -Rₑ-S-S-S-R_{f}-, -Rₑ-Rₕ-R_{f}-, or -Rₑ-O-Rₕ-R_{f}-, R_{g} being -H or substituted or unsubstituted alkyl, Rₑ being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rₕ being C₃₋₈ cycloalkyl, heterocyclyl consisting of 3-12 atoms, C₆₋₁₀ aryl, or heteroaryl consisting of 5-10 atoms, and t1 and t2 being independently integers not less than 1.

According to an embodiment of the present disclosure, L₁ is optionally substituted -Rₑ-, -RₑS(=O)₂N(R_{g})R_{f}-, -RₑN(R_{g})S(=O)₂R_{f}-, -RₑOR_{f}-, -RₑNR_{f}-, -RₑN=R_{f}-, -Rₑ-Rₕ-R_{f}-, or -Rₑ-O-Rₕ-R_{f}-, R_{g} being -H or substituted or unsubstituted alkyl, Rₑ being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rh being C₃₋₈ cycloalkyl, heterocyclyl consisting of 3-12 atoms, C₆₋₁₀ aryl, or heteroaryl consisting of 5-10 atoms, and t1 and t2 being independently integers not less than 1.

According to an embodiment of the present disclosure, the substituent of L₁ is F, Cl, Br, CN, NO₂, =O, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, heterocyclyl consisting of 3-12 atoms, C₆₋₁₀ aryl, or heteroaryl consisting of 5-10 atoms.

According to an embodiment of the present disclosure, R₂ₐ and R_{2b} are each independently H, -OR^{b}, or -NC(=O)R^{c}R^{d}, and R₂ₐ and R_{2b} are not all H.

According to an embodiment of the present disclosure, the ring A is optionally heterocyclyl consisting of 5-10 atoms that is substituted with one or more R'.

According to an embodiment of the present disclosure, Z is cyano.

According to an embodiment of the present disclosure, m is 1.

According to an embodiment of the present disclosure, R' is independently F, Cl, Br, C₁₋₆ alkyl, or C₁₋₆ haloalkyl.

According to a second aspect of the present disclosure, the present disclosure provides a compound. The compound is of the following structures or is a stereoisomer, a tautomer, a nitrogen oxide, a solvate, a metabolite, a pharmaceutically acceptable salt, or a prodrug of the following structures,

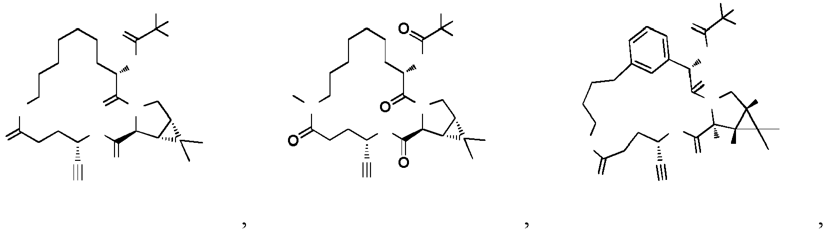

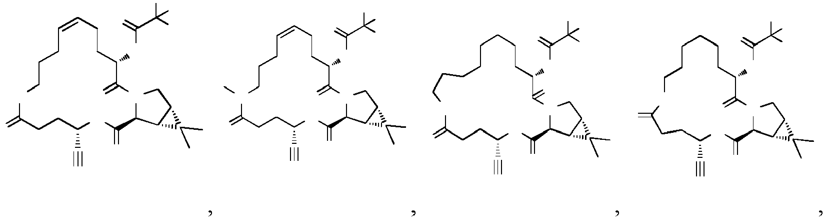

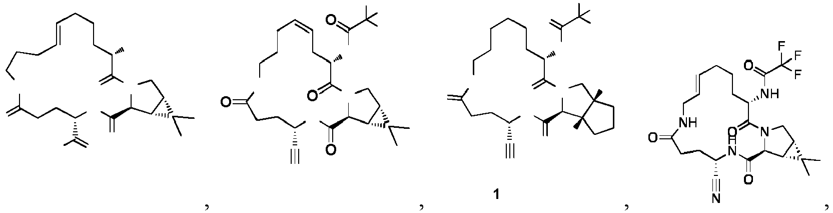

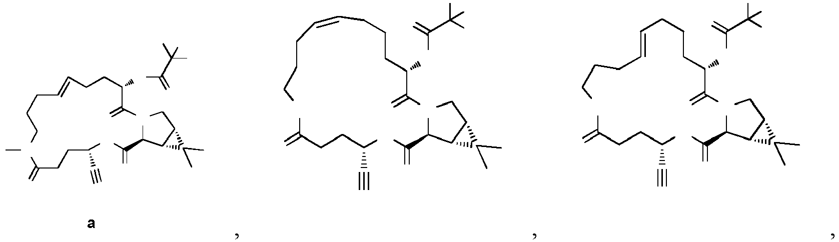

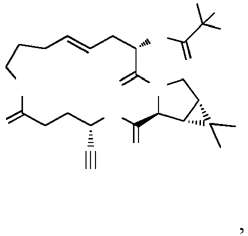

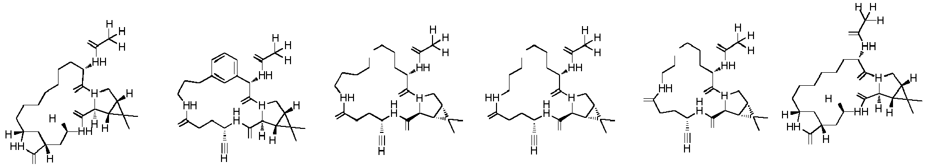

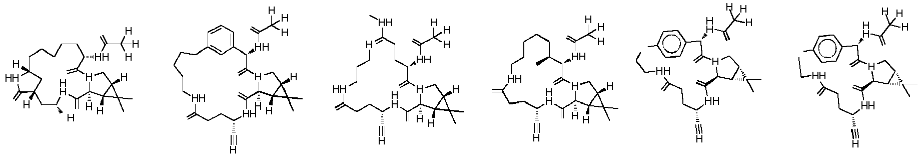

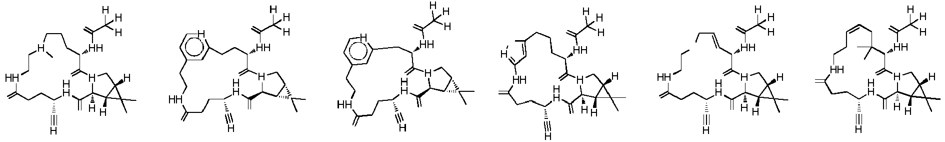

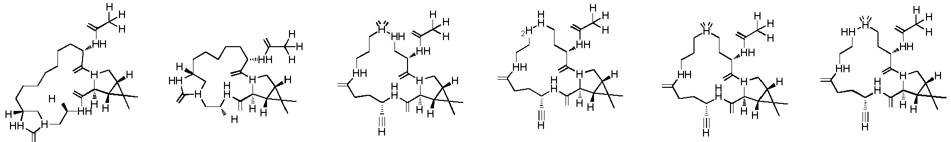

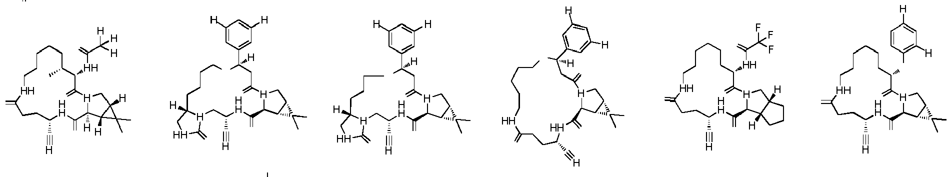

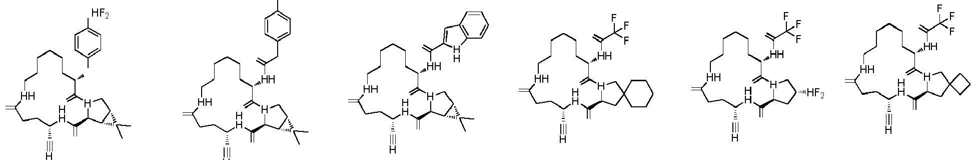

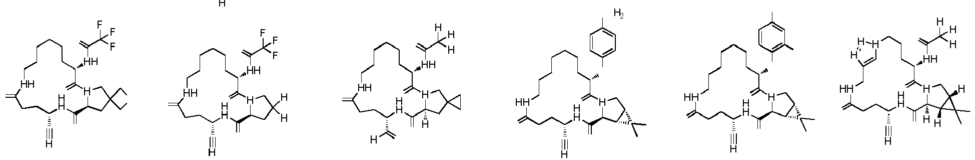

According to a third aspect of the present disclosure, the present disclosure provides a pharmaceutical composition, including an effective amount of the compound above.

According to an embodiment of the present disclosure, the pharmaceutical composition further includes a pharmaceutically acceptable carrier, an adjuvant, a medium, or a combination thereof.

According to an embodiment of the present disclosure, the pharmaceutical composition further includes one or more therapeutic agents, and the therapeutic agent is at least one of other drugs against coronaviruses, drugs suppressing cytokine storms, drugs boosting immunity, and drugs regulating intestinal flora.

According to a fourth aspect of the present disclosure, the present disclosure provides use of the compound above or the pharmaceutical composition above in preparation of drugs, the drugs being used for preventing coronavirus infection or treating or reducing associated diseases of patients caused by coronavirus infection.

According to an embodiment of the present disclosure, the coronavirus includes at least one of HCoV-229E, HCoV-OC43, HCoV-NL63, HCoV-HKU1, SARS-CoV, 2019-nCoV, and MERS-Cov.

According to a fifth aspect of the present disclosure, the present disclosure provides use of the compound above or the pharmaceutical composition above in preparation of drugs, the drugs being used for inhibiting M^{pro} or inhibiting coronavirus replication.

According to a sixth aspect of the present disclosure, the present disclosure provides the compound above or the pharmaceutical composition above for preventing coronavirus infection or treating or reducing associated diseases of patients caused by coronavirus infection.

According to a seventh aspect of the present disclosure, the present disclosure provides the compound above or the pharmaceutical composition above for inhibiting M^{pro} or inhibiting coronavirus replication.

According to an eighth aspect of the present disclosure, the present disclosure provides a method for preventing coronavirus infection or treating or reducing associated diseases of patients caused by coronavirus infection. According to an embodiment of the present disclosure, the method includes administering to a subject a pharmaceutically acceptable dose of the compound above or the pharmaceutical composition above.

According to a ninth aspect of the present disclosure, the present disclosure provides a method for inhibiting M^{pro} or inhibiting coronavirus replication. According to an embodiment of the present disclosure, the method includes contacting M^{pro} or the coronavirus with the compound above or the pharmaceutical composition above.

Unless otherwise specified, the present disclosure includes stereoisomers, geometric isomers, tautomers, solvates, hydrates, metabolites, salts, and pharmaceutically acceptable prodrugs of all the compounds in the present disclosure.

In some embodiments, the salts are pharmaceutically acceptable salts. The term "pharmaceutically acceptable" means that substances or compositions have to be chemically and/or toxicologically compatible with other ingredients including a formulation and/or mammals to be treated with the substances or compositions.

The compound of the present disclosure further includes its salt. The salt is not necessarily a pharmaceutically acceptable salt, but can be used for preparation and/or purification of the compound of the present disclosure and/or for isolation of an intermediate of an enantiomer of the compound of the present disclosure.

The compound of the present disclosure, including its salt, can also be obtained in a form of a hydrate, or includes other solvents for crystallization. The compound of the present disclosure can form a solvate with a pharmaceutically usable solvent (including water) inherently or by design. Therefore, the present disclosure further includes solvated and unsolvated forms of the compound.

In addition, the compound of the present disclosure may include several asymmetric centers or a form of a mixture of commonly described racemates. The present disclosure further includes a racemic mixture, a part of a racemic mixture, and an enantiomer and a diastereomer that are obtained by isolation.

The compound of the present disclosure may exist in the form of a possible isomer, a rotamer, an atropisomer, a tautomer, or a mixture thereof. The present disclosure may further include a mixture of an isomer, a rotamer, an atropisomer, and a tautomer of the compound of the present disclosure, or a part of a mixture of an isomer, a rotamer, an atropisomer, and a tautomer, or an isomer, a rotamer, an atropisomer, and a tautomer that have been isolated.

In addition, the compound of the present disclosure includes compounds that are defined in the present disclosure and that are labeled by various isotopes, for example, those compounds in which radioisotopes such as ³H, ¹⁴C, and ¹⁸F are present, or compounds in which non-radioisotopes such as ²H and ¹³C are present.

Moreover, the present disclosure relates to methods for preparing, isolating, and purifying the compound shown in formula (I).

The foregoing content describes only some aspects of the present disclosure, but the present disclosure is not limited to these aspects. These aspects and other aspects will be described more specifically and completely below.

### Definitions and General Terms

Examples of some implementations of the present disclosure described in detail herein are illustrated with the accompanying structural and chemical formulas. The present disclosure is intended to cover all alternative, modified, and equivalent technical solutions, which are all included within the scope of the present disclosure as defined by the claims. A person skilled in the art has to recognize that many methods and materials similar or equivalent to those described in the present disclosure can be used for implementing the present disclosure. The present disclosure is by no means limited to the methods and materials described in the present disclosure. When one or more of the referenced literature, patents, and similar materials differ from or contradict the present disclosure (including, but not limited to, the terms defined, application of the terms, the techniques described, and the like), the present disclosure prevails.

It is to be further recognized that some features of the present disclosure are described in multiple separate implementations for clarity, or may be provided in combination in a single embodiment. Conversely, various features of the present disclosure are described in a single implementation for brevity, or may be provided separately or in any proper subcombination.

Unless otherwise indicated, technical and scientific terms used in the present disclosure have the same meanings as those conventionally understood by a person skilled in the art to which the present disclosure belongs. Unless otherwise indicated, all patent disclosure publications cited in all the content disclosed by the present disclosure are incorporated herein by reference in their entirety.

The following definitions are applied in the present disclosure unless otherwise indicated. For the purpose of the present disclosure, chemical elements are defined according to the Periodic Table of Elements, CAS database, and Handbook of Chemical Drugs, 75th Ed, 1994. In addition, general principles of organic chemistry are described in "Organic Chemistry", Thomas Sorrell, University Science Books, Sausalito, 1999, and "March's Advanced Organic Chemistry", Michael B. Smith and Jerry March, John Wiley & Sons, New York, 2007, so all content of the present disclosure incorporates references.

The term "subject" used in the present disclosure refers to an animal. Typically, the animal is a mammal. The subject also refers to a primate (for example, a human), a cow, a sheep, a goat, a horse, a dog, a cat, a rabbit, a rat, a mouse, a fish, a bird, and the like. In some implementations, the subject is a primate. In some other implementations, the subject is a human.

The terms "subject", "treated object", and "patient" used in the present disclosure are used interchangeably. The terms "subject", "treated object", and "patient" refer to animals (such as chickens, quails, turkeys, or other birds or mammals), particularly "mammals" including non-primates (such as cows, pigs, horses, goats, rabbits, guinea pigs, rats, cats, dogs, and mice) and primates (such as monkeys, chimpanzees, and humans), more particularly humans. In an implementation, the treated object is a non-human animal, for example, a domestic animal (such as a horse, a cow, a pig, or a goat) or a pet (such as a dog, a cat, a guinea pig, or a rabbit). In some other implementations, the "patient" refers to a human.

The present disclosure also includes isotopic-labeled compounds that are identical to those compounds described herein except for the fact that one or more atoms are substituted with atoms with an atomic mass or a mass number different from that of naturally occurring atoms. Exemplary isotopes may also be introduced into the compounds of the present disclosure, including isotopes of hydrogen, carbon, nitrogen, oxygen, phosphorus, sulfur, fluorine, and chlorine, such as ²H, ³H, ¹³C, ¹⁴C, ¹⁵N, ¹⁶O, ¹⁷O, ³¹P, ³²P, ³⁶S, ¹⁸F, and ³⁷Cl.

The compounds of the present disclosure including the foregoing isotopes and/or other isotopes of other atoms, as well as pharmaceutically acceptable salts of the compounds, are all included within the scope of the present disclosure. The compounds of the present disclosure labeled by isotopes, for example, radioisotopes such as ³H and ¹⁴C, can be used for drug and/or substrate tissue distribution analysis. Tritium ³H and carbon-14 ¹⁴C are particularly preferred due to ease of preparation and detection. In addition, substitution with a heavy isotope, for example, deuterium ²H, can provide some therapeutic advantages from higher metabolic stability, for example, an increased in vivo half-life or a reduced dosage requirement. Therefore, the heavy isotope may be preferred in some cases.

The stereochemical definitions and conventions used in the present disclosure are generally in accordance with S.P.Parker, Ed., McGraw-Hill Dictionary of Chemical Terms (1984), McGraw-Hill Book Company, New York; and Eliel, E. and Wilen, S., "Stereochemistry of Organic Compounds", John Wiley & Sons, Inc., New York, 1994. The compound of the present disclosure may include asymmetric centers or chiral centers, and thus exists in different stereoisomeric forms. It is anticipated that all stereoisomeric forms of the compound of the present disclosure, including, but not limited to, diastereoisomers, enantiomers, atropisomers, and mixtures thereof such as racemic mixtures, are also included within the scope of the present disclosure. Many organic compounds exist in optically active forms, that is, the compounds have the ability to rotate the plane of plane-polarized light. When describing optically active compounds, the prefixes D and L orR and S are used to denote the absolute configuration of a molecule based on a chiral center (or multiple chiral centers) in the molecule. The prefixes d and *l* or (+) and (-) are symbols that indicate the rotation of plane-polarized light caused by the compound. (-) or *l* indicates that the compound is levorotatory. The compound prefixed with (+) or *d* is dextrorotatory. For a given chemical structure, these stereoisomers are identical except that they are mirror images of each other. Specifically, stereoisomers may also be referred to as enantiomers, and a mixture of the isomers is usually referred to as a mixture of the enantiomers. A 50:50 mixture of enantiomers is referred to as a racemic mixture or racemate. The racemic mixture or racemate may occur when there is no stereoselectivity or stereospecificity in a chemical reaction or method.

Based on selection of raw materials and methods, the compound of the present disclosure may exist in the form of one of possible isomers or a mixture thereof, for example, as a pure optical isomer or as an isomer mixture such as a mixture of racemates and diastereoisomers, depending on a quantity of asymmetric carbon atoms. The optical (*R*)- or (*S*)-isomer may be prepared with chiral synthons or chiral preparations, or may be split using conventional techniques. If this compound contains a double bond, the substituent may be in the *E* or *Z* configuration. If this compound contains a disubstituted cycloalkyl group, the substituent of the cycloalkyl group may be in the *cis-* or *trans-* configuration.

The compound of the present disclosure may include asymmetric centers or chiral centers, and thus exists in different stereoisomer forms. It is anticipated that all stereoisomer forms of the compound of the present disclosure, including, but not limited to, diastereoisomers, enantiomers, atropisomers, geometric (or conformational) isomers, and mixtures thereof such as racemic mixtures, are included within the scope of the present disclosure.

Unless otherwise indicated, the structure described in the present disclosure also denotes all isomers (such as enantiomers, diastereomers, atropisomers, and geometric (or conformational) isomers) including this structure, for example, *R* and *S* conformations of respective asymmetric centers, (*Z*) and (*E*) double-bond isomers, and (*Z*) and (*E*) conformational isomers. Therefore, a single stereochemical isomer, a mixture of enantiomers, a mixture of diastereomers, and a mixture of geometric (or conformational) isomers of the compound of the present disclosure are within the scope of the present disclosure.

The term "tautomer" or "tautomeric form" refers to structural isomers of different energies that can be interconverted through a low energy barrier. If tautomerization is possible (for example, in a solution), chemical equilibrium of tautomers can be achieved. For example, proton tautomers (also referred to as prototropic tautomers) include interconversion by protolysis, for example, keto-enol isomerization and imine-enamine isomerization. Valence tautomers include interconversion by recombination of some bonding electrons. A specific example of keto-enol tautomerization is the interconversion of pentane-2,4-dione and 4-hydroxypentyl-3-enyl-2-one tautomers. Another example of tautomerization is phenol-keto tautomerization. A specific example of phenol-keto tautomerization is the interconversion of pyridine-4-ol and pyridine-4(1*H*)-keto tautomers. Unless otherwise indicated, all tautomer forms of the compound of the present disclosure are within the scope of the present disclosure.

The term "nitrogen oxide" used in the present disclosure refers to an N-oxide that can be formed by oxidizing one or more nitrogen atoms when the compound contains several amine functional groups. A particular example of the *N*-oxide is an *N*-oxidesof tertiary amine or an *N*-oxide containing nitrogen heterocyclic atoms. Corresponding amine may be treated with an oxidizing agent such as hydrogen peroxide or a peracid (such as peroxycarboxylic acid) to form an *N*-oxide (see Advanced Organic Chemistry, Wiley Interscience, 4th edition, Jerry March, pages). In particular, the N-oxide may be prepared by the method of L. W. Deady (Syn. Comm. 1977, 7, 509-514). For example, an amine compound undergoes a reaction with m-chloroperbenzoic acid (MCPBA) in an inert solvent such as dichloromethane.

The term "solvate" in the present disclosure refers to an associate formed by one or more solvent molecules with the compound of the present disclosure. A solvent that forms the solvate includes, but is not limited to, water, isopropanol, ethanol, methanol, dimethyl sulfoxide, ethyl acetate, acetic acid, and aminoethanol. The term "hydrate" refers to an associate formed by water as the solvent molecule.

The term "metabolite" refers to a product obtained by metabolism of a specific compound or its salt in the body. A metabolite of a compound may be identified by techniques well known in the art, and its activity may be characterized by experimental methods as described in the present disclosure. Such product may be obtained by oxidation, reduction, hydrolysis, amidation, deamidation, esterification, de-esterification, enzymatic lysis, and the like of an administered compound. Correspondingly, the present disclosure includes metabolites of the compounds, including metabolites resulting from sufficient contact between the compounds of the present disclosure and mammals for a period of time.

The term "pharmaceutically acceptable salt" used in the present disclosure refers to an organic salt and an inorganic salt of the compound of the present disclosure. The pharmaceutically acceptable salt is well known in the art, as documented in S. M. Berge et al., describe pharmaceutically acceptable salts in detail in J. Pharmaceutical Sciences, 1977, 66: 1-19. A pharmaceutically acceptable salt formed by non-toxic acid includes, but is not limited to, inorganic acid salts formed by reaction with amino groups such as hydrochloride, hydrobromide, phosphate, sulfate, and perchlorate, and organic acid salts such as acetate, oxalate, maleate, tartrate, citrate, succinate, and malonate. These salts may be obtained by other methods such as ion exchange as documented in the literature of books. Other pharmaceutically acceptable salts include adipate, alginate, ascorbate, aspartate, benzenesulfonate, benzoate, bisulfate, borate, butyrate, camphorate, camphorsulfonate, cyclopentylpropionate, digluconate, dodecylsulfate, ethanesulfonate, formate, fumarate, gluceptate, glycerophosphate, gluconate, hemisulfate, heptanoate, caproate, hydriodate, 2-hydroxy-ethanesulfonate, lactobionate, lactate, laurate, lauryl sulfate, malate, malonate, methanesulfonate, 2-naphthalene sulfonate, nicotinate, nitrate, oleate, palmitate, pamoate, pectate, persulfate, 3-phenylpropionate, picrate, pivalate, propionate, stearate, thiocyanate, p-toluenesulfonate, undecanoate, pentanoate, and the like. Salts obtained with proper alkali include salts of alkali metals, alkaline earth metals, ammonium, and N⁺(C₁₋₄ alkyl)₄. The present disclosure is also intended to conceptualize quaternary ammonium salts formed by any compound including an N group. Water-soluble or oil-soluble or disperse products can be obtained by quaternization. Salts of alkali metals or alkaline earth metals include salts of sodium, lithium, potassium, calcium, magnesium, and the like. The pharmaceutically acceptable salt further includes proper and non-toxic ammonium, quaternary ammonium salts, and amine cations formed by counter ions, such as halides, hydroxides, carboxylates, sulfates, phosphates, nitrates, C₁₋₈ sulfonates, and aromatic sulfonates.

The term "prodrug" used in the present disclosure indicates a compound that converts in vivo into the compound shown in formula (I). Such conversion is affected by hydrolysis of the prodrug in the blood or enzymatic conversion to a parent structure in the blood or tissue. The prodrug compounds of the present disclosure may be esters. Examples of existing applications in which esters can be used as prodrugs are phenyl esters, aliphatic (C₁₋₂₄) esters, acyloxy methyl esters, carbonates, carbamates, and amino acid esters. For example, if a compound in the present disclosure includes a hydroxyl group, the compound can be acylated to obtain a compound in the form of a prodrug. Other prodrug forms include phosphate esters. For example, these compounds in the form of phosphate esters are obtained by phosphorylating hydroxyl groups on parent structures. For a complete discussion of the prodrug, refer to the following literature: T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

Any asymmetric atoms (such as carbon) of the compound of the present disclosure may be present in a racemate-enriched or enantiomer-enriched form, for example, in the (*R*)-, (*S*)-, or (*R*, *S*)-configuration. In some implementations, each asymmetric atom has at least a 50% enantiomeric excess, at least a 60% enantiomeric excess, at least a 70% enantiomeric excess, at least an 80% enantiomeric excess, at least a 90% enantiomeric excess, at least a 95% enantiomeric excess, or at least a 99% enantiomeric excess in the (*R*)- or (*S*)-configuration. If possible, a substituent on atoms with an unsaturated double bond may be in the cis-(*Z*)- or trans-(*E*)-form.

Therefore, as described in the present disclosure, the compound of the present disclosure may exist in the form of a possible isomer, a rotamer, an atropisomer, a tautomer, or a mixture thereof, for example, in the form of a substantially pure geometric (cis- or trans-) isomer, a diastereoisomer, an optical isomer (enantiomer), a racemate, or a mixture thereof.

Any resulting mixture of isomers may be separated into pure or substantially pure geometric or optical isomers, diastereoisomers, and racemates based on physicochemical differences of the components, for example, by chromatography and/or stepwise crystallization.

A racemate of any resulting end product or intermediate may be split into optical enantiomers by known methods familiar to a person skilled in the art, for example, by separating the obtained diastereoisomeric salt thereof. A racemic product may also be separated by chiral chromatography, for example, by high pressure liquid chromatography (HPLC) with a chiral adsorbent. Particularly, an enantiomer may be prepared by asymmetric synthesis (for example, Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972)).

As described in the present disclosure, the compound of the present disclosure may be optionally substituted with one or more substituents as the compounds of the general formulas above, or as the particular examples, subclasses, and classes of compounds included in the present disclosure that are in the embodiments. It is to be understood that the term "optionally substituted" is interchangeable with the term "substituted or unsubstituted". The term "optionally" or "optional" means that an event or condition subsequently described may, but is not required to, occur, and that the description includes a case in which the event or condition occurs as well as a case in which the event or condition does not occur. In general, the term "optionally", whether or not preceding the term "substituted", indicates that one or more hydrogen atoms in a given structure are substituted with a specific substituent. Unless otherwise indicated, an optional substituent may substitute for any substitutable position of the group. When more than one position of a given structural formula can be substituted with one or more substituents selected from a specific group, the substituents may substitute for the positions uniformly or differently. The substituent may be, but is not limited to, F, Cl, Br, CN, N3, OH, NH₂, NO₂, oxo (=O), -C(=O)R^{a}, -C(=O)OR^{b}, -S(=O)₂R^{b}, -C(=O)NR^{c}R^{d}, - NC(=O)R^{c}R^{d}, -OR^{b}, -NR^{c}R^{d}, R^{b}0-C₁₋₄ alkylene, R^{d}R^{c}N-C₁₋₄ alkylene, C₁₋₆ alkyl, C₁₋₆ aliphatic, C₁₋₆ haloalkyl, C₁₋₆ alkoxy, C₁₋₆ alkylamino, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-14 atoms, or (heteroaryl consisting of 5-14 atoms)-C₁₋₄ alkylene. R^{a}, R^{b}, R^{c}, and R^{d} are defined in the present disclosure as above.

In addition, unless otherwise explicitly indicated, the descriptions "each independently" and "independently" are interchangeable in the present disclosure, and are to be broadly construed to mean either that specific options expressed between the same symbols do not affect each other in different groups, or that specific options expressed between the same symbols do not affect each other in the same groups.

In various parts of this specification, the substituents of the compounds disclosed herein are described based on group types or ranges. In particular, the present disclosure includes independent sub-combinations of members of these group types and ranges. For example, the term "C₁₋₆ alkyl" specifically refers to independently disclosed methyl, ethyl, C₃ alkyl, C₄ alkyl, C₅ alkyl, and C₆ alkyl.

A linker substituent is described in various parts of the present disclosure. When the structure clearly requires a linker group, the Markush variables listed for the group are to be understood as linker groups. For example, if the structure requires a linker group and the Markush variables for the group are defined and listed as "alkyl" or "aryl", it is to be understood that the "alkyl" or "aryl" respectively indicates a linked alkylene group or arylene group.

The term "alkyl" or "alkyl group" used in the present disclosure denotes a monovalent hydrocarbon atomic group with a saturated straight or branched chain containing, for example, 1-20 carbon atoms. Unless otherwise specified, the alkyl group contains 1-20 carbon atoms. In some embodiments, the alkyl group contains 1-10 carbon atoms. In some other embodiments, the alkyl group contains 1-9 carbon atoms. In some other embodiments, the alkyl group contains 1-8 carbon atoms. In some other embodiments, the alkyl group contains 1-6 carbon atoms. In some other embodiments, the alkyl group contains 1-4 carbon atoms. In some other embodiments, the alkyl group contains 1-3 carbon atoms.

The term "branched alkyl" used in the present disclosure denotes a monovalent hydrocarbon atomic group with a saturated branched chain containing, for example, 3-30 carbon atoms. Unless otherwise specified, the branched alkyl group contains 3-30 carbon atoms. In some embodiments, the branched alkyl group contains 3-10 carbon atoms. In some other embodiments, the branched alkyl group contains 3-9 carbon atoms. In some other embodiments, the branched alkyl group contains 3-8 carbon atoms. In some other embodiments, the branched alkyl group contains 3-6 carbon atoms. In some other embodiments, the branched alkyl group contains 3-4 carbon atoms. In some other embodiments, the branched alkyl group contains 3 carbon atoms.

Examples of the alkyl group include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tertiary butyl (t-Bu, -C(CH₃)₃), n-amyl (-CH₂CH₂CH₂CH₂CH₃), 2-amyl (-CH(CH₃)CH₂CH₂CH₃), 3-amyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-amyl (-C(CH₃)₂CH₂CH₂CH₃), 3-methyl-2-amyl (-CH(CH₃)CH(CH₃)CH₂CH₃), 4-methyl-2-amyl (-CH(CH₃)CH₂CH(CH₃)₂), 3-methyl-3-amyl (-C(CH₃)(CH₂CH₃)₂), 2-methyl-3-amyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, and the like. The alkyl group may be independently unsubstituted or substituted with one or more substituents described in the present disclosure.

The term "alkyl" and its prefix "alkyl-" used in the present disclosure include straight and branched saturated carbon chains.

The term "alkylene" denotes a saturated divalent hydrocarbyl group obtained by removing two hydrogen atoms from a straight or branched saturated hydrocarbyl group. Unless otherwise specified, the alkylene group contains 1-10 carbon atoms. In some other embodiments, the alkylene group contains 1-6 carbon atoms. In some other embodiments, the alkylene group contains 1-4 carbon atoms. In some other embodiments, the alkylene group contains 1-2 carbon atoms. Such examples include methylene (-CH₂-), ethylene (-CH₂CH₂-), isopropylidene (-CH(CH₃)CH₂-), and the like. The alkylene group may be independently unsubstituted or substituted with one or more substituents described in the present disclosure.

The term "alkenyl" denotes a monovalent hydrocarbyl group with a straight or branched chain containing, for example, 2-30 carbon atoms, or 2-12 carbon atoms, or 2-8 carbon atoms, or 2-6 carbon atoms, or 2-4 carbon atoms. At least one C-C is in an sp² double-bond unsaturated state. A branched-chain monovalent hydrocarbyl group is also referred to as a branched alkenyl group, for example, C₃-C₃₀ branched alkenyl. The alkenyl group may be independently unsubstituted or substituted with one or more substituents described in the present disclosure, including groups with "cis", "trans", "*Z*", or "*E*" positioning. Specific examples include, but are not limited to, vinyl (-CH=CH₂), allyl (-CH₂CH=CH₂), and the like.

The term "alkenylene" denotes an unsaturated monovalent hydrocarbyl group obtained by removing two hydrogen atoms from a straight or branched unsaturated hydrocarbyl group. At least one C-C is in an sp² double-bond unsaturated state.

The term "alkynyl" denotes a monovalent hydrocarbyl group with a straight or branched chain containing, for example, 2-30 carbon atoms, or 2-12 carbon atoms, or 2-8 carbon atoms, or 2-6 carbon atoms, or 2-4 carbon atoms. At least one C-C is in an sp triple-bond unsaturated state. A branched-chain monovalent hydrocarbyl group is also referred to as a branched alkynyl group, for example, C₃-C₃₀ branched alkynyl. The alkynyl group may be independently unsubstituted or substituted with one or more substituents described in the present disclosure. Specific examples include, but are not limited to, ethynyl (-C=CH), 2-propargyl (-CH₂C≡CH), 1-propargyl (-C≡C-CH₃), and the like.

The term "alkynylene" denotes an unsaturated monovalent hydrocarbyl group obtained by removing two hydrogen atoms from a straight or branched unsaturated hydrocarbyl group. At least one C-C is in an sp triple-bond unsaturated state.

The term "alkoxy" indicates that an alkyl group is linked, by an oxygen atom, to the rest of the molecule. The alkyl group is defined in the present disclosure as above. Unless otherwise specified, the alkoxy group contains 1-20 carbon atoms. In some embodiments, the alkoxy group contains 1-10 carbon atoms. In some other embodiments, the alkoxy group contains 1-8 carbon atoms. In some other embodiments, the alkoxy group contains 1-6 carbon atoms. In some other embodiments, the alkoxy group contains 1-4 carbon atoms. In some other embodiments, the alkoxy group contains 1-3 carbon atoms.

The term "linker" used in the present disclosure denotes a group that links two groups together. For example, in formula (I), the linker denotes a group that links a C atom to an L₂ group, including but not limited to: a peptide linker (such as a valine-lysine sequence), a small-molecule chemical group linker (such as a straight-chain alkyl group), and the like, with a chain or ring structure. The chain structure includes a straight- or branched-chain structure. The ring structure is shown in 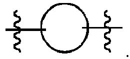 The ring B may be cycloalkyl, heterocyclyl, aryl, or heteroaryl. When the linker is a linker of a chain structure, an atomic number of a main chain of the linker refers to a quantity of atoms on the main chain, for example, an atomic number of a main chain in isobutyl (*i*-Bu, -CH₂CH(CH₃)₂) is 3. When the linker is a linker of a chain structure, if a ring structure is inserted in the chain structure, such as -Rₑ-Rₕ-R_{f}-, an atomic number of a main chain is a sum of the lesser quantity of atoms between linking sites of Rₕ and Rₑ or R_{f} (including the atom at the linking site) and the quantity of atoms on the main chain of Rₑ and R_{f}. For example, a linker of the chain of 7. 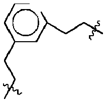 structure has an atomic number of a main

Examples of the alkoxy group include, but are not limited to, methoxy (MeO, - OCH₃), ethoxy (EtO, -OCH₂CH₃), 1-propoxy (n-PrO, n-propoxy, -OCH₂CH₂CH₃), 2-propoxy (i-PrO, i-propoxy, -OCH(CH₃)₂), 1-butoxy (n-BuO, n-butoxy, -OCH₂CH₂CH₂CH₃), 2-methyl-1-propoxy (*i*-BuO, *i*-butoxy, -OCH₂CH(CH₃)₂), 2-butoxy (s-BuO, s-butoxy, - OCH(CH₃)CH₂CH₃), 2-methyl-2-propoxy (t-BuO, t-butoxy, -OC(CH₃)₃), 1-pentyloxy (n-pentyloxy, -OCH₂CH₂CH₂CH₂CH₃), 2-pentyloxy (-OCH(CH₃)CH₂CH₂CH₃), 3-pentyloxy (-OCH(CH₂CH₃)₂), 2-methyl-2-butoxy (-OC(CH₃)₂CH₂CH₃), 3-methyl-2-butoxy (-OCH(CH₃)CH(CH₃)₂), 3-methyl-1-butoxy (-OCH₂CH₂CH(CH₃)₂), 2-methyl-1-butoxy (-OCH₂CH(CH₃)CH₂CH₃), and the like. The alkoxy group may be independently unsubstituted or substituted with one or more substituents described in the present disclosure.

The term "haloalkyl", "haloalkenyl", or "haloalkoxy" indicates that an alkyl, alkenyl, or alkoxy group is substituted with one or more halogen atoms. Such examples include, but are not limited to, trifluoromethyl, trifluoromethoxy, and the like.

The terms "carbon ring", "carbocyclyl", and "carbocyclic" are interchangeable herein, which refer to a non-aromatic carbocyclic system that is saturated or contains one or more unsaturated elements and that includes 3-14 cyclic carbon atoms. In some implementations, a quantity of carbon atoms is 3-12. In some other implementations, a quantity of carbon atoms is 3-10. In some other implementations, a quantity of carbon atoms is 3-8. In some other implementations, a quantity of carbon atoms is 5-6. In some other implementations, a quantity of carbon atoms is 6-8. The "carbocyclyl" includes a monocyclic, bicyclic, or polycyclic and fused, spiro, or bridged carbon ring system, and also includes a polycyclic ring system in which a carbon ring may be fused with one or more non-aromatic carbon rings or heterocyclic rings, one or more aromatic rings, or combinations thereof, where linking atomic groups or dots are on the carbon ring. Bicyclic carbocyclyl includes bridged bicyclic carbocyclyl, fused bicyclic carbocyclyl, and spiro bicyclic carbocyclyl. A "fused" bicyclic ring system includes two rings that share two neighboring rong atoms. A bridged bicyclic group includes two rings that share three or four neighboring ring atoms. Spiro ring systems share one ring atom. Proper carbocyclyl groups include, but are not limited to, cycloalkyl, cycloalkenyl, and cycloalkynyl. Examples of the carbocyclyl groups further include, but are not limited to, cyclopropyl, cyclobutyl, cyclopentyl, 1-cyclopentyl-1-alkenyl, 1-cyclopentyl-2-alkenyl, 1-cyclopentyl-3-alkenyl, cyclohexyl, 1-cyclohexyl-1-alkenyl, 1-cyclohexyl-2-alkenyl, 1-cyclohexyl-3-alkenyl, cyclohexadienyl, cycloheptyl, cyclooctyl, cyclononyl, cyclodecyl, cycloundecyl, cyclododecyl, and the like. Bridged carbocyclyl groups include, but are not limited to, bicyclo[2.2.2]octyl, bicyclo[2.2.1]heptyl, bicyclo[3.3.1]nonyl, bicyclo[3.2.3]nonyl, and the like.

The term "cycloalkyl" refers to a monocyclic, bicyclic, or tricyclic saturated system containing 3-12 cyclic carbon atoms, with one or more linking dots linked to the rest of the molecule. In some embodiments, the cycloalkyl is a ring system containing 3-10 cyclic carbon atoms. In some other embodiments, the cycloalkyl is a ring system containing 3-8 cyclic carbon atoms. In some other embodiments, the cycloalkyl is a ring system containing 3-6 cyclic carbon atoms. In some other embodiments, the cycloalkyl is a ring system containing 5-6 cyclic carbon atoms. The cycloalkyl group may be independently unsubstituted or substituted with one or more substituents described in the present disclosure.

The terms "heterocyclyl" and "heterocyclic ring" are interchangeable herein, which refer to a saturated or partially unsaturated, non-aromatic monocyclic, bicyclic, or tricyclic ring system containing 3-12 ring atoms. At least one ring atom is selected from nitrogen, sulfur, and oxygen atoms. This ring system includes one or more linking dots linked to the rest of the molecule. The term "heterocyclyl" includes a monocyclic, bicyclic, or polycyclic and fused, spiro, or bridged heterocyclic ring system, and also includes a polycyclic ring system in which a heterocyclic ring may be fused with one or more non-aromatic carbon rings or heterocyclic rings, one or more aromatic rings, or combinations thereof, where linking atomic groups or dots are on the heterocyclic ring. Bicyclic heterocyclyl includes bridged bicyclic heterocyclyl, fused bicyclic heterocyclyl, and spiro bicyclic heterocyclyl. Unless otherwise indicated, the heterocyclyl may be carbon-based or nitrogen-based, and the -CH₂-group may be optionally substituted by -C(=O)-. The sulfur atom of the ring may be optionally oxidized to an S-oxide. The nitrogen atom of the ring may be optionally oxidized to an *N*-oxide. In some implementations, the heterocyclyl is monocyclic or bicyclic heterocyclyl consisting of 3-10 atoms. In some other implementations, the heterocyclyl is monocyclic or bicyclic heterocyclyl consisting of 3-8 atoms. In some other implementations, the heterocyclyl is monocyclic or bicyclic heterocyclyl consisting of 3-6 atoms. In some other implementations, the heterocyclyl is monocyclic or bicyclic heterocyclyl consisting of 6-8 atoms. In some other implementations, the heterocyclyl is heterocyclyl consisting of 5-6 atoms. In some other implementations, the heterocyclyl is heterocyclyl consisting of 4 atoms. In some other implementations, the heterocyclyl is heterocyclyl consisting of 5 atoms. In some other implementations, the heterocyclyl is heterocyclyl consisting of 6 atoms. In some other implementations, the heterocyclyl is heterocyclyl consisting of 7 atoms. In some other implementations, the heterocyclyl is heterocyclyl consisting of 8 atoms.

Examples of the heterocyclyl include, but are not limited to: oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothiophene, dihydrothiophene, 1,3-dioxolan, dithiolane, tetrahydropyran, dihydropyran, 2*H*-pyran, 4*H-*pyran, tetrahydrothiopyranyl, piperidinyl, morpholinyl, thiomorpholinyl, piperazinyl, dioxanyl, dithianyl, thioxanyl, homopiperazinyl, homopiperidinyl, oxacycloheptanyl, thiocycloheptanyl, oxazepinyl, diazepinyl, thiazepinyl, indolinyl, 1,2,3,4-tetrahydroisoquinolinyl, 1,3-benzodioxolyl, and 2-oxa-5-azabicyclo[2.2.1]hept-5-yl. Examples of substitution of the -CH₂-group in the heterocyclyl with -C(=O)- include, but are not limited to, 2-oxopyrrolidinyl, oxo-1,3-thiazolidinyl, 2-piperidinonyl, 3,5-dioxopiperidinyl, and pyrimidinedionyl. Examples of oxidization of sulfur atoms in the heterocyclyl include, but are not limited to, sulfolanyl and 1,1-dioxothiomorpholinyl. Bridged heterocyclyl groups include, but are not limited to, 2-oxabicyclo[2.2.2]octyl, 1-azabicyclo[2.2.2]octyl, 3-azabicyclo[3.2.1]octyl, and the like. The heterocyclyl group may be optionally substituted by one or more substituents described in the present disclosure.

The term "bridge" refers to a bond, an atom, or an unbranched chain of atoms linking two different parts of a molecule. Two atoms (usually, but not always, two tertiary carbon atoms) linked by a bridge are denoted as "bridgeheads".

The term "spiro" refers to a ring system with one atom (usually a quaternary carbon atom) as the only common atom between two rings.

The term "consisting of n atoms", n being an integer, typically describes a quantity of ring-forming atoms in a molecule, that is, the quantity of ring-forming atoms in the molecule is n. For example, piperidinyl is heterocyclyl consisting of six atoms, and 1,2,3,4-tetrahydronaphthalene is a carbocyclyl group consisting of 10 atoms.

The term "heteroatom" refers to O, S, N, P, and Si, including any oxidized N, S, and P, a form of primary, secondary, tertiary amines and quaternary ammonium salts, or a form of substituted hydrogen on the nitrogen atom in the heterocyclic ring, such as N (N in 3,4-dihydro-2H-pyrrolyl), NH (NH in pyrrolidinyl), or NR (NR in N-substituted pyrrolidinyl).

The term "halogen" refers to F, Cl, Br, or I.

The term "N₃" denotes an azide structure. This group can be linked to another group, for example, to a methyl group to form methyl azide (MeN₃) or to a phenyl group to form benzene azide (PhN₃).

The term "aryl" may be used alone or as a large part of "aralkyl", "aralkoxy", or "aryloxyalkyl", indicating monocyclic, bicyclic, and tricyclic carbocyclic ring systems containing 6-14 ring atoms, 6-12 ring atoms, or 6-10 ring atoms. At least one ring system is aromatic. Each ring system includes a ring consisting of 3-7 atoms and includes one or more attachment points linked to the rest of the molecule. The term "aryl" is interchangeable with the term "aromatic ring" or "aryl ring", for example, the aromatic ring may include phenyl, naphthyl, and anthryl. The aryl group may be independently unsubstituted or substituted by one or more substituents described in the present disclosure.

The term "heteroaryl" may be used alone or as a large part of "heteroarylalkyl" or "heteroarylalkoxy", indicating monocyclic, bicyclic, and tricyclic ring systems containing 5-14 ring atoms, 5-12 ring atoms, 5-10 ring atoms, or 5-6 ring atoms. At least one ring system is aromatic, and at least one ring system includes one or more heteroatoms. Each ring system includes a ring consisting of 5-7 atoms and includes one or more attachment points linked to the rest of the molecule. Unless otherwise indicated, the heteroaryl may be carbon-based or nitrogen-based, and the -CH₂- group may be optionally substituted by -C(=O)-. The sulfur atom of the ring may be optionally oxidized to an S-oxide. The nitrogen atom of the ring may be optionally oxidized to an *N*-oxide. The term "heteroaryl" is interchangeable with the term "heteroaromatic ring" or "heteroaromatic compound". In some implementations, the heteroaryl is heteroaryl consisting of 5-12 atoms including one, two, three, or four heteroatoms independently selected from O, S, and N. In some other implementations, the heteroaryl is heteroaryl consisting of 5-10 atoms including one, two, three, or four heteroatoms independently selected from O, S, and N. In some other implementations, the heteroaryl is heteroaryl consisting of 5-6 atoms including one, two, three, or four heteroatoms independently selected from O, S, and N. In some other implementations, the heteroaryl is heteroaryl consisting of 5 atoms including one, two, three, or four heteroatoms independently selected from O, S, and N. In some other implementations, the heteroaryl is heteroaryl consisting of 6 atoms including one, two, three, or four heteroatoms independently selected from O, S, and N.

In some other embodiments, the heteroaryl includes, but is not limited to, the following monocyclic groups: 2-furanyl, 3-furanyl, *N*-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, N-pyrrolyl, 2-pyrrolyl, 3-pyrrolyl, 2-pyridinyl, 3-pyridinyl, 4-pyridinyl, 2-pyrimidinyl, 4-pyrimidinyl, 5-pyrimidinyl, pyridazinyl (such as 3-pyridazinyl), 2-thiazolyl, 4-thiazolyl, 5-thiazolyl, tetrazolyl (such as 5*H*-tetrazolyl and 2*H*-tetrazolyl), triazolyl (such as 2-triazolyl, 5-triazolyl, 4*H*-1,2,4-triazolyl, 1*H*-1,2,4-triazolyl, and 1,2,3-triazolyl), 2-thienyl, 3-thienyl, pyrazolyl (such as 2-pyrazolyl and 3-pyrazolyl), isothiazolyl, 1,2,3-oxadiazolyl, 1,2,5-oxadiazolyl, 1,2,4-oxadiazolyl, 1,3,4-oxadiazolyl, 1,2,3-thiodiazolyl, 1,3,4-thiodiazolyl, 1,2,5-thiodiazolyl, pyrazinyl, and 1,3,5-triazinyl; and also includes, but is not limited to, the following bicyclic groups: benzimidazolyl, benzofuranyl, benzothienyl, indolyl (such as 2-indolyl), purinyl, quinolinyl (such as 2-quinolinyl, 3-quinolinyl, and 4-quinolinyl), isoquinolinyl (such as 1-isoquinolinyl, 3-isoquinolinyl, or 4-isoquinolinyl), oxathiyl, 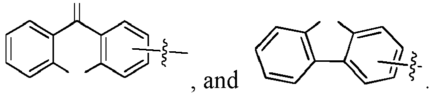 The heteroaryl group may be optionally substituted by one or more substituents described in the present disclosure.

The term "carboxyl", whether used alone or in conjunction with other terms such as "carboxyalkyl", denotes -CO₂H. The term "carbonyl", whether used alone or in conjunction with other terms such as "aminocarbonyl" or "acyloxy", denotes -(C=O)-.

The term "alkylamino" includes "*N*-alkylamino" and "*N*,*N*-dialkylamino", where the amino group is independently substituted with one or two alkyl groups. In some embodiments, the alkylamino is a lower-priority alkylamino group in which one or two C₁₋₆ alkyl groups are linked to the nitrogen atom. In some other embodiments, the alkylamino is a lower-priority C₁₋₃ alkylamino group. A proper alkylamino group may be monoalkylamino or dialkylamino. Such examples include, but are not limited to, *N*-methylamino, *N*-ethylamino, *N*,*N*-dimethylamino, *N*,*N*-diethylamino, and the like.

The term "arylamino" indicates that an amino group is substituted with one or two aryl groups. Such examples include, but are not limited to, *N*-phenylamino. In some embodiments, an aryl ring on the arylamino may be further substituted.

The term "aminoalkyl" includes a straight or branched C₁₋₁₀ alkyl group substituted with one or more amino groups. In some embodiments, the aminoalkyl is "lower-priority C₁₋₆ aminoalkyl" substituted with one or more amino groups. Such examples include, but are not limited to, aminomethyl, aminoethyl, aminopropyl, aminobutyl, and aminohexyl.

As described in the present disclosure, a substituent is linked to a center ring with a bond to form a ring system, indicating that the substituent can be substituted at any substitutable position. This ring system includes a monocyclic, bicyclic, or polycyclic system.

The term "unsaturated" used in the present disclosure indicates that a group has one or more degrees of unsaturation.

The term "comprise" or "include" is open-ended expression, that is, includes the content specified in the present disclosure, but does not exclude the content in other aspects.

As described in the present disclosure, the term "pharmaceutically acceptable carrier" includes any solvent, dispersion medium, coating material, surfactant, antioxidant, preservative (such as an antibacterial agent and an antifungal agent), isotonic agent, salt, drug stabilizer, binder, excipient, dispersing agent, lubricant, sweetener, flavorant, colorant, or combination thereof. These carriers are known to a person skilled in the art (as described in Remington's Pharmaceutical Sciences, 18th Ed. Mack Printing Company, 1990, pp. 1289-1329). Except for a carrier that is incompatible with the active ingredient, any conventional carriers that can be used in treatment or in a pharmaceutical composition are encompassed.

The term "inhibiting M^{pro} or inhibiting coronavirus replication" used in the present disclosure includes reducing the expression or activity of M^{pro} or reducing coronavirus replication (for example, a reduction of at least 10%), and completely inhibiting the expression or activity of M^{pro} or completely inhibiting coronavirus replication (that is, 100% inhibition). In some implementations, the expression or activity of M^{pro} or the coronavirus replication is inhibited by at least 50%, at least 65%, at least 75%, at least 85%, at least 90%, or at least 95%.

The term "effective amount" of the compound of the present disclosure refers to an amount that elicits an expected biological response. In the present disclosure, the expected biological response is inhibition of M^{pro}, prevention of recurrence, evolution, onset, or progression of symptoms associated with a coronavirus infection, or enhancement or improvement of prophylactic or therapeutic effects of another coronavirus infection therapy used. An exact amount of the compound to be administered to a treated object depends on an administration mode, severity, and characteristics of the treated object such as health status, age, gender, weight, and tolerance to the drug. A technician can determine a proper dose based on these and other factors. When administered in combination with other therapeutic agents, for example, at least one of other drugs against coronaviruses, drugs suppressing cytokine storms, drugs boosting immunity, and drugs regulating intestinal flora, the "effective amount" of the second agent depends on the type of the drug being used. Proper dosages of approved agents are known and may be adjusted by a technician based on the condition of a treated object, the type of symptoms, and the amount of the compound of the present disclosure that is used. In the case of an unspecified amount, the effective amount can be adopted. For example, the compound of the present disclosure may be administered to a treated object for therapeutic or prophylactic treatment in a dose range of about 0.01-100 mg/weight/day.

The term "treatment" used in the present disclosure refers to both therapeutic treatment and prophylactic treatment. For example, the therapeutic treatment includes reducing or ameliorating the progression, severity, and/or duration of symptoms of a coronavirus infection, or ameliorating one or more symptoms (in particular, one or more recognizable symptoms) of a coronavirus infection, as a result of the application of one or more therapies (for example, the administration of one or more therapeutic agents (such as the compound and composition in the present disclosure)). In a particular implementation, the therapeutic treatment includes improving at least one measurable physical parameter of the symptom of the coronavirus infection. In other implementations, the therapeutic treatment includes inhibiting the progression of the symptom of the coronavirus infection by (for example) stabilizing the recognizable symptom physically or by (for example) stabilizing the physical parameter physiologically or physically. In other implementations, the therapeutic treatment includes alleviating or stabilizing a disease of the coronavirus infection, such as respiratory distress.

The term "protecting group" or "PG" refers to a substituent that reacts with other functional groups to block or protect specific functionality. For example, the "amino-protecting group" refers to a substituent linked to an amino group to block or protect the functionality of the amino group in a compound. Proper amino-protecting groups include acetyl, trifluoroacetyl, tert-butoxycarbonyl (BOC, Boc), benzyloxycarbonyl (CBZ, Cbz), and 9-fluorenylmethoxycarbonyl (Fmoc). Similarly, the "hydroxyl-protecting group" refers to a substituent of hydroxyl to block or protect the functionality of the hydroxyl. Proper protecting groups include acetyl and methylsilyl. The "carboxyl-protecting group" refers to a substituent of carboxyl to block or protect the functionality of the carboxyl. The carboxyl-protecting groups usually include -CH₂CH₂SO₂Ph, cyanoethyl, 2-(trimethylsilyl)ethyl, 2-(trimethylsilyl)ethoxymethyl, 2-(p-toluenesulfonyl)ethyl, 2-(p-nitrophenylsulfonyl)ethyl, 2-(diphenylphosphinyl)ethyl, nitroethyl, and the like. For general descriptions of protecting groups, refer to the following literature: T W. Greene, Protective Groups in Organic Synthesis, John Wiley&Sons, New York, 1991; and P. J. Kocienski, Protecting Groups, Thieme, Stuttgart, 2005.

### Description of Compound of The Present Disclosure

The genomic data shows that the novel coronavirus is mainly translated into two multimeric proteins: pp1a and pp1ab. These multimeric proteins are hydrolyzed by the main protease (M^{pro}), also referred to as 3C-like protease (3CL pro), and papain-like protease (PL Pro) into functional proteins. These proteases are necessary for replication of viruses such as RdRp or nsp13. Without these functional proteins being released by hydrolysis, viruses cannot complete replication. Therefore, the inhibition of the function of M^{pro} of viruses can stop viral replication in the human body. In addition, M^{pro} is conserved in coronaviruses. For example, there are 96% identity and 100% identity between SARS-Cov-2 and SARS-Cov-1 respectively on a main protease sequence and active sites. It has been reported that coronavirus M^{pro} can specifically cleave the multimeric protein pp1ab (790 kDa) at 11 cleavage sites, whereas none of the human proteases have similar cleavage specificity. This indicates that high selectivity and low toxicity of drugs can be achieved by targeting M^{pro} of viruses, making M^{pro} one of the highly desirable antiviral targets. Therefore, it is necessary to develop an effective inhibitor against viral M^{pro}.

The present disclosure provides a macrocyclic peptidomimetic inhibitor with a novel structure that can be reversibly covalently bound to novel coronavirus M^{pro}, and a preparation method and use thereof. Such compound shows strong inhibitory activity against M^{pro}, and therefore has the potential to be used as a novel M^{pro} inhibitor for the prevention and treatment of novel coronavirus infections.

According to a first aspect of the present disclosure, the present disclosure provides a compound, being a compound shown in formula (I) or a stereoisomer, a tautomer, a nitrogen oxide, a solvate, a metabolite, a pharmaceutically acceptable salt, or a prodrug of the compound shown in formula (I), 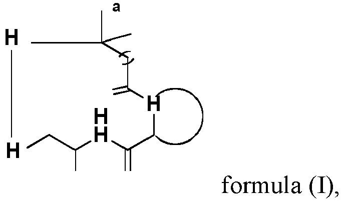
L₁ being a linker;
L₂ being -(CH₂)ₘCONR₁- or 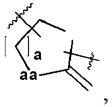 Y being C or N;
R₁ being H, F, Cl, Br, CN, NO₂, -OR^{b}, -NR^{c}R^{d}, or C₁₋₆ alkyl;
R₂ₐ and R_{2b} being each independently H, -C(=O)R^{a}, -C(=O)OR^{b}, -S(=O)₂R^{b}, - C(=O)NR^{c}R^{d}, -NC(=O)R^{c}R^{d}, -OR^{b}, -NR^{c}R^{d}, R^{b}0-C₁₋₄ alkylene, R^{d}R^{c}N-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, and (heteroaryl consisting of 5-10 atoms)-C₁₋₄ alkylene being each independently unsubstituted or substituted with one, two, three, or four substituents, the substituent being independently F, Cl, Br, CN, =O, -OR^{b}, -NR^{c}R^{d}, C₁₋₆ alkyl, C₁₋₆ haloalkyl, R^{b}0-C₁₋₄ alkylene, or R^{d}R^{c}N-C₁₋₄ alkylene, and R₂ₐ and R_{2b} being not all H;
ring A being optionally heterocyclyl consisting of 3-10 atoms or heteroaryl consisting of 5-10 atoms that is substituted with one or more R';
Z being cyano, aziridine, epoxypropane, trifluoromethyl ketone, aldehyde, - CH(O(C=O)R₃)SO₃Na, -CH(O(C=O)OR₃)SO₃Na, -CH(O(C=O)NHR₃)SO₃Na, - (C=O)(C=O)NHR₃, -(C=O)(P=O)(OR₃), -(C=O)COOR₃, acetoxymethyl ketone, Michael receptor, or substituted or unsubstituted heterocyclyl consisting of 3-6 atoms or heteroaryl consisting of 5-10 atoms, R₃ being H, F, Cl, Br, CN, NO₂, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene;
m being an integer between 1 and 6; n being 1 or 2; k being 0 or 1;
R' being independently H, F, Cl, Br, CN, NO₂, =O, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, heterocyclyl consisting of 3-12 atoms, C₆₋₁₀ aryl, or heteroaryl consisting of 5-10 atoms, C₃₋₈ cycloalkyl, heterocyclyl consisting of 3-12 atoms, C₆₋₁₀ aryl, or heteroaryl consisting of 5-10 atoms being independently unsubstituted or substituted with one, two, three, or four substituents, and the substituent being independently F, Cl, Br, CN, =O, -OR^{b}, -NR^{c}R^{d}, C₁₋₆ alkyl, C₁₋₆ haloalkyl, R^{b}0-C₁₋₄ alkylene, or R^{d}R^{c}N-C₁₋₄ alkylene; and
R^{a}, R^{b}, R^{c}, and R^{d} being each independently H, C₁₋₆ alkyl, C₁₋₆ haloalkyl, heterocyclyl consisting of 3-6 atoms, C₆₋₁₀ aryl, heteroaryl consisting of 5-10 atoms, or heterocycle consisting of 3-6 atoms that is formed by R^{c}, R^{d}, and nitrogen atoms linked to R^{c} and R^{d}, C₁₋₆ alkyl, heterocycle consisting of 3-6 atoms, C₆₋₁₀ aryl, and heteroaryl consisting of 5-10 atoms being each independently unsubstituted or substituted with one, two, three, or four substituents, and the substituent being independently F, Cl, CN, OH, NH₂, C₁₋₆ alkyl, C₁₋₆ haloalkyl, C₁₋₆ alkoxy, or C₁₋₆ alkylamino.

According to an embodiment of the present disclosure, the compound is a compound shown in formula (II) or formula (III) or a stereoisomer, a tautomer, a nitrogen oxide, a solvate, a metabolite, a pharmaceutically acceptable salt, or a prodrug of the compound shown in formula (II) or formula (III),

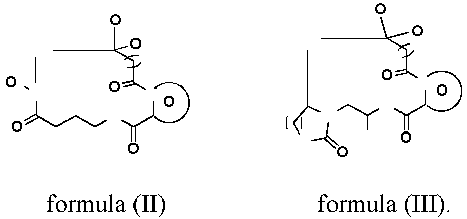

R₂ₐ, R_{2b}, k, R₁, n, z, and ring A are defined in the present disclosure as above.

According to an embodiment of the present disclosure, the compound is a compound shown in formula (IV) or formula (V) or a stereoisomer, a tautomer, a nitrogen oxide, a solvate, a metabolite, a pharmaceutically acceptable salt, or a prodrug of the compound shown in formula (IV) or formula (V),

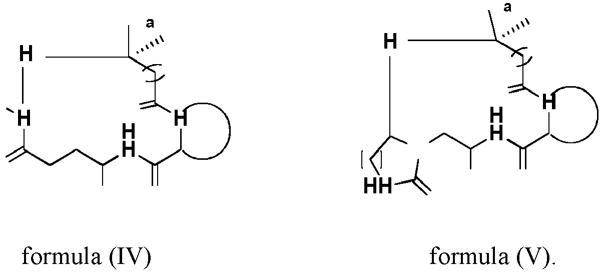

R₂ₐ, R_{2b}, k, R₁, n, z, and ring A are defined in the present disclosure as above.

According to an embodiment of the present disclosure, the compound is a compound shown in formula (VI) or formula (VII) or a stereoisomer, a tautomer, a nitrogen oxide, a solvate, a metabolite, a pharmaceutically acceptable salt, or a prodrug of the compound shown in formula (VI) or formula (VII),

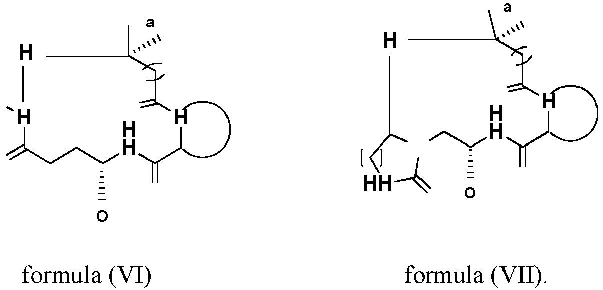

R₂ₐ, R_{2b}, k, R₁, n, z, and ring A are defined in the present disclosure as above.

According to an embodiment of the present disclosure, the compound is a compound shown in formula (VIII) or formula (IX) or a stereoisomer, a tautomer, a nitrogen oxide, a solvate, a metabolite, a pharmaceutically acceptable salt, or a prodrug of the compound shown in formula (VIII) or formula (IX),

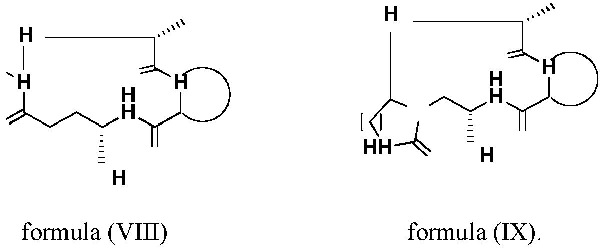

R₁, n, and ring A are defined in the present disclosure as above. R_{2b} is -C(=O)R^{a}, -C(=O)OR^{b}, -S(=O)₂R^{b}, -C(=O)NR^{c}R^{d}, -NC(=O)R^{c}R^{d}, -OR^{b}, -NR^{c}R^{d}, R^{b}0-C₁₋₄ alkylene, R^{d}R^{c}N-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, and (heteroaryl consisting of 5-10 atoms)-C₁₋₄ alkylene are each independently unsubstituted or substituted with one, two, three, or four substituents, and the substituent is independently F, Cl, Br, CN, =O, -OR^{b}, -NR^{c}R^{d}, C₁₋₆ alkyl, C₁₋₆ haloalkyl, R^{b}0-C₁₋₄ alkylene, or R^{d}R^{c}N-C₁₋₄ alkylene.

According to an embodiment of the present disclosure, the compound is a compound shown in formula (X) or formula (XI) or a stereoisomer, a tautomer, a nitrogen oxide, a solvate, a metabolite, a pharmaceutically acceptable salt, or a prodrug of the compound shown in formula (X) or formula (XI),

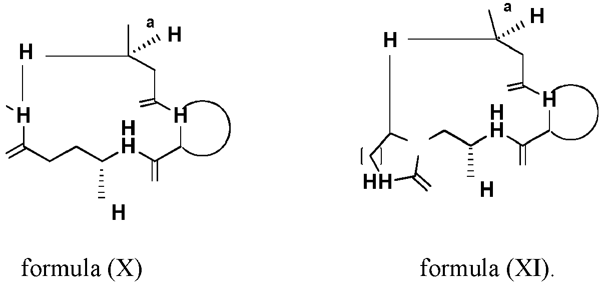

R₁, n, and ring A are defined in the present disclosure as above. R₂ₐ is -C(=O)R^{a}, -C(=O)OR^{b}, -S(=O)₂R^{b}, -C(=O)NR^{c}R^{d}, -NC(=O)R^{c}R^{d}, -OR^{b}, -NR^{c}R^{d}, R^{b}0-C₁₋₄ alkylene, R^{d}R^{c}N-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, and (heteroaryl consisting of 5-10 atoms)-C₁₋₄ alkylene are each independently unsubstituted or substituted with one, two, three, or four substituents, and the substituent is independently F, Cl, Br, CN, =O, -OR^{b}, -NR^{c}R^{d}, C₁₋₆ alkyl, C₁₋₆ haloalkyl, R^{b}O-C₁₋₄ alkylene, or R^{d}R^{c}N-C₁₋₄ alkylene.

According to an embodiment of the present disclosure, L₁ is a chain linker, and an atomic number of a main chain of the chain linker is 5, 6, 7, 8, 9, or 10, preferably, the atomic number of the main chain of the chain linker is 6.

According to an embodiment of the present disclosure, L₁ is optionally substituted -Rₑ-, -RₑCH(OH)R_{f}-, -RₑC(=O)R_{f}-, -RₑC(=O)OR_{f}-, -RₑOC(=O)R_{f}-, -RₑC(=O)SR_{f}-, -RₑSC(=O)R_{f}-, -RₑC(=O)N(R_{g})R_{f}-, -RₑN(R_{g})C(=O)R_{f}-, -RₑS(=O)₂N(R_{g})R_{f}-, - RₑN(R_{g})S(=O)₂R_{f}-, -RₑN(R_{g})C(=O)OR_{f}-, -RₑOC(=O)N(R_{g})R_{f}-, -RₑOR_{f}-, -RₑNR_{f}-, -RₑN=R_{f}-, - Rₑ-O-O-R_{f}-, -RₑSR_{f}-, -Rₑ-S-S-R_{f}-, -Rₑ-S-S-S-R_{f}-, -Rₑ-Rₕ-R_{f}-, or -Rₑ-O-Rₕ-R_{f}-, R_{g} being -H or substituted or unsubstituted alkyl, Rₑ being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rₕ being C₃₋₈ cycloalkyl, heterocyclyl consisting of 3-12 atoms, C₆₋₁₀ aryl, or heteroaryl consisting of 5-10 atoms, and t1 and t2 being independently integers not less than 1.

According to an embodiment of the present disclosure, L₁ is optionally substituted -Rₑ-, -RₑS(=O)₂N(R_{g})R_{f}-, -RₑN(R_{g})S(=O)₂R_{f}-, -RₑOR_{f}-, -RₑNR_{f}-, -RₑN=R_{f}-, -Rₑ-Rₕ-R_{f}-, or -Rₑ-O-Rₕ-R_{f}-, R_{g} being -H or substituted or unsubstituted alkyl, Rₑ being -**H**H , C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rh being C₃₋₈ cycloalkyl, heterocyclyl consisting of 3-12 atoms, C₆₋₁₀ aryl, or heteroaryl consisting of 5-10 atoms, and t1 and t2 being independently integers not less than 1.

According to an embodiment of the present disclosure, L₁ is optionally substituted -Rₑ-, Rₑ is -**H**H -, and t1 is an integer not less than 1, such as 5, 6, 7, 8, 9, or 10, preferably 6.

According to an embodiment of the present disclosure, Rₑ is C₃-C₃₀ branched alkylene.

According to an embodiment of the present disclosure, Rₑ is C₂-C₃₀ straight-chain alkenylene.

According to an embodiment of the present disclosure, Rₑ is C₃-C₃₀ branched alkenylene.

According to an embodiment of the present disclosure, Rₑ is C₃-C₃₀ branched alkenylene.

According to an embodiment of the present disclosure, Rₑ is C₂-C₃₀ straight-chain alkynylene.

According to an embodiment of the present disclosure, Rₑ is C₃-C₃₀ branched alkynylene.

According to an embodiment of the present disclosure, L₁ is a bond.

According to an embodiment of the present disclosure, L₁ is optionally substituted -RₑS(=O)₂N(R_{g})R_{f}-, where R_{g} is -H or substituted or unsubstituted alkyl, Rₑ is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, and t1 and t2 are independently integers not less than 1, preferably t1 + t2 + 2 = 5, 6, 7, 8, 9, or 10, preferably 6.

According to an embodiment of the present disclosure, L₁ is optionally substituted -RₑN(R_{g})S(=O)₂R_{f}-, where R_{g} is -H or substituted or unsubstituted alkyl, Rₑ is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, and t1 and t2 are independently integers not less than 1, preferably t1 + t2 + 2 = 5, 6, 7, 8, 9, or 10, preferably 6.

According to an embodiment of the present disclosure, L₁ is optionally substituted -RₑOR_{f}-, where Rₑ is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, and t1 and t2 are independently integers not less than 1, preferably t1 + t2 +1 = 5, 6, 7, 8, 9, or 10, preferably 6.

According to an embodiment of the present disclosure, L₁ is optionally substituted -RₑNR_{f}-, where Rₑ is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, and t1 and t2 are independently integers not less than 1, t1 + t2 +1 = 5, 6, 7, 8, 9, or 10, preferably 6.

According to an embodiment of the present disclosure, L₁ is optionally substituted -RₑN=R_{f}-, where Rₑ is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, and t1 and t2 are independently integers not less than 1, t1 + t2 +1 = 5, 6, 7, 8, 9, or 10, preferably 6.

According to an embodiment of the present disclosure, L₁ is optionally substituted -Rₑ-Rₕ-R_{f}-, where Rₑ is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rₕ is C₃₋₈ cycloalkyl, heterocyclyl consisting of 3-12 atoms, C₆₋₁₀ aryl, or heteroaryl consisting of 5-10 atoms, and t1 and t2 are independently integers not less than 1, preferably a sum of the lesser quantity of atoms between linking sites of Rₕ and Rₑ or R_{f} (including the atom at the linking site) and the quantity of atoms on the main chain of Rₑ and R_{f} is 5, 6, 7, 8, 9, or 10, preferably 6.

According to an embodiment of the present disclosure, L₁ is optionally substituted -Rₑ-O-Rₕ-R_{f}-, where Rₑ is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} is -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rₕ is C₃₋₈ cycloalkyl, heterocyclyl consisting of 3-12 atoms, C₆₋₁₀ aryl, or heteroaryl consisting of 5-10 atoms, and t1 and t2 are independently integers not less than 1, preferably a sum of the lesser quantity of atoms between linking sites of Rₕ and O or R_{f} (including the atom at the linking site) and the quantity of atoms on the main chain of Rₑ and R_{f} is 4, 5, 6, 7, 8, or 9, preferably 5.

According to an embodiment of the present disclosure, the substituent of L₁ is F, Cl, Br, CN, NO₂, =O, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, heterocyclyl consisting of 3-12 atoms, C₆₋₁₀ aryl, or heteroaryl consisting of 5-10 atoms.

According to an embodiment of the present disclosure, a substituent of L₁ is F.

According to an embodiment of the present disclosure, a substituent of L₁ is Cl.

According to an embodiment of the present disclosure, a substituent of L₁ is Br.

According to an embodiment of the present disclosure, a substituent of L₁ is CN.

According to an embodiment of the present disclosure, a substituent of L₁ is NO₂.

According to an embodiment of the present disclosure, a substituent of L₁ is =O.

According to an embodiment of the present disclosure, a substituent of L₁ is - OR^{b}.

According to an embodiment of the present disclosure, a substituent of L₁ is - NR^{c}R^{d}.

According to an embodiment of the present disclosure, a substituent of L₁ is - S(=O)₂R^{b}.

According to an embodiment of the present disclosure, a substituent of L₁ is C₁₋₆ alkyl.

According to an embodiment of the present disclosure, a substituent of L₁ is C₁₋₆ haloalkyl.

According to an embodiment of the present disclosure, a substituent of L₁ is C₃₋₈ cycloalkyl.

According to an embodiment of the present disclosure, a substituent of L₁ is C₃₋₈ cycloalkenyl.

According to an embodiment of the present disclosure, a substituent of L₁ is heterocyclyl consisting of 3-12 atoms.

According to an embodiment of the present disclosure, a substituent of L₁ is C₆₋₁₀ aryl.

According to an embodiment of the present disclosure, a substituent of L₁ heteroaryl consisting of 5-10 atoms.

According to an embodiment of the present disclosure, R₂ₐ and R_{2b} are each independently H, -OR^{b}, or -NC(=O)R^{c}R^{d}, and R₂ₐ and R_{2b} are not all H.

According to an embodiment of the present disclosure, the ring A is optionally heterocyclyl consisting of 5-10 atoms that is substituted with one or more R'.

According to an embodiment of the present disclosure, Z is cyano.

According to an embodiment of the present disclosure, m is 1.

According to an embodiment of the present disclosure, R' is independently F, Cl, Br, C₁₋₆ alkyl, or C₁₋₆ haloalkyl.

According to another aspect, the present disclosure provides a pharmaceutical composition, including an effective amount of the compound above.

According to an embodiment of the present disclosure, the pharmaceutical composition further includes a pharmaceutically acceptable carrier, an adjuvant, a medium, or a combination thereof.

According to an embodiment of the present disclosure, the pharmaceutical composition further includes one or more therapeutic agents, and the therapeutic agent is at least one of other drugs against coronaviruses, drugs suppressing cytokine storms, drugs boosting immunity, and drugs regulating intestinal flora.

According to an embodiment of the present disclosure, the other drugs against coronaviruses include at least one of Paxlovid (Pfizer), Junshi VV116 (Junshi), VV993 (Junshi), Proxalutamide (Kintor Pharma), Azvudine (Genuine Biotech), S-217622 (Shionogi), and FB2001 (Frontier Biotechnologies).

According to an embodiment of the present disclosure, the drugs suppressing cytokine storms include at least one of Thalidomide, Fingolimod, leflunomide, chloroquine, hydroxychloroquine, GNS561, VPS34, Tocilizumab, and Sarilumab.

According to still another aspect, the present disclosure provides use of the compound above or the pharmaceutical composition above in preparation of drugs, the drugs being used for preventing coronavirus infection or treating or reducing associated diseases of patients caused by coronavirus infection.

According to an embodiment of the present disclosure, the coronavirus includes at least one of HCoV-229E, HCoV-OC43, HCoV-NL63, HCoV-HKU1, SARS-CoV, 2019-nCoV, and MERS-Cov.

According to yet still another aspect, the present disclosure provides use of the compound above or the pharmaceutical composition above in preparation of drugs, the drugs being used for inhibiting M^{pro} or inhibiting coronavirus replication.

In some implementations, the salts are pharmaceutically acceptable salts. The term "pharmaceutically acceptable" means that substances or compositions have to be chemically and/or toxicologically compatible with other ingredients including a formulation and/or mammals to be treated with the substances or compositions.

The compound of the present disclosure further includes other salts thereof. The other salts are not necessarily pharmaceutically acceptable salts, and can be used for preparation and/or purification of the compound of the present disclosure and/or for isolation of an intermediate of an enantiomer of the compound of the present disclosure.

Pharmaceutically usable acid addition salts can be formed from inorganic and organic acids, such as acetate, aspartate, benzoate, benzenesulfonate, bromide/hydrobromide, bicarbonate/carbonate, bisulfate/sulfate, camphorsulfonate, chloride/hydrochloride, chlorotheophylline, citrate, ethanedisulfonate, fumarate, gluceptate, gluconate, glucuronate, hippurate, hydriodate/iodide, hydroxyethyl sulfonate, lactate, lactobionate, lauryl sulfate, malate, maleate, malonate, mandelate, mesylate, methyl sulfate, naphthoate, naphthalenesulfonate, nicotinate, nitrate, octadecanoate, oleate, oxalate, palmitate, pamoate, phosphate/hydrogen phosphate/dihydrogen phosphate, polygalactonate, propionate, stearate, succinate, sulfosalicylate, tartrate, toluene sulfonate, and trifluoroacetate.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, hydroxyacetic acid, oxalic acid, maleic acid, malonic acid, succinic acid, fumaric acid, tartaric acid, citric acid, benzoic acid, mandelic acid, methanesulfonic acid, ethanesulfonic acid, p-toluenesulfonic acid, sulfosalicylic acid, and the like.

Pharmaceutically usable base addition salts can be formed from inorganic and organic alkalis.

Inorganic alkalis from which salts can be derived include, for example, ammonium salts and metals of groups I to XII of the periodic table. In some implementations, the salts are derived from sodium, potassium, ammonium, calcium, magnesium, iron, silver, zinc, and copper. Particularly proper salts include ammonium, potassium, sodium, calcium and magnesium salts.

Organic alkalis from which salts can be derived include primary, secondary, and tertiary amines, and substituted amines including naturally occurring substituted amines, cyclic amines, basic ion exchange resins, and the like. Some organic amines include, for example, isopropylamine, benzathine, cholinate, diethanolamine, diethylamine, lysine, meglumine, piperazine, and trometamol.

The pharmaceutically usable salt in the present disclosure may be synthesized from a parent compound and a basic or acidic part by conventional chemical methods. Generally, such salts may be prepared by reacting free acid forms of these compounds with stoichiometric amounts of proper alkalis (such as hydroxides, carbonates, and bicarbonates of Na, Ca, Mg, or K), or by reacting free alkali forms of these compounds with stoichiometric amounts of proper acids. Such reactions are usually carried out in water or organic solvents or mixtures thereof. Usually, in a proper case, a non-aqueous medium such as ether, ethyl acetate, ethanol, isopropanol, or acetonitrile is required. Lists of some other proper salts can be found in, for example, "Remington's Pharmaceutical Sciences", 20th edition, Mack Publishing Company, Easton, Pa., (1985) and "Handbook of Pharmaceutical Salts: Properties, Selection, and Use", Stahl and Wermuth (Wiley-VCH, Weinheim, Germany, 2002).

In addition, the compound of the present disclosure, including its salt, can also be obtained in a form of a hydrate, or includes other solvents for crystallization. The compound of the present disclosure can form a solvate with a pharmaceutically usable solvent (including water) inherently or by design. Therefore, the present disclosure is intended to include solvated and unsolvated forms.

Any structural formulas given in the present disclosure are also intended to indicate these compounds in unlabeled forms and in isotopic-labeled forms. An isotopic-labeled compound has the structure shown in the general formula given in the present disclosure, except that one or more atoms are substituted by atoms with the selected atomic weight or mass number. Exemplary isotopes may be introduced into the compounds of the present disclosure, including isotopes of hydrogen, carbon, nitrogen, oxygen, phosphorus, sulfur, fluorine, and chlorine, such as ²H, ³H, ¹¹C, ¹³C, ¹⁴C, ¹⁵N, ¹⁸F, ³¹P, ³²P, ³⁶S, ³⁷Cl, or ¹²⁵I.

In addition, the compound of the present disclosure includes compounds that are defined in the present disclosure and that are labeled by various isotopes, for example, those compounds in which radioisotopes such as ³H, ¹⁴C, and ¹⁸F are present, or compounds in which non-radioisotopes such as ²H and ¹³C are present. Such isotopic-labeled compounds may be used in metabolic studies (with ¹⁴C), reaction kinetics studies (for example, with ²H or ³H), and detection or imaging techniques, such as positron emission tomography (PET) or single photon emission computed tomography (SPECT) that includes determination of drug or substrate tissue distribution, or may be used in radiotherapy of patients. ¹⁸F-labeled compounds are particularly desirable for PET or SPECT studies. The isotopic-labeled compounds of formula (I) may be prepared by conventional techniques familiar to a person skilled in the art or by replacing originally used unlabeled reagents with proper isotopic-labeled reagents, as described in the embodiments and preparation processes in the present disclosure.

In addition, substitution with a heavy isotope, particularly deuterium (²H or D), can provide some therapeutic advantages that result from higher metabolic stability, for example, from an increased in vivo half-life, a reduced dosage requirement, or an improved therapeutic index. It is to be understood that deuterium in this context is used as a substituent of the compound of formula (I). The concentration of a heavy isotope, particularly deuterium, may be defined by an isotope enrichment factor. The term "isotope enrichment factor" used in the present disclosure refers to a ratio of an isotopic abundance to a natural abundance of a designated isotope. If a substituent of the compound of the present disclosure is designated as deuterium, the compound has, for each designated deuterium atom, at least 3500 (52.5% deuterium doping at each designated deuterium atom), at least 4000 (60% deuterium doping), at least 4500 (67.5% deuterium doping), at least 5000 (75% deuterium doping), at least 5500 (82.5% deuterium doping), at least 6000 (90% deuterium doping), at least 6333.3 (95% deuterium doping), at least 6466.7 (97% deuterium doping), at least 6600 (99% deuterium doping), or at least 6633.3 (99.5% deuterium doping) isotope enrichment factors. The pharmaceutically usable solvate in the present disclosure includes those solvates in which a crystallizing solvent may be isotopically substituted, such as D₂O, acetone-*d₆*, or DMSO-*d₆*.

### Composition, Formulation, and Administration of Compound of The present Disclosure

The present disclosure provides a pharmaceutical composition, including an effective amount of the compound of the present disclosure or stereoisomers thereof. According to a specific embodiment of the present disclosure, the pharmaceutical composition further includes at least one pharmaceutically acceptable carrier, diluent, adjuvant, or medium, and optionally other therapeutic and/or prophylactic ingredients. In some implementations, the pharmaceutical composition includes an effective amount of at least one pharmaceutically acceptable carrier, diluent, adjuvant, or medium.

The pharmaceutically acceptable carrier may contain inert ingredients that do not excessively inhibit the biological activity of the compound. The pharmaceutically acceptable carrier has to be biocompatible, for example, non-toxic, non-inflammatory, nonimmunogenic, or no other adverse reactions or side effects once administered to patients, and can be prepared by using standard pharmaceutical techniques.

As described in the present disclosure, the pharmaceutical composition or pharmaceutically acceptable composition in the present disclosure further includes a pharmaceutically acceptable carrier, adjuvant, or excipient, including, as applied in the present disclosure, any solvent, diluent, liquid excipient, dispersing agent, suspending agent, surfactant, isotonic agent, thickening agent, emulsifying agent, preservative, solid binder, or lubricant, as is proper for specific target dosage forms. Various carriers for formulating the pharmaceutically acceptable composition and well-known preparation methods thereof are disclosed in Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York. Except for conventional carrier media that are incompatible with the compound of the present disclosure, for example, those produce adverse biological effects or harmful interactions with any other components in the pharmaceutically acceptable composition, any other conventional carrier media and use thereof fall within the scope of the present disclosure.

Some examples of substances that can be used as the pharmaceutically acceptable carrier include, but are not limited to, ion exchangers, aluminum oxide, aluminum stearate, lecithin, serum proteins (such as human serum albumin), buffer substances (such as Tween 80, phosphates, glycine, sorbic acid, or potassium sorbate), partial glyceride mixtures of saturated vegetable fatty acids, water, salts or electrolytes (such as protamine sulfate, disodium hydrogen phosphate, potassium hydrogen phosphate, sodium chloride, or zinc salts), silica gel, magnesium trisilicate, polyvinylpyrrolidone, polyacrylate, waxes, polyethylenepolypropylene-oxide-block copolymers, methylcellulose, hydroxypropyl methyl cellulose, lanolin, sugars (such as lactose, glucose, and sucrose), starch (such as cornstarch and potato starch), cellulose and its derivatives (such as sodium carboxymethyl cellulose, ethyl cellulose, and cellulose acetate), powdered tragacanth gum, malt, gels, talc, excipients (such as cocoa butter and suppository waxes), oils (such as peanut, cottonseed, safflower, sesame, olive, corn, and soybean oils), glycols (such as propylene glycol or polyethylene glycol), esters (such as ethyl oleate and ethyl dodecanoate), agar, buffers (such as magnesium hydroxide and aluminum hydroxide), alginic acid, non-pyrogenic water, isotonic saline, Ringer's solution, ethanol and phosphate buffers, and other non-toxic compatible lubricants (such as sodium lauryl sulfate and magnesium stearate). In addition, at the discretion of a formulator, colorants, anti-adhesion agents, coating agents, sweeteners and flavor enhancers, preservatives and antioxidants may also be present in the composition.

The compound or composition of the present disclosure may be administered in any proper ways. The compound and pharmaceutically acceptable composition may be administered to humans or other animals orally, rectally, parenterally, intracisternally, intravaginally, intraperitoneally, topically (for example, by powder, ointment, or drops), or by oral or nasal sprays, depending on the severity of diseases being treated.

Liquid dosage forms for oral administration include, but are not limited to, pharmaceutically acceptable emulsions, microemulsions, solutions, suspending agents, syrups, and elixirs. In addition to the active compounds, the liquid dosage forms may include inert diluents commonly used in the art, for example, water or other solvents, solubilizers, and emulsifiers, such as ethanol, isopropanol, ethyl carbonate, ethyl acetate, benzyl alcohol, benzyl benzoate, propylene glycol, 1,3-butanediol, dimethylformamide, oils (especially cottonseed, peanut, corn, germ, olive, castor, and sesame oils), glycerin, tetrahydrofurfuryl alcohol, fatty acid esters of polyethylene glycol and sorbitan, and mixtures thereof. In addition to the inert diluents, the oral composition may also include adjuvants, such as wetting agents, emulsifying and suspending agents, sweeteners, flavorants, and flavor enhancers.

Injectable formulations, such as sterile injectable water or oil suspending agents, may be prepared by known techniques using proper dispersing or wetting agents and suspending agents. Sterile injectable formulations may also be sterile injectable solutions, suspending agents, or emulsions in non-toxic parenterally-acceptable diluents or solvents, for example, solutions in 1,3-butanediol. Water, Ringer's solution, U.S.P., and isotonic sodium chloride solution may be used in acceptable media and solvents. In addition, it is customary to use sterile non-volatile oils as solvents or suspension media. In this case, any odorless non-volatile oil may be used, including synthetic monoglycerides or diglycerides. Moreover, fatty acids, such as octadecenoic acid, are used for preparing injections.

For example, injectable formulations may be sterilized by filtration through a bacterial retention filter or by addition of a sterilizing agent in the form of a sterile solid composition that can be dissolved or dispersed in sterile water or other sterile injectable media prior to use.

In order to prolong the action of the compound or composition in the present disclosure, it is often desirable to slow the absorption of the compound by subcutaneous or intramuscular injection. This can be achieved by using liquid suspensions of poorly watersoluble crystals or amorphous substances. In this case, the absorption rate of the compound depends on its dissolution rate, and the dissolution rate depends on the crystal size and crystal form. Alternatively, the absorption of the compound administered parenterally is slowed down by dissolving or suspending the compound in an oil medium. Injectable storage forms are prepared by forming microcapsule matrices of the compound in biodegradable polymers such as polylactide-polyglycolic acid. Based on a compound-to-polymer ratio and properties of the particular polymer used, the release rate of the compound can be controlled. Examples of other biodegradable polymers include polyorthoester and polyanhydride. Injectable storage formulations may alternatively be prepared by sequestering the compound in liposomes or microemulsions compatible with body tissues.

Compositions for transrectal or vaginal administration are in particular suppositories prepared by mixing the compounds of the present disclosure with proper non-irritating excipients or carriers, such as cocoa butter, polyethylene glycol, or suppository waxes. The excipients or carriers are solid at ambient temperature but liquid at body temperature, and therefore melt in the rectal or vaginal cavity and release the active compound.

Oral solid dosage forms include capsules, tablets, pills, powders, and granules. In these solid dosage forms, the active compound is mixed with at least one inert and pharmaceutically acceptable excipient or carrier such as sodium citrate or dicalcium phosphate and/or a) fillers or swellers such as starch, lactose, sucrose, glucose, mannitol, and silicic acid, b) binders such as carboxymethylcellulose, alginate, gel, polyvinylpyrrolidone, sucrose, and Arabic gum, c) humectants such as glycerin, d) disintegrating agents such as agar, calcium carbonate, potato or tapioca starch, alginic acid, some silicates, and sodium carbonate, e) solution-blocking agents such as paraffin wax, f) absorption accelerators such as quaternary ammonium compounds, g) wetting agents such as cetearyl alcohol and glycerol monostearate, h) absorbents such as kaolin and bentonite, and i) lubricants such as talc, calcium stearate, magnesium stearate, solid polyethylene glycol, sodium lauryl sulfate, and mixtures thereof. The dosage form may also include buffers in the case of capsules, tablets, and pills.

Similar types of solid compositions may also be used as fillers in soft and hard gel capsules using excipients such as lactose or milk sugar and polymeric polyethylene glycol. Solid dosage forms of tablets, lozenges, capsules, pills, and granules may be prepared with coatings and shells, such as enteric coatings and other coatings well known in the pharmaceutical field. The solid dosage forms may optionally include emulsions and may also have properties of the composition to optionally release only the active ingredient in a delayed manner, or preferably, release in a part of the intestinal tract. Examples of embedded compositions that may be used include polymers and waxes. Similar types of solid compositions may also be used as fillers in soft and hard gel capsules using excipients such as lactose or milk sugar and polymeric polyethylene glycol.

Active compounds may also be presented in a micro-sealed form with one or more of the foregoing excipients. Solid dosage forms of tablets, lozenges, capsules, pills, and granules may be prepared with coatings and shells, such as enteric coatings, controlled-release coatings, and other coatings well known in the pharmaceutical field. In these solid dosage forms, the active compounds may be mixed with at least one inert diluent, such as sucrose, lactose, or starch. Usually, such dosage forms may also include additional substances other than the inert diluent, for example, tablet lubricants and other tablet adjuvants, such as magnesium stearate and microcrystalline cellulose. The dosage form may also include buffers in the case of capsules, tablets, and pills. The solid dosage forms may optionally include emulsions and may also have properties of the composition to optionally release only the active ingredient in a delayed manner, or preferably, release in a part of the intestinal tract. Examples of embedded compositions that may be used include polymers and waxes.

Dosage forms of the compound of the present disclosure for topical or transdermal administration include unguents, ointments, creams, lotions, gels, powders, solutions, sprays, inhalers, or patches. Under aseptic conditions, the active compounds with pharmaceutically acceptable carriers and any required preservatives or buffers that may be required, ophthalmic formulations, ear drops, and eye drops also fall within the scope of the present disclosure. In addition, the present disclosure includes the use of skin patches with the additional advantage of controlled delivery of the compound to the body. Such dosage forms may be prepared by dissolving or dispersing the compound in a proper medium. Absorption enhancers may also be used to increase the flow of the compound through the skin. The rate can be controlled by providing a rate control film or by dispersing the compound in a polymeric matrix or gel.

The composition of the present disclosure may also be administered orally, parenterally, by inhalation sprays topically, rectally, nasally, orally, vaginally, or by catheter indwelling. The term "parenteral" used in the present disclosure includes, but is not limited to, subcutaneous, intravenous, intramuscular, intra-articular, synovial cavity, intrasternal, intrathecal, intrahepatic, intra-lesional, and intracranial injection or infusion techniques. In particular, the composition is administered orally, intraperitoneally, or intravenously.

Sterile injectable forms of the composition of the present disclosure may be water or oil suspensions. These suspensions may be prepared following techniques known in the art using proper dispersing or wetting agents and suspending agents. Sterile injectable formulations may also be sterile injectable solutions or suspensions in non-toxic parenterally-acceptable diluents or solvents, for example, solutions in 1,3-butanediol. Water, Ringer's solution, and isotonic sodium chloride solution may be used in acceptable media and solvents. In addition, it is customary to use sterile non-volatile oils as solvents or suspension media. In this case, any odorless non-volatile oil may be used, including synthetic monoglycerides or diglycerides. In addition, fatty acids such as octadecenoic acid and glycerol ester derivatives thereof are used for preparing injections, just as naturally-occurring pharmaceuticallyacceptable oils, such as olive oil or castor oil, which are particularly in the form of polyoxyethylene. These oil solutions or suspensions may also contain long-chain alcohol diluents or dispersing agents, such as carboxymethyl cellulose or similar dispersing agents commonly used in the preparation of pharmaceutically acceptable dosage forms (including emulsions and suspensions). Other commonly used surfactants such as Tweens and Spans, and other emulsifiers or bioavailability enhancers commonly used in the production of pharmaceutically acceptable solid, liquid, or other dosage forms may also be used for preparation.

The pharmaceutical composition in the present disclosure may be administered orally in any orally acceptable dosage form, including but not limited to, capsules, tablets, aqueous suspensions, or solutions. In the case of tablets for oral administration, common carriers include, but are not limited to, lactose and starch. A lubricant, such as magnesium stearate, is also commonly added. For oral administration in the capsule form, useful diluents include lactose and dry corn starch. When oral administration requires an aqueous suspension, the active ingredient is combined with an emulsifier and a suspending agent. If required, some sweeteners, flavor enhancers, or colorants may also be added.

Alternatively, the pharmaceutical composition in the present disclosure may be administered in the form of a suppository for rectal administration. These pharmaceutical compositions may be prepared by mixing reagents and non-irritating excipients. The excipients are solid at room temperature but liquid at rectal temperature, and therefore melt in the rectum to release the drug. Such substances include, but are not limited to, cocoa butter, beeswax, and polyethylene glycol.

Particularly, when therapeutic targets include easily accessible regions or organs by topical administration in drops, including eye, skin, or lower intestinal diseases, the pharmaceutical composition in the present disclosure may also be administered topically. Proper topical formulations can be easily prepared for all these regions or organs.

Topical administration in drops to the lower intestine can be implemented with rectal suppository formulations (see above) or proper enema formulations. Topical skin patches may also be used.

For topical administration in drops, the pharmaceutical composition may be prepared as a proper ointment containing the active component suspended or dissolved in one or more carriers. Carriers for topical administration in drops of the compound of the present disclosure include, but are not limited to, mineral oil, vaseline oil, white vaseline, propylene glycol, polyoxyethylene, polyoxypropylene compounds, emulsifying waxes, and water. Alternatively, the pharmaceutical composition may be prepared as a proper lotion or cream containing the active component suspended or dissolved in one or more pharmaceutically acceptable carriers. Proper carriers include, but are not limited to, mineral oil, sorbitan monostearate, polysorbate 60, cetyl ester wax, cetearyl stearyl alcohol, 2-octyldodecanol, benzyl alcohol, and water.

For ophthalmic use, the pharmaceutical composition may be prepared, with or without a preservative such as benzalkonium chloride, as a micronized suspension in isotonic pH-adjusted sterile saline, or in particular as a solution in isotonic pH-adjusted sterile saline. Alternatively, for ophthalmic use, the pharmaceutical composition may be prepared as an ointment such as vaseline.

The pharmaceutical composition may also be administered by using a nasal aerosol or by inhalation. Such composition is prepared by using techniques well known in the pharmaceutical field and is prepared as a solution in saline with benzyl alcohol and other proper preservatives, absorption enhancers to improve bioavailability, fluorocarbons and/or other conventional solubilizers or dispersing agents.

The compound for use in the method of the present disclosure may be prepared into a unit dosage form. The term "unit dosage form" means a physically discrete unit for use as a unit dose for a treated object. Each unit contains a predetermined amount of active substance calculated to produce a desired therapeutic effect, optionally in combination with a proper pharmaceutical carrier. The unit dosage form may be used as one time of a single-time daily dose or a multiple-times daily dose (for example, about one to four or more times daily). When the multiple-times daily dose is used, the unit dosage form may be the same or different for each time.

### Use of Compound and Composition of The present Disclosure

The above compound and pharmaceutical composition provided in the present disclosure may be used in the preparation of drugs for preventing coronavirus infection or treating or reducing associated diseases of patients caused by coronavirus infections. For example, the associated disease caused by coronavirus infection is novel coronavirus pneumonia.

The present disclosure further provides use of the compound or the pharmaceutical composition thereof in preparation of drugs for inhibiting M^{pro} or inhibiting coronavirus replication.

The present disclosure provides a method for preventing coronavirus infection or treating or reducing associated diseases of patients caused by coronavirus infection. The method includes administering to an individual or patient in need of treatment a prophylactically or therapeutically effective amount of the compound or the pharmaceutical composition thereof. The associated disease caused by coronavirus infection is novel coronavirus pneumonia. In addition, the compound or the pharmaceutical composition thereof provided in the present disclosure may be co-administered with other therapies or therapeutic agents. The co-administration may be implemented simultaneously, sequentially, or at certain intervals.

The dosage of the compound or pharmaceutical composition required to produce a therapeutic, prophylactic, or delayed effect usually depends on a specific compound administered, a patient, a specific disease or condition and its severity, a route and frequency of administration, and the like, and needs to be determined by an attending physician based on a specific case. For example, the compound or pharmaceutical composition provided in the present disclosure may be administered via the transvenous route once a week or even at longer intervals.

In summary, the present disclosure provides a novel compound. The compound may be used as an M^{pro} or coronavirus replication inhibitor. The compound of the present disclosure is suitable to be prepared into drugs with various dosage forms, which can be widely used for preventing coronavirus infection or treating or reducing associated diseases of patients caused by coronavirus infection.

The compound and pharmaceutical composition of the present disclosure are beneficial for human treatment, and also can be used by veterinarians in the treatment of mammals including pets, introduced animals, and farm animals. Some other examples of animals include horses, dogs, and cats. Herein, the compound of the present disclosure includes pharmaceutically acceptable derivatives thereof.

### General Synthesis Process

The present disclosure is described in examples below. However, it is to be understood that the present disclosure is not limited to these examples, but merely provides methods for implementing the present disclosure.

Usually, the compound of the present disclosure may be prepared by the method described in the present disclosure. The substituent is defined as shown in formula (I) unless otherwise specified. The following reaction solutions and examples are for further exemplifying the present disclosure.

A person skilled in the art will recognize that: many other compounds of the present disclosure can be properly prepared based on the chemical reactions described herein, and other methods for preparing the compounds of the present disclosure are considered to be within the scope of the present disclosure. For example, the synthesis of those unexemplified compounds according to the present disclosure can be successfully implemented through modification by a person skilled in the art, for example, protecting an interfering group properly by using other known reagents in addition to those described in the present disclosure or by making some conventional modifications to reaction conditions. In addition, the reaction conditions disclosed in the present disclosure or known reaction conditions are also recognized to be applicable to the preparation of other compounds of the present disclosure.

In the examples described below, unless otherwise indicated, all temperatures are set at degrees Celsius. The solvents used in the present disclosure are commercially available. The reagents were purchased from commercial suppliers such as Aldrich Chemical Company, Arco Chemical Company and Alfa Chemical Company, J&K Scientific Ltd., and were used without further purification unless otherwise indicated.

For detection in the following examples, the LCMS models are Agilent 1200 Series, Agilent 1260 Infinity 2, and Agilent 1260-6125B, and the NMR model is the AVANCE 3 400MHZ Ultra shield TM Digital NMR.

The following abbreviations are used in the present disclosure:
- DIEA: N,N-diisopropylethylamine
- EDCI: 1-ethyl-(3-dimethylaminopropyl)carbodiimide
- HOBT: 1-hydroxybenzotriazole
- SGC: silica gel column chromatography
- HATU: 2-(7-azabenzotriazole)-N,N,N',N'-tetramethyluronium hexafluorophosphate
- DMF: N,N-dimethylformamide
- THF: tetrahydrofuran
- TFA: trifluoroacetic acid
- TFAA: trifluoroacetic anhydride
- TEA: triethylamine
- PE: petroleum ether at 60-90°C
- EA: ethyl acetate
- DCM: dichloromethane

The compounds are named according to conventional naming rules in the art or by using the ChemDraw software.

The following synthetic solution lists experimental steps of preparing the compound disclosed in the present disclosure.

### Synthetic solution I:

Ring A is defined in the present disclosure as above, and n' is an integer of 1-6. Ingredients IIa-2 and IIa-5 are used to form a linker. A person skilled in the art may carry out corresponding substitutions, heteroatom substitutions, or cyclic structure substitutions on the ingredient IIa-2 or IIa-5 to form a linker with corresponding substituents, heteroatom substituents, or cyclic structure substituents.

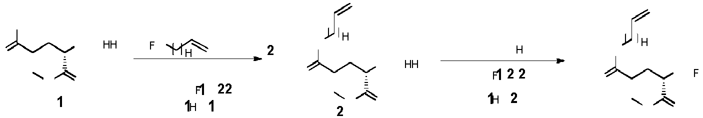

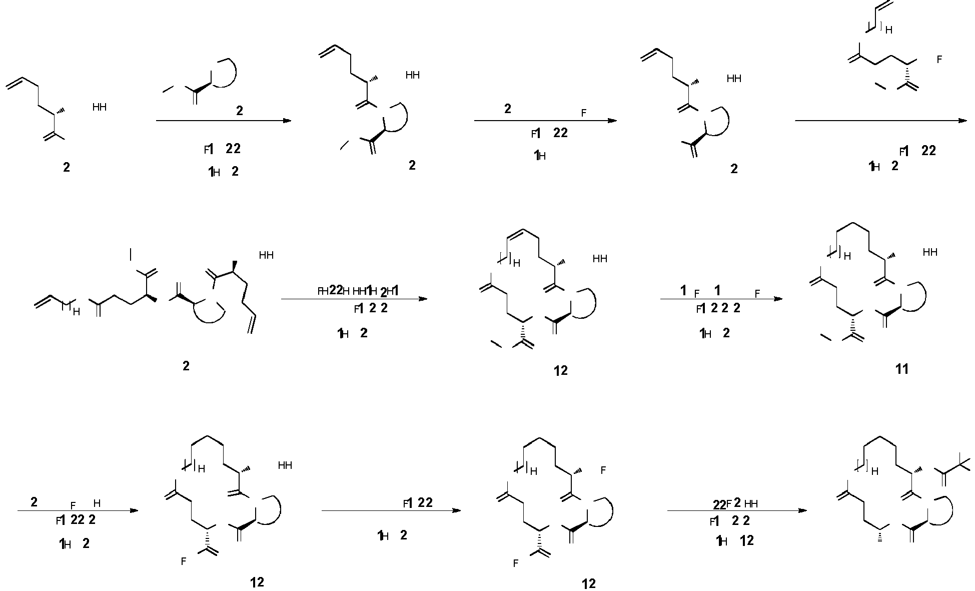

A starting ingredient IIa-1 and a substituted primary amine with an olefin terminal condense to obtain a compound IIa-3, and the compound Iia-3 is deprotected from the Boc group to obtain IIa-4. A compound IIa-5 or another substituted analog condenses with a cyclic amino acid ester to obtain IIa-7. The ester hydrolyzes and then condenses with IIa-4 to obtain a compound IIa-9. Then, the olefin in the molecule catalyzed by the Grubbs 2^{nd} catalyst underdoes the metathesis reaction to obtain a macrocyclic peptidomimetic compound IIa-10. IIa-10 is hydrogenated and reduced with platinum dioxide at atmospheric pressure and room temperature to obtain IIa-11. Then, IIa-11 undergoes ammonolysis to obtain IIa-12, and IIa-12 is deprotected from Boc to obtain a compound IIa-13. The primary amide compound IIa-13 dehydrates with trifluoroacetic anhydride, and the side-chain primary amine undergoes amidation to obtain a macrocyclic peptidomimetic target compound IIa.

### DESCRIPTION OF EMBODIMENTS

The following examples are intended to illustrate the present disclosure, but are not intended to limit the scope of the present disclosure.

### Preparation Examples

In the following preparation examples, the preparation processes of the compounds of the present disclosure are described in detail by using some of the compounds of the present disclosure as examples.

### Example 1

### Synthesis of N-((3S, 15S, 18aS, 19aR, 19bS)-3-cyano-19,19-dimethyl-1,6,16-trioxyeicosanylhydro-2H-cyclopropyl[3,4]pyrrole[1,2-a][1,4,9]triazocyclooctadecyl-15-yl)-2,2,2-trifluoroacetamide (compound 1)

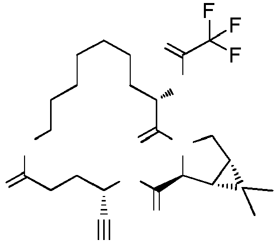

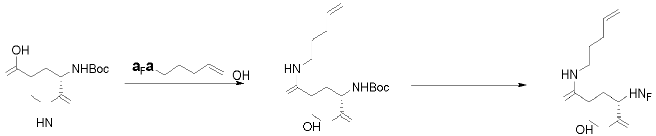

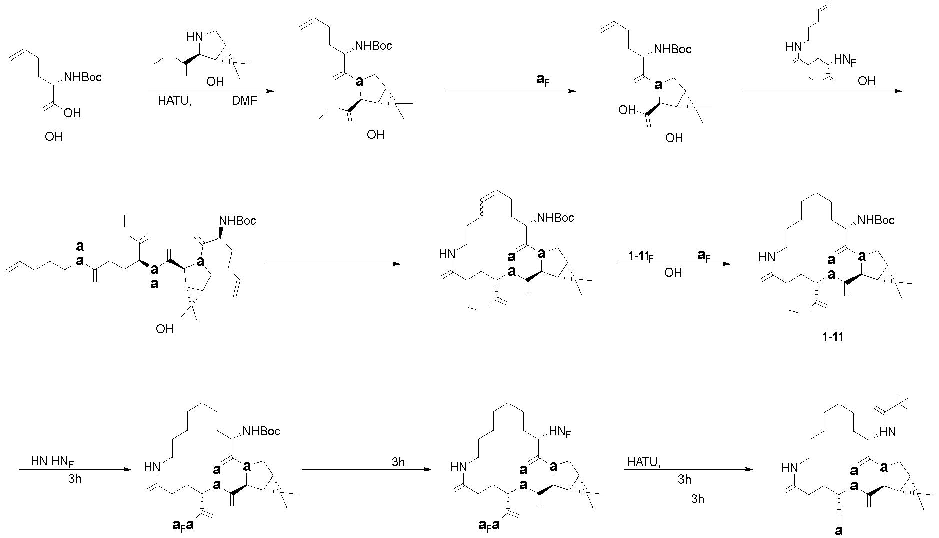

### Step 1

A compound 1-1 (2.0 g, 7.66 mmol) and a compound 1-2 (716 mg, 8.43 mmol) were dissolved in dichloromethane (30 mL), and DIEA (2.96 g, 22.98 mmol), HOBT (1.34 g, 9.96 mmol), and EDCI (1.9 g, 9.96 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 1-3 (1.3 g, yield: 52%). LCMS (M+Na)⁺ = 351.2.

### Step 2

The compound 1-3 (330 mg, 1 mmol) was dissolved in methanol (1 mL), a 4M hydrochloric acid methanol solution (2.5 mL, 10 mmol) was added at zero degrees to react for 2 h, and concentrated to remove the solvent, to obtain a colorless oily compound 1-4 (230 mg, yield: 100%). LCMS (M+H)⁺ = 229.3.

### Step 3

A compound 1-5 (3 g, 13.1 mmol), a compound 1-6 (2.43 g, 14.4 mmol), and DIEA (5.07 g, 39.3 mmol) were dissolved in DMF (30 mL), and HATU (6.47 g, 17.03 mmol) was added, to react at room temperature overnight. The reaction was quenched with water at zero degree, extracted with ethyl acetate, dried with saturated brine, dried with anhydrous sodium sulfate, and concentrated to remove the solvent, to obtain a crude product. The crude product was purified by SGC (PE:EA= 2: 1) to obtain a colorless oily compound 1-7 (3 g, yield: 60.2%). LCMS (M+Na)⁺ = 381.3.

### Step 4

The compound 1-7 (380 mg, 1 mmol) was dissolved in THF (10 mL) and added dropwise at zero degrees in LiOH (72 mg, 3 mmol) dissolved in water to react at room temperature for 2 h, adjusted with 1M hydrochloric acid to pH 4-5, extracted with ethyl acetate, dried with anhydrous sodium sulfate, and concentrated to remove the solvent, to obtain a colorless oily compound 1-8 (300 mg, yield: 79%). LCMS (M+Na)⁺ = 389.3.

### Step 5

The compound 1-8 (180 mg, 0.49 mmol) and the compound 1-4 (123 mg, 0.54 mmol) were dissolved in a mixed solvent of dichloromethane (5 mL) and DMF (5 mL), and DIEA (190 mg, 0.42 mmol), HOBT (105 mg, 0.64 mmol), and EDCI (122 mg, 0.64 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA= 2: 1) to obtain a colorless oily compound 1-9 (160 mg, yield: 57%). LCMS (M+H)⁺ = 577.2.

### Step 6

The compound 1-9 (110 mg, 0.19 mmol) was dissolved in dichloromethane (110 mL), and the Grubbs 2^{nd} catalyst (16 mg, 0.02 mmol) was added, to react at room temperature overnight. 1 mL of ethyl vinyl ether was added at zero degrees and stirred for 1 h for quenching. The reaction solution was filtered and concentrated to remove the solvent, to obtain a colorless oily compound 1-10a&b (90 mg, yield: 86%). LCMS (M+H)⁺ = 549.4.

### Step 7

The compound 1-10a&b (90 mg, 0.16 mmol) was dissolved in ethanol (20 mL), PtO₂ (18 mg, 0.08 mmol) was added, and hydrogen substitution was carried out three times, to react at room temperature for 0.5 h. The reaction solution was filtered and concentrated to remove the solvent, to obtain a colorless oily compound 1-11 (75 mg, yield: 85%). LCMS (M+H)⁺ = 551.3.

### Step 8

The compound 1-11 (75 mg, 0.14 mmol) was dissolved in 7M ammonia in methanol (20 mL), to react in a sealed tube at room temperature for 24 h. The reaction solution was concentrated to remove the solvent, to obtain a colorless oily compound 1-12 (70 mg, yield: 93%). LCMS (M+H)⁺ = 536.4.

### Step 9

The compound 1-12 (15 mg, 0.03 mmol) was dissolved in DCM (5 mL), and added dropwise in TFA (0.5 mL) in an ice-water bath. The reaction solution reacted at zero degrees for 3 h. The reaction solution was concentrated to remove the solvent, to obtain a colorless oily compound 1-13 (10 mg, yield: 77%). LCMS (M+H)⁺ = 436.3.

### Step 10

The compound 1-13 (10 mg, 0.02 mmol) was dissolved in THF (10 mL), and added dropwise in pyridine (16 mg, 0.2 mmol) in an ice-water bath. TFAA (13 mg, 0.06 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain a white solid compound 1 (7.2 mg, yield: 70%). LCMS (M+H)⁺ = 514.3. 1H NMR (400 MHz, DMSO) δ 9.60 (d, *J* = 6 Hz, 1H), 9.04 (d, *J* = 8.8 Hz, 1H), 7.60 (dd, *J* = 3.6 Hz, 7.6 Hz, 1H), 5.00-4.94 (m, 1H), 4.40-4.35 (m, 1H), 4.14 (s, 1H), 3.87-3.83 (m, 1H), 3.75 (d, *J* = 10 Hz, 1H), 3.40-3.36 (m, 1H), 2.81-2.78 (m, 1H), 2.24-2.04 (m, 4H), 1.83-1.80 (m, 2H), 1.57(dd, *J* = 5.2 Hz, 7.6 Hz, 1H), 1.46-1.41 (m, 2H), 1.34-1.23 (m, 9H), 1.02 (s, 3H), 0.91 (s, 3H).

### Example 2

### Synthesis of N-((3S, 15S, 18aS, 19aR, 19b5)-3-cyano-7,19,19-trimethyl-1,6,16-trioxyeicosanylhydro-2H-cyclopropyl[3,4]pyrrole[1,2-a][1,4,9]triazocyclooctadecene-15-yl)-2,2,2-trifluoroacetamide (compound 2)

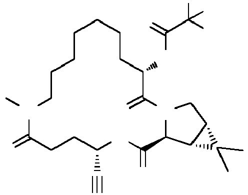

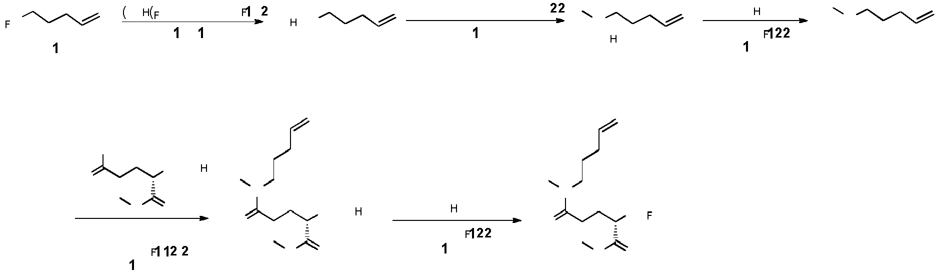

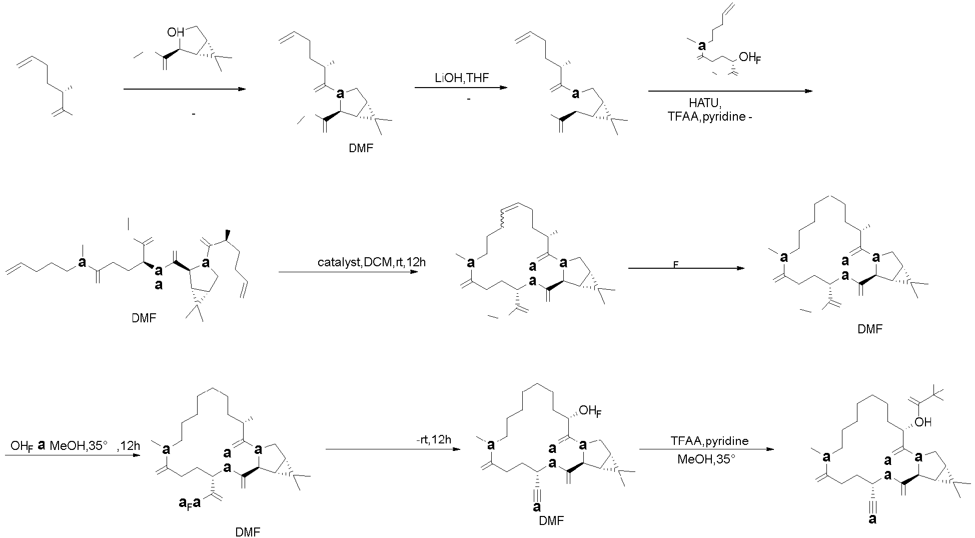

### Step 1

A compound 2-1 (2.0 g, 16.5 mmol) and TEA (3.56 g, 33.0 mmol) were dissolved in dichloromethane (30 mL), and (Boc)₂O (3.6 g, 22.98 mmol) was added dropwise at 0°C, to react at room temperature for 8 h. The reaction was quenched with water at zero degrees, extracted with ethyl acetate, dried with saturated brine, dried with anhydrous sodium sulfate, and concentrated to remove the solvent, to obtain a colorless oily compound 2-2 (1.6 g, yield: 52%). LCMS (M+Na)⁺ = 186.2.

### Step 2

The compound 2-2 (1.6 g, 8.6 mmol) was dissolved in THF (30 mL), and NaH (516 mg, 12.9 mmol) was added in batches at 0°C. CH₃I (6.1 g, 43 mmol) was added dropwise after stirring for 0.5 h, to react at 50°C for 2 h. The reaction was quenched with water at zero degrees, extracted with ethyl acetate, dried with saturated brine, dried with anhydrous sodium sulfate, and concentrated to remove the solvent, to obtain a crude product. The crude product was purified by SGC (DCM:MeOH = 50:1) to obtain a colorless oily compound 2-3 (1.6 g, yield: 93%). LCMS (M+Na)⁺ = 200.2.

### Step 3

The compound 2-3 (1.6 g, 8.0 mmol) was dissolved in EA (10 mL), a 4M hydrochloric acid ethyl acetate solution (20 mL) was added at zero degrees to react for 2 h, and concentrated to remove the solvent, to obtain a colorless oily compound 2-4 (800 mg, yield: 100%). LCMS (M+H)⁺ = 100.3.

### Step 4

The compound 2-4 (800 mg, 8.0 mmol) and a compound 2-5 (2.1 g, 8.0 mmol) were dissolved in dichloromethane (25 mL), and DIEA (5.2 g, 40 mmol), HOBT (1.3 g, 10.0 mmol), and EDCI (1.9 g, 10.0 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 2-6 (1.4 g, yield: 52%). LCMS (M+Na)⁺ = 343.1.

### Step 5

The compound 2-6 (1.4 g, 4.1 mmol) was dissolved in EA (10 mL), a 4M hydrochloric acid ethyl acetate solution (20 mL) was added at zero degrees to react for 2 h, and concentrated to remove the solvent, to obtain a colorless oily compound 2-7 (990 mg, yield: 100%). LCMS (M+H)⁺ = 243.3.

### Step 6

A compound 2-8 (3 g, 13.1 mmol), a compound 2-9 (2.43 g, 14.4 mmol), and DIEA (5.07 g, 39.3 mmol) were dissolved in DMF (30 mL), and HATU (6.47 g, 17.03 mmol) was added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 2-10 (3 g, yield: 60.2%). LCMS (M+Na)⁺ = 381.3.

### Step 7

The compound 2-10 (3.3 g, 7.9 mmol) was dissolved in THF (20 mL) and added dropwise at zero degrees in LiOH (569 mg, 23.7 mmol) dissolved in water to react at room temperature for 2 h, adjusted with 1M hydrochloric acid to pH 4-5, extracted with ethyl acetate, dried with anhydrous sodium sulfate, and concentrated to remove the solvent, to obtain a colorless oily compound 2-11 (3.2 g, yield: 100%). LCMS (M+Na)⁺ = 367.2.

### Step 8

The compound 2-11 (450 mg, 1.2 mmol) and the compound 2-7 (300 mg, 1.2 mmol) were dissolved in a mixed solvent of dichloromethane (5 mL) and DMF (5 mL), and DIEA(464 mg, 3.6 mmol), HOBT (195 mg, 1.2 mmol), and EDCI (267 mg, 1.4 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 2-12 (350 mg, yield: 49%). LCMS (M+Na)⁺ = 591.2.

### Step 9

The compound 2-12 (300 mg, 0.51 mmol) was dissolved in dichloromethane (100 mL), and the Grubbs 2^{nd} catalyst (16 mg, 0.05 mmol) was added, to react at room temperature overnight. 2 mL of ethyl vinyl ether was added at zero degrees and stirred for 1 h for quenching. The reaction solution was filtered and concentrated to remove the solvent, to obtain a colorless oily compound 1-13a&b (290 mg, yield: 94%). LCMS (M+H)⁺ = 563.4.

### Step 10

The compound 2-13a&b (100 mg, 0.18 mmol) was dissolved in ethanol (20 mL), PtO₂ (18 mg, 0.08 mmol) was added, and hydrogen substitution was carried out three times, to react at room temperature for 0.5 h. The reaction solution was filtered and concentrated to remove the solvent, to obtain a colorless oily compound 2-14 (90 mg, yield: 89%). LCMS (M+H)⁺ = 565.3.

### Step 11

The compound 2-14 (90 mg, 0.14 mmol) was dissolved in 7M ammonia in methanol (20 mL), to react in a sealed tube at room temperature for 24 h. The reaction solution was concentrated to remove the solvent, to obtain a colorless oily compound 2-15 (75 mg, yield: 97%). LCMS (M+H)⁺ = 550.4.

### Step 12

The compound 2-15 (35 mg, 0.06 mmol) was dissolved in DCM (5 mL), and added dropwise in TFA (0.5 mL) in an ice-water bath. The reaction solution reacted at zero degrees for 3 h. The reaction solution was concentrated to remove the solvent, to obtain a colorless oily compound 2-16 (20 mg, yield: 70%). LCMS (M+H)⁺ = 450.3.

### Step 13

The compound 2-16 (20 mg, 0.04 mmol) was dissolved in THF (10 mL), and added dropwise in pyridine (16 mg, 0.2 mmol) in an ice-water bath. TFAA (13 mg, 0.06 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain a white solid compound 2 (7.2 mg, yield: 70%).

LCMS (M+H)⁺ = 528.3. 1H NMR (400 MHz, DMSO) δ 9.71 (d, *J* = 4 Hz, 1H), 8.90 (d, *J* = 4.8 Hz, 1H), 4.97-4.92 (m, 1H), 4.42-4.34 (m, 1H), 4.17-4.15 (m, 1H), 4.13 (s, 1H), 3.86-3.78 (m, 2H), 2.81 (s, 3H), 2.58-2.54 (m, 2H), 2.41-2.33 (m, 2H), 2.17-2.03 (m, 1H), 1.85-1.75 (m, 2H), 1.57-1.54 (m, 1H), 1.48-1.19 (m, 5H), 1.14-1.10 (m, 6H), 1.02 (s, 3H), 0.92 (s, 3H).

### Example 3

### Synthesis of N-((11S, 14S, 15R, 3S, 13S)-13-cyano-16,16-dimethyl-2,10,15-trioxo-13,9,14-triaza-1(3,4)-bicyclo[3.1.0]hexane-4(1,3)-benzocyclopentane-3-yl)-2,2,2-trifluoroacetamide (compound 3)

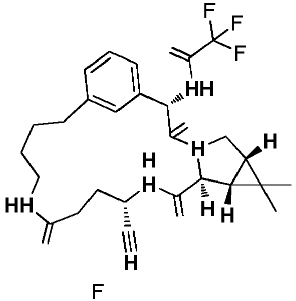

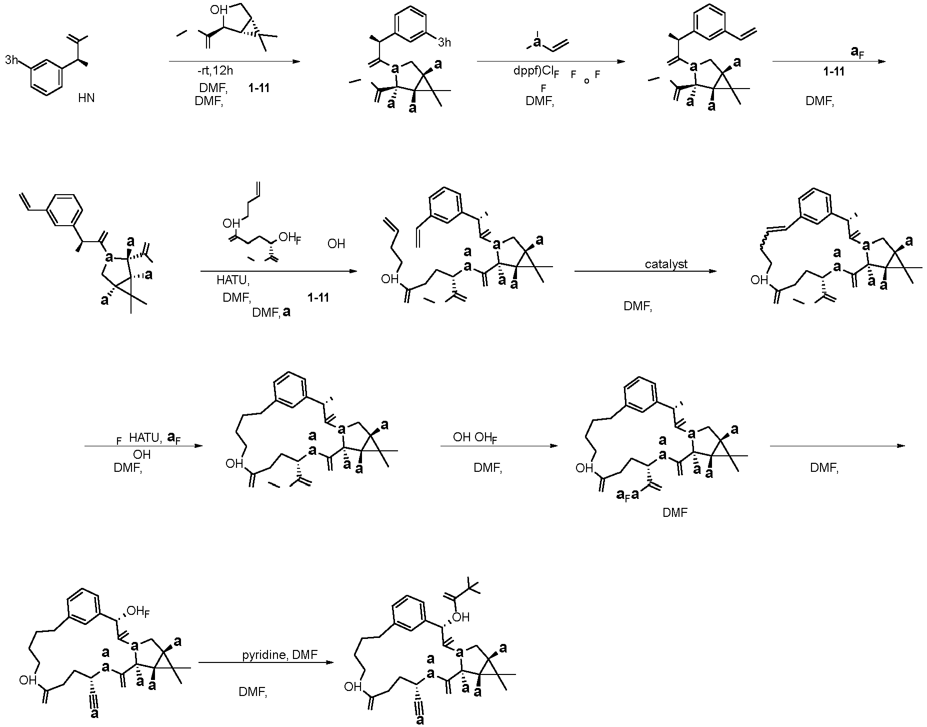

### Step 1

A compound 3-1 (400 mg, 1.2 mmol) and a compound 3-2 (245 mg, 1.2 mmol) were dissolved in dichloromethane (15 mL), and DIEA (497 mg, 3.6 mmol) and HATU (532 mg, 1.4 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 3-3 (350 mg, yield: 61%). LCMS (M+H)⁺ = 481.2.

### Step 2

The compound 3-3 (300 mg, 0.6 mmol) was dissolved in dioxane (3 mL) and water (1 mL). Potassium carbonate (166 mg, 1.2 mmol) and a compound 3-4 (65 mg, 0.9 mmol) were added. The catalyst Pd(dppf)Cl₂ (44 mg, 0.06 mmol) was added in a nitrogen atmosphere. The system was subjected to nitrogen displacement three times, heated to 100°C and stirred to react overnight, and concentrated to remove most of the solvent. Ethyl acetate was redissolved. The organic phase was washed with water, washed with saturated brine, dried with anhydrous sodium sulfate, and concentrated to remove the solvent, to obtain a crude product. The crude product was purified by SGC (PE:EA = 2:1) to obtain a white solid compound 3-5 (250 mg, yield: 94%). LCMS (M+H)⁺ = 429.1.

### Step 3

The compound 3-5 (250 mg, 0.58 mmol) was dissolved in THF (3 mL) and water (3 mL), and lithium hydroxide (43 mg, 1.8 mmol) was added and stirred at room temperature to react for 1 h, adjusted with 1M dilute hydrochloric acid to weak acidity, and extracted with ethyl acetate. The organic phase was backwashed with saturated brine, dried with anhydrous sodium sulfate, and concentrated to remove the solvent, to obtain a white solid compound 3-6 (240 mg, yield: 99%). LCMS (M+H)⁺ = 415.2.

### Step 4

The compound 3-6 (240 mg, 0.58 mmol) and the compound 1-4 (123 mg, 0.58 mmol) were dissolved in a mixed solvent of dichloromethane (5 mL) and DMF (5 mL), and DIEA (374 mg, 2.9 mmol), HOBT (115 mg, 0.70 mmol), and EDCI (134 mg, 0.70 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 3-7 (240 mg, yield: 68%). LCMS (M+Na)⁺ = 611.2.

### Step 5

The compound 3-7 (240 mg, 0.39 mmol) was dissolved in dichloromethane (110 mL), and the Grubbs 2^{nd} catalyst (16 mg, 0.02 mmol) was added, to react at room temperature overnight. 3 mL of ethyl vinyl ether was added at zero degrees and stirred for 1 h for quenching. The reaction solution was filtered and concentrated to remove the solvent, to obtain a colorless oily compound 1-8a&b (45 mg, yield: 20%). LCMS (M+H)⁺ = 583.4.

### Step 6

The compound 3-8a&b (45 mg, 0.07 mmol) was dissolved in ethanol (20 mL), PtO₂ (18 mg, 0.08 mmol) was added, and hydrogen substitution was carried out three times, to react at room temperature for 0.5 h. The reaction solution was filtered and concentrated to remove the solvent, to obtain a colorless oily compound 3-9 (33 mg, yield: 73%). LCMS (M+H)⁺ = 585.3.

### Step 7

The compound 3-9 (33 mg, 0.06 mmol) was dissolved in 7M ammonia in methanol (20 mL), to react in a sealed tube at room temperature for 24 h. The reaction solution was concentrated to remove the solvent, to obtain a colorless oily compound 3-10 (22 mg, yield: 68%). LCMS (M+H)⁺ = 570.4.

### Step 8

The compound 3-10 (22 mg, 0.04 mmol) was dissolved in DCM (5 mL), and added dropwise in TFA (0.5 mL) in an ice-water bath. The reaction solution reacted at zero degrees for 3 h. The reaction solution was concentrated to remove the solvent, to obtain a colorless oily compound 3-11 (18 mg, yield: 100%). LCMS (M+H)⁺ = 470.3.

### Step 9

The compound 3-11 (18 mg, 0.05 mmol) was dissolved in THF (10 mL), and added dropwise in pyridine (16 mg, 0.2 mmol) in an ice-water bath. TFAA (13 mg, 0.06 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain a white solid compound 3 (10.5 mg, yield: 50%).

LCMS (M+H)⁺ = 548.1. 1H NMR (400 MHz, DMSO) δ 9.89 (d, J = 5.9 Hz, 1H), 9.11 (d, J = 8.9 Hz, 1H), 7.66 (t, J = 5.4 Hz, 1H), 7.31-7.23 (m, 2H), 7.21 (s, 1H), 7.15 (d, J = 6.4 Hz, 1H), 5.57 (d, J = 5.9 Hz, 1H), 4.94 (dd, J = 15.5, 8.2 Hz, 1H), 4.23 (s, 1H), 3.74 (d, J = 10.5 Hz, 1H), 3.35-3.23 (m, 2H), 2.88 (d, J = 8.2 Hz, 1H), 2.39-2.20 (m, 2H), 2.13 (dd, J = 13.7, 7.0 Hz, 2H), 1.85-1.75 (m, 1H), 1.67-1.31 (m, 6H), 1.26 (d, J = 7.6 Hz, 1H), 1.02 (s, 3H), 1.00 (s, 3H).

### Example 4

### Synthesis of N-((3S, 15S, 18aS, 19aR, 19bS, Z)-3-cyano-19,19-dimethyl-1,6,16-trioxo-1,3,4,5,6,7,8,9,10,13,14,15,16,18,18a,19,19a,19b-octadecahydro-2H-cyclopropane[3,4]pyrrole[1,2-a][1,4,9]triazacyclooctadecene-15-yl)-2,2,2-trifluoroacetamide (compound 4)

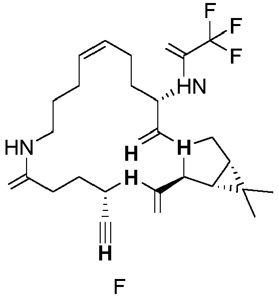

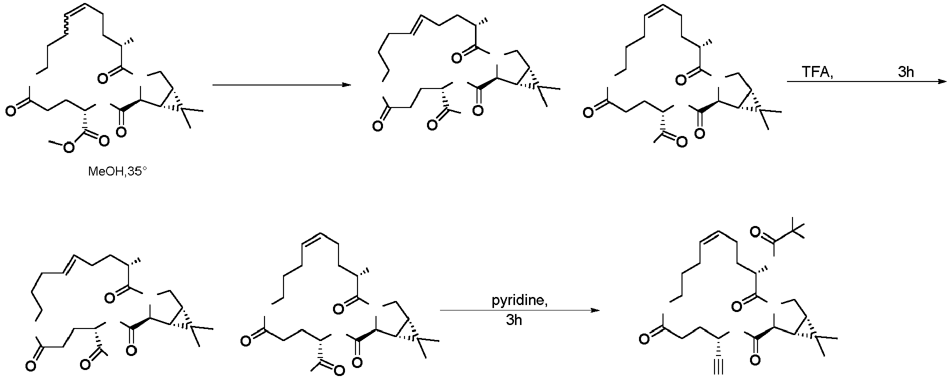

### Step 1

The compound 1-10a&b (75 mg, 0.14 mmol) was dissolved in 7M ammonia in methanol (20 mL), to react in a sealed tube at room temperature for 24 h. The reaction solution was concentrated to remove the solvent, to obtain colorless oily compounds 4-2a and 4-2b (70 mg, yield: 93%). LCMS (M+H)⁺ = 534.3.

### Step 2

The compounds 4-2a and 4-2b (30 mg, 0.06 mmol) were dissolved in DCM (5 mL), and added dropwise in TFA (0.5 mL) in an ice-water bath. The reaction solution reacted at zero degrees for 3 h. The reaction solution was concentrated to remove the solvent, to obtain colorless oily compounds 4-3a and 4-3b (20 mg, yield: 77%). LCMS (M+H)⁺ = 434.3.

### Step 3

The compounds 4-3a and 4-3b (20 mg, 0.05 mmol) were dissolved in THF (10 mL), and added dropwise in pyridine (32 mg, 0.4 mmol) in an ice-water bath. TFAA (26 mg, 0.12 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain a white solid compound 4 (13 mg, yield: 51%).

LCMS (M+H)⁺ = 512.3. 1H NMR (400 MHz, DMSO) δ 9.67 (d, *J* = 5.6 Hz, 1H), 9.06 (d, *J* = 8.8 Hz, 1H), 7.08 (dd, *J* = 4.8 Hz, 6.4 Hz, 1H), 5.42-5.22 (m, 2H), 4.91-4.82 (m, 1H), 4.45-4.40 (m, 1H), 4.14 (s, 1H), 3.88-3.84 (m, 1H), 3.79 (d, *J* = 11.2 Hz, 1H), 3.41-3.36 (m, 1H), 3.33-3.31 (m, 1H), 2.36-2.24 (m, 3H), 2.17-2.10 (m, 2H), 1.89-1.59 (m, 7H), 1.36 (d, *J* = 5.4 Hz, 1H), 1.24-1.22 (m, 1H), 1.24 (s, 3H), 0.95(s, 3H).

### Example 5

### Synthesis of N-((3S, 15S, 18aS, 19aR, 19bS, E/Z)-3-cyano-7.19,19-trimethyl-1,6,16-trioxo-1,3,4,5,6,7,8,9,10,13,14,15,16,18,18a,19,19a,19b-octadecahydro-2H-cyclopropane[3,4]pyrrole[1,2-a][1,4,9]triazacyclooctadecene-15-yl)-2,2,2-trifluoroacetamide (compounds 5a and 5b)

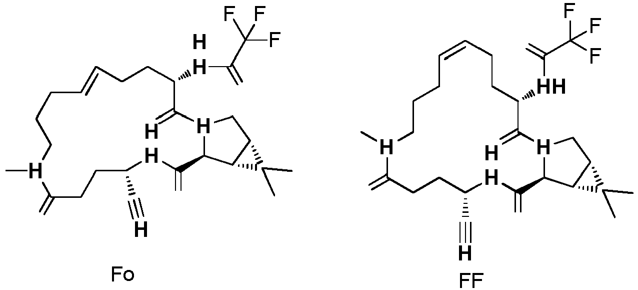

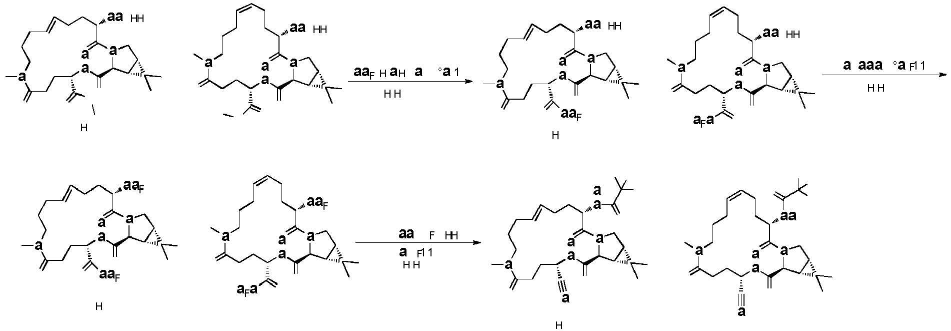

### Step 1

The compounds 2-13a and 2-13b (100 mg, 0.18 mmol) were dissolved in 7M ammonia in methanol (20 mL), to react in a sealed tube at 35°C for 24 h. The reaction solution was concentrated to remove the solvent, to obtain colorless oily compounds 5-2a and 5-2b (50 mg, yield: 50%). LCMS (M+H)⁺ = 548.4.

### Step 2

The compounds 5-2a and 5-2b (50 mg, 0.09 mmol) were dissolved in DCM (5 mL), and added dropwise in TFA (0.5 mL) in an ice-water bath. The reaction solution reacted at zero degrees for 3 h. The reaction solution was concentrated to remove the solvent, to obtain colorless oily compounds 5-3a and 5-3b (40 mg, yield: 98%). LCMS (M+H)⁺ = 448.3.

### Step 3

The compounds 5-3a and 5-3b (40 mg, 0.09 mmol) were dissolved in THF (10 mL), and added dropwise in pyridine (16 mg, 0.2 mmol) in an ice-water bath. TFAA (13 mg, 0.06 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain white solid compounds 5a (15 mg, yield: 32%) and 5b (3.5 mg, yield: 7%). LCMS (M+H)⁺ = 526.3.

5a or 5b: ¹H NMR (400 MHz, DMSO) δ 9.68 (d, J = 5.5 Hz, 1H), 8.94 (d, J = 9.2 Hz, 1H), 5.37 (d, J = 6.5 Hz, 2H), 4.92 (t, J = 9.7 Hz, 1H), 4.35 (dd, J = 13.1, 6.7 Hz, 1H), 4.22-4.07 (m, 2H), 3.89-3.73 (m, 2H), 3.12 (d, J = 60.7 Hz, 1H), 2.85 (s, 2H), 2.75 (s, 1H), 2.38-2.22 (m, 2H), 2.14 (d, J = 20.8 Hz, 2H), 1.75 (dd, J = 26.7, 21.7 Hz, 2H), 1.62 (d, J = 6.5 Hz, 2H), 1.56 (dd, J = 13.1, 8.5 Hz, 2H), 1.34 (d, J = 9.1 Hz, 2H), 1.24 (s, 2H), 0.95 (s, 3H), 0.89 (s, 3H).

5b or 5a: ¹H NMR (400 MHz, DMSO) δ 9.80-9.45 (m, 1H), 9.14-8.85 (m, 1H), 5.34 (s, 2H), 4.99-4.80 (m, 1H), 4.41 (dd, J = 14.6, 8.4 Hz, 1H), 4.20 (t, J = 23.9 Hz, 2H), 3.97-3.83 (m, 1H), 3.79 (d, J = 10.1 Hz, 2H), 2.79 (d, J = 5.7 Hz, 3H), 2.33 (s, 2H), 2.04 (d, J = 42.2 Hz, 4H), 1.77 (dd, J = 46.6, 17.2 Hz, 4H), 1.63-1.33 (m, 4H), 1.10-1.02 (s, 3H), 0.89 (s, 3H).

Note: The NMR data above indicates that when the compound 5a is NMR-characterized as ¹H NMR (400 MHz, DMSO) δ 9.68 (d, J = 5.5 Hz, 1H), 8.94 (d, J = 9.2 Hz, 1H), 5.37 (d, J = 6.5 Hz, 2H), 4.92 (t, J = 9.7 Hz, 1H), 4.35 (dd, J = 13.1, 6.7 Hz, 1H), 4.22-4.07 (m, 2H), 3.89-3.73 (m, 2H), 3.12 (d, J = 60.7 Hz, 1H), 2.85 (s, 2H), 2.75 (s, 1H), 2.38-2.22 (m, 2H), 2.14 (d, J = 20.8 Hz, 2H), 1.75 (dd, J = 26.7, 21.7 Hz, 2H), 1.62 (d, J = 6.5 Hz, 2H), 1.56 (dd, J = 13.1, 8.5 Hz, 2H), 1.34 (d, J = 9.1 Hz, 2H), 1.24 (s, 2H), 0.95 (s, 3H), 0.89 (s, 3H), the compound 5b is NMR-characterized as ¹H NMR (400 MHz, DMSO) δ 9.80-9.45 (m, 1H), 9.14-8.85 (m, 1H), 5.34 (s, 2H), 4.99-4.80 (m, 1H), 4.41 (dd, J = 14.6, 8.4 Hz, 1H), 4.20 (t, J = 23.9 Hz, 2H), 3.97-3.83 (m, 1H), 3.79 (d, J = 10.1 Hz, 2H), 2.79 (d, J = 5.7 Hz, 3H), 2.33 (s, 2H), 2.04 (d, J = 42.2 Hz, 4H), 1.77 (dd, J = 46.6, 17.2 Hz, 4H), 1.63-1.33 (m, 4H), 1.10-1.02 (s, 3H), 0.89 (s, 3H); or
when the compound 5a is NMR-characterized as ¹H NMR (400 MHz, DMSO) δ 9.80-9.45 (m, 1H), 9.14-8.85 (m, 1H), 5.34 (s, 2H), 4.99-4.80 (m, 1H), 4.41 (dd, J = 14.6, 8.4 Hz, 1H), 4.20 (t, J = 23.9 Hz, 2H), 3.97-3.83 (m, 1H), 3.79 (d, J = 10.1 Hz, 2H), 2.79 (d, J = 5.7 Hz, 3H), 2.33 (s, 2H), 2.04 (d, J = 42.2 Hz, 4H), 1.77 (dd, J = 46.6, 17.2 Hz, 4H), 1.63-1.33 (m, 4H), 1.10-1.02 (s, 3H), 0.89 (s, 3H), the compound 5b is NMR-characterized as ¹H NMR (400 MHz, DMSO) δ 9.68 (d, J = 5.5 Hz, 1H), 8.94 (d, J = 9.2 Hz, 1H), 5.37 (d, J = 6.5 Hz, 2H), 4.92 (t, J = 9.7 Hz, 1H), 4.35 (dd, J = 13.1, 6.7 Hz, 1H), 4.22-4.07 (m, 2H), 3.89-3.73 (m, 2H), 3.12 (d, J = 60.7 Hz, 1H), 2.85 (s, 2H), 2.75 (s, 1H), 2.38-2.22 (m, 2H), 2.14 (d, J = 20.8 Hz, 2H), 1.75 (dd, J = 26.7, 21.7 Hz, 2H), 1.62 (d, J = 6.5 Hz, 2H), 1.56 (dd, J = 13.1, 8.5 Hz, 2H), 1.34 (d, J = 9.1 Hz, 2H), 1.24 (s, 2H), 0.95 (s, 3H), 0.89 (s, 3H).

### Example 6

### Synthesis of N-((3S, 16S, 19aS, 20aR, 20bS)-3-cyano-20,20-dimethyl-1,6,17-trioxydocosanylhydrocyclopropa[3,4]pyrrole[1,2-a][1,4,9]triazacyclononanedioic acid-16-yl)-2,2,2-trifluoroacetamide (compound 6)

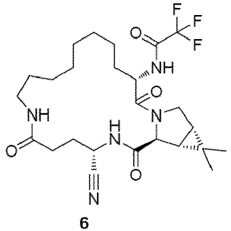

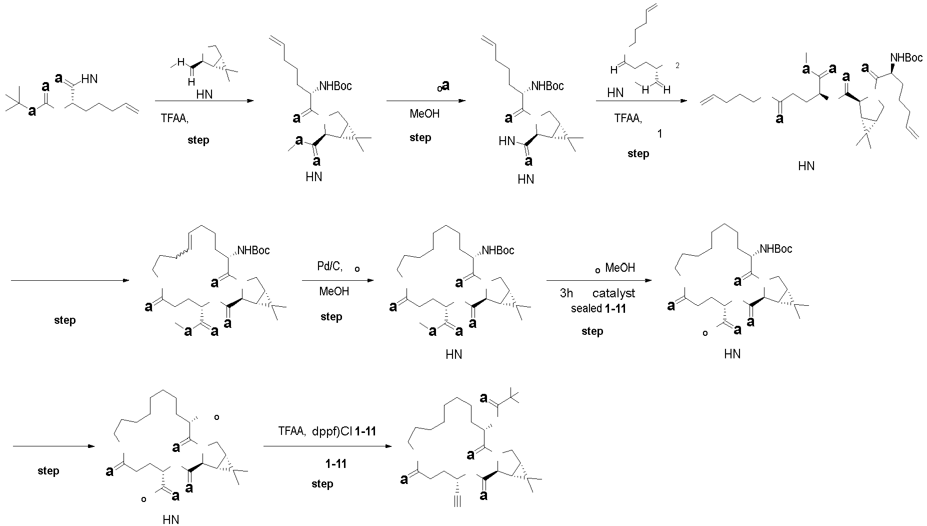

### Step 1

A compound 6-1 (650 mg, 2.67 mmol) and a compound 6-2 (460 mg, 2.67 mmol) were dissolved in DMF (10 mL), and HATU (1.52 g, 4.01 mmol) and TEA (540 mg, 5.35 mmol) were added. The reaction system reacted at room temperature under nitrogen protection for 1 h. The reaction system was quenched with water and extracted with ethyl acetate (60 mL). The organic phase was washed with water (50 mL × 3), dried with anhydrous sodium sulfate, and spin-dried to obtain a light-yellow colloidal compound 6-3 (1.2 g, crude product). LCMS (M+H)⁺ = 395.4.

### Step 2

The compound 6-3 (1.1 g, 2.79 mmol) was dissolved in methanol (12 mL), and a solution of LiOH·H₂O (585 mg, 14.0 mmol) in water (10 mL) was added. The reaction system reacted at room temperature for 2 h. The reaction system was adjusted to pH 5 by adding a 1M hydrochloric acid solution, and spin-dried to remove the methanol. The reaction system was extracted with ethyl acetate (15 mL). The organic phase was washed with water (10 mL × 2), dried with anhydrous sodium sulfate, and spin-dried to obtain a light-yellow solid compound 6-4 (980 mg, yield: 92%). LCMS (M+H)⁺ = 381.4.

### Step 3

The compound 6-4 (490 mg, 1.29 mmol) and the compound 1-4 (294 mg, 1.29 mmol) were dissolved in DMF (7 mL), and HATU (1735 mg, 1.94 mmol) and TEA (260 mg, 2.58 mmol) were added. The reaction system reacted at room temperature under nitrogen protection for 2 h. The reaction system was quenched with water (30 mL) and extracted with ethyl acetate (60 mL). The organic phase was back-extracted with water (50 mL × 3), dried with anhydrous sodium sulfate, and spin-dried to obtain a crude product. The crude product was separated by medium-pressure liquid chromatography to obtain a colorless colloidal compound 6-5 (460 mg, yield: 61%). LCMS (M+H)⁺ = 591.4.

### Step 4

The Grubbs 2^{nd} catalyst (170 mg, 0.20 mmol) was added in a solution of the compound 6-5 (400 mg, 0.68 mmol) in anhydrous dichloromethane (150 mL) under nitrogen protection. The reaction system reacted at room temperature under nitrogen protection for 2 h. The reaction system was spin-dried to obtain a crude product. The crude product was separated by medium-pressure liquid chromatography to obtain a light-yellow colloidal compound 6-6a&b (230 mg, yield: 60%). LCMS (M+H)⁺ = 563.3.

### Step 5

The compound 6-6a&b (90 mg, 0.16 mmol) was dissolved in methanol (10 mL), and wet palladium carbon (20 mg) was added. The reaction system reacted at room temperature under hydrogen protection for 2 h. The reaction system was filtered with diatomaceous earth to obtain a filtrate, and the filtrate was spin-dried to obtain a colorless colloidal compound 6-7 (91 mg, yield: 100%). LCMS (M+H)⁺ = 565.4.

### Step 6

The compound 6-7 (130 mg, 0.23 mmol) was dissolved in methanol (5 mL), and a 7M ammonia in methanol solution (10 mL) was added. The reaction system reacted in a tetrafluoro tank at 70 degrees overnight. The reaction system was spin-dried to obtain a colorless colloidal compound 6-8 (120 mg, yield: 95%). LCMS (M+H)⁺ = 550.3.

### Step 7

The compound 6-8 (120 mg, 0.22 mmol) was dissolved in methanol (5 mL), and a 4M hydrogen chloride ethyl acetate solution (5 mL) was added. The reaction system reacted at room temperature for 2 h. The reaction system was spin-dried to obtain a colorless colloidal compound 6-9 (95 mg, yield: 90%). LCMS (M+H)⁺ = 450.3.

### Step 8

The compound 6-9 (95 mg, 0.21 mmol) was dissolved in anhydrous THF (5 mL) under nitrogen protection, and pyridine (0.3 mL) and TFAA (0.3 mL) were added in an ice-water bath. The reaction solution reacted at room temperature for 2 h. The reaction solution was poured in ice water (20 mL) and extracted with ethyl acetate (20 mL). The organic phase was washed with water (20 mL), dried with anhydrous sodium sulfate, and spin-dried to obtain a crude product. The crude product was separated by high-performance liquid chromatography to obtain a white solid 6 (28 mg, yield: 25%).

LCMS: (M+H)⁺ = 528.3. ¹H NMR (400 MHz, DMSO-*d₆*) δ 9.69 (br, 1H), 9.07 (d, *J* = 7.6 Hz, 1H), 8.03 (t, *J* = 5.6 Hz, 1H), 4.75 (q, *J* = 7.2 Hz, 1H), 4.15 (s, 1H), 3.86 (dd, *J* = 10.4, 5.6 Hz, 1H), 3.75 (d, *J* = 10.4 Hz, 1H), 3.23-3.33 (m, 1H), 2.81-2.87 (m, 1H), 2.25-2.33 (m, 1H), 2.12-2.19 (m, 1H), 1.97-2.03 (m, 1H), 1.81-1.89 (m, 2H), 1.53-1.56 (m, 2H), 1.22-1.39 (m, 12H), 1.02 (s, 3H), 0.89 (s, 3H).

### Example 7

### Synthesis of N-((3S, 14S, 17aS, 18aR, 18bS)-3-cyano-18,18-dimethyl-1,6,15-trioxyeicosanylhydrocyclopropyl[3,4]pyrrole[1,2-a][1,4,9]triazacycloheptanedioic acid-14-yl)-2,2,2-trifluoroacetamide (compound 7)

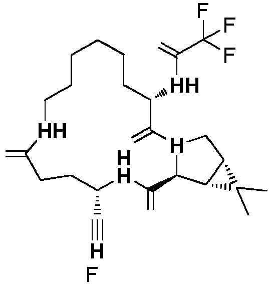

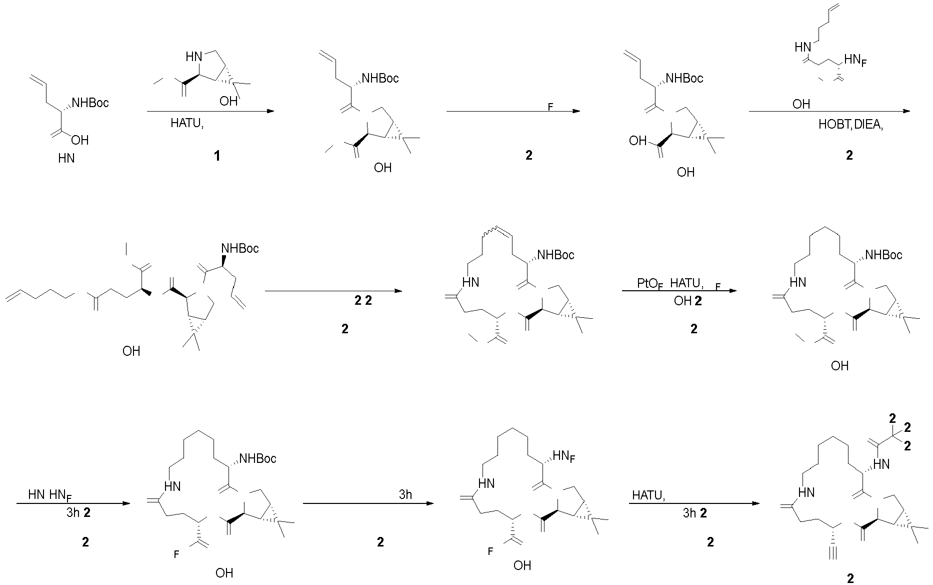

### Step 1

A compound 7-1 (500.0 mg, 2.3 mmol) and a compound 7-2 (524 mg, 2.56 mmol) were dissolved in DMF (30 mL), and DIEA(900 mg, 7.0 mmol) and HATU (1.06 g, 2.8 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 7-3 (650 mg, yield: 76%). LCMS (M+Na)⁺ = 367.2.

### Step 2

The compound 7-3 (650 mg, 1.8 mmol) was dissolved in THF (20 mL) and added dropwise at zero degrees in LiOH (128 mg, 5.3 mmol) dissolved in water to react at room temperature for 2 h, adjusted with 1M hydrochloric acid to pH 4-5, extracted with ethyl acetate, dried with anhydrous sodium sulfate, and concentrated to remove the solvent, to obtain a colorless oily compound 7-4 (500 mg, yield: 80%). LCMS (M+Na)⁺ = 353.2.

### Step 3

The compound 7-4 (500 mg, 1.4 mmol) and the compound 1-4 (320 mg, 1.4 mmol) were dissolved in a mixed solvent of dichloromethane (5 mL) and DMF (5 mL), and DIEA (542 mg, 4.2 mmol), HOBT (277 mg, 1.7 mmol), and EDCI (325 mg, 1.7 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 7-5 (300 mg, yield: 38%). LCMS (M+Na)⁺ = 563.2.

### Step 4

The compound 7-5 (300 mg, 0.53 mmol) was dissolved in dichloromethane (150 mL), and the Grubbs 2^{nd} catalyst (16 mg, 0.05 mmol) was added, to react at room temperature overnight. 1 mL of ethyl vinyl ether was added at zero degrees and stirred for 1 h for quenching. The reaction solution was filtered and concentrated to remove the solvent, to obtain a brown oily compound 7-6a&b (285 mg, yield: 100%). LCMS (M+H)⁺ = 535.4.

### Step 5

The compound 7-6a&b (100 mg, 0.18 mmol) was dissolved in ethanol (20 mL), PtO₂ (18 mg, 0.08 mmol) was added, and hydrogen substitution was carried out three times, to react at room temperature for 0.5 h. The reaction solution was filtered and concentrated to remove the solvent, to obtain a colorless oily compound 7-7 (80 mg, yield: 80%). LCMS (M+H)⁺ = 537.3.

### Step 6

The compound 7-7 (80 mg, 0.15 mmol) was dissolved in 7M ammonia in methanol (10 mL), to react in a sealed tube at room temperature for 24 h. The reaction solution was concentrated to remove the solvent, to obtain a colorless oily compound 7-8 (50 mg, yield: 64%). LCMS (M+H)⁺ = 522.4.

### Step 7

The compound 7-8 (50 mg, 0.06 mmol) was dissolved in DCM (5 mL), and added dropwise in TFA (0.5 mL) in an ice-water bath. The reaction solution reacted at zero degrees for 3 h. The reaction solution was concentrated to remove the solvent, to obtain a colorless oily compound 7-9 (40 mg, yield: 99%). LCMS (M+H)⁺ = 422.3.

### Step 8

The compound 7-9 (40 mg, 0.04 mmol) was dissolved in THF (10 mL), and added dropwise in pyridine (16 mg, 0.2 mmol) in an ice-water bath. TFAA (13 mg, 0.06 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain a white solid compound 7 (15 mg, yield: 31%).

LCMS (M+H)⁺ = 500.2. 1H NMR (400 MHz, DMSO) δ 9.75 (d, J = 6.9 Hz, 1H), 9.31 (d, J = 7.6 Hz, 1H), 7.87 (dd, J = 7.4, 4.0 Hz, 1H), 4.91 (dd, J = 12.7, 7.4 Hz, 1H), 4.47 (d, J = 7.0 Hz, 1H), 4.13 (s, 1H), 3.93 (dd, J = 10.4, 5.4 Hz, 1H), 3.71 (d, J = 10.5 Hz, 1H), 3.54-3.43 (m, 1H), 2.74-2.66 (m, 1H), 2.34-2.27 (m, 2H), 2.04-1.86 (m, 2H), 1.77 (dd, J = 13.7, 7.0 Hz, 1H), 1.54 (dt, J = 15.4, 7.8 Hz, 2H), 1.34 (d, J = 7.6 Hz, 4H), 1.27-1.13 (m, 5H), 1.02 (s, 3H), 0.87 (s, 3H).

### Example 8

### Synthesis of N-((3S, 16S, 19aS, 20aR, 20bS, Z/E)-3-cyano-20,20-dimethyl-1,6,17-trioxo-1,2,3,4,5,6,7,8,9,10,13,14,15,16,17,19,19a,20,20a,20b-eicosanylhydrocyclopropyl[3,4]pyrrole[1,2-a][1,4,9]triazacyclonon-16-yl)-2,2,2-trifluoroacetamide (compounds 8a and 8b)

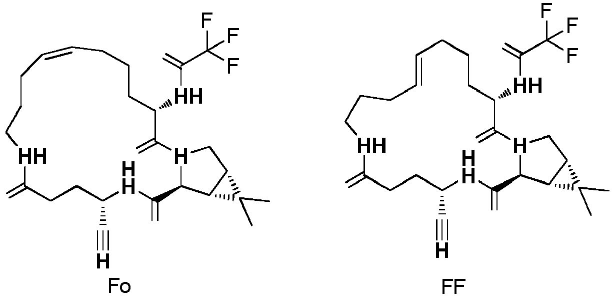

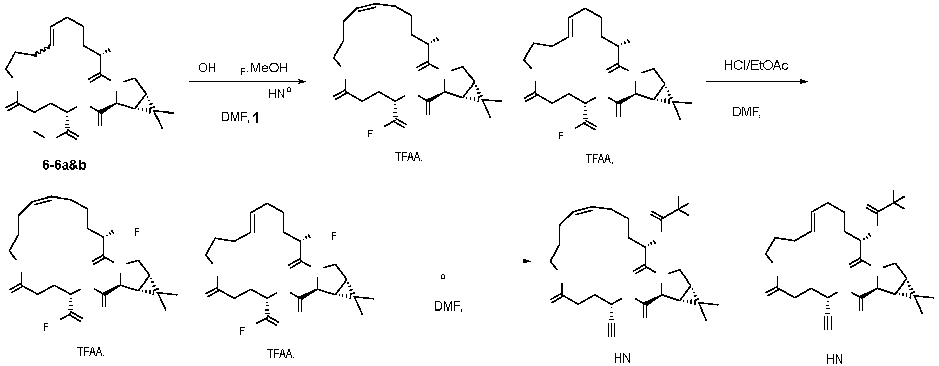

### Step 1

The compound 6-6a&b (140 mg, 0.25 mmol) was dissolved in methanol (5 mL), and a 7M ammonia in methanol solution (10 mL) was added. The reaction system reacted in a tetrafluoro tank at 70 degrees overnight. The reaction system was spin-dried to obtain colorless colloidal compounds 8-2a and 8-2b (135 mg, yield: 99%). LCMS (M+H)⁺ = 548.4.

### Step 2

The compounds 8-2a and 8-2b (130 mg, 0.24 mmol) were dissolved in methanol (5 mL), and a 4M hydrogen chloride ethyl acetate solution (4 mL) was added. The reaction system reacted at room temperature for 2 h. The reaction system was spin-dried to obtain colorless solid compounds 8-3a and 8-3b (110 mg, yield: 96%). LCMS (M+H)⁺ = 448.4.

### Step 3

The compounds 8-3a and 8-3b (110 mg, 0.23 mmol) were dissolved in anhydrous THF (5 mL), and pyridine (0.3 mL) and TFAA (0.3 mL) were added in an ice-water bath. The reaction solution reacted at room temperature for 2 h. The reaction solution was poured in ice water (20 mL) and extracted with ethyl acetate (20 mL). The organic phase was washed with water (20 mL), dried with anhydrous sodium sulfate, and spin-dried to obtain a crude product. The crude product was separated by high-performance liquid chromatography to obtain white solid compounds 8a (13 mg, yield: 11%) and 8b (28 mg, yield: 23%). LCMS: (M+H)⁺ = 526.2.

The compound 8a or 8b is NMR-characterized as: ¹H NMR (400 MHz, DMSO-*d₆*) δ 9.75 (d, *J* = 6.8 Hz, 1H), 9.23 (d, *J* = 6.8 Hz, 1H), 7.83 (t, *J* = 5.6 Hz, 1H), 5.30-5.39 (m, 2H), 4.70 (q, *J* = 7.2 Hz, 1H), 4.38 (q, *J* = 7.2 Hz, 1H), 4.11 (s, 1H), 3.88 (dd, *J* = 10.4, 5.6 Hz, 1H), 3.73 (d, *J* = 10.4 Hz, 1H), 3.15-3.22 (m, 1H), 2.91-2.97 (m, 1H), 2.30-2.36 (m, 1H), 2.18-2.25 (m, 1H), 1.99-2.09 (m, 2H), 1.86-1.98 (m, 5H), 1.43-1.58 (m, 6H), 1.32-1.36 (m, 1H), 1.02 (s, 3H), 0.88 (s, 3H).

The compound 8b or 8a is NMR-characterized as: ¹H NMR (400 MHz, DMSO-*d₆*) δ 9.75 (d, *J* = 6.4 Hz, 1H), 9.05 (d, *J* = 8.0 Hz, 1H), 7.56 (t, *J* = 5.6 Hz, 1H), 5.34-5.37 (m, 2H), 4.81-4.87 (m, 1H), 4.32-4.37 (m, 1H), 4.13 (s, 1H), 3.81-3.85 (m, 1H), 3.70-3.73 (m, 1H), 3.13-3.18 (m, 1H), 2.94-2.99 (m, 1H), 2.16-2.21 (m, 2H), 1.86-2.06 (m, 7H), 1.66-1.74 (m, 1H), 1.53-1.58 (m, 2H), 1.40-1.49 (m, 3H), 1.30-1.36 (m, 1H), 1.02 (s, 3H), 0.89 (s, 3H).

Note: The NMR data above indicates that when the compound 8a is NMR-characterized as ¹H NMR (400 MHz, DMSO-*d₆*) δ 9.75 (d, *J* = 6.8 Hz, 1H), 9.23 (d, *J* = 6.8 Hz, 1H), 7.83 (t, *J* = 5.6 Hz, 1H), 5.30-5.39 (m, 2H), 4.70 (q, *J* = 7.2 Hz, 1H), 4.38 (q, *J* = 7.2 Hz, 1H), 4.11 (s, 1H), 3.88 (dd, *J* = 10.4, 5.6 Hz, 1H), 3.73 (d, *J* = 10.4 Hz, 1H), 3.15-3.22 (m, 1H), 2.91-2.97 (m, 1H), 2.30-2.36 (m, 1H), 2.18-2.25 (m, 1H), 1.99-2.09 (m, 2H), 1.86-1.98 (m, 5H), 1.43-1.58 (m, 6H), 1.32-1.36 (m, 1H), 1.02 (s, 3H), 0.88 (s, 3H), the compound 8b is NMR-characterized as ¹H NMR (400 MHz, DMSO-*d₆*) δ 9.75 (d, *J* = 6.4 Hz, 1H), 9.05 (d, *J* = 8.0 Hz, 1H), 7.56 (t, *J* = 5.6 Hz, 1H), 5.34-5.37 (m, 2H), 4.81-4.87 (m, 1H), 4.32-4.37 (m, 1H), 4.13 (s, 1H), 3.81-3.85 (m, 1H), 3.70-3.73 (m, 1H), 3.13-3.18 (m, 1H), 2.94-2.99 (m, 1H), 2.16-2.21 (m, 2H), 1.86-2.06 (m, 7H), 1.66-1.74 (m, 1H), 1.53-1.58 (m, 2H), 1.40-1.49 (m, 3H), 1.30-1.36 (m, 1H), 1.02 (s, 3H), 0.89 (s, 3H); or
when the compound 8a is NMR-characterized as ¹H NMR (400 MHz, DMSO-*d₆*) δ 9.75 (d, *J* = 6.4 Hz, 1H), 9.05 (d, *J* = 8.0 Hz, 1H), 7.56 (t, *J* = 5.6 Hz, 1H), 5.34-5.37 (m, 2H), 4.81-4.87 (m, 1H), 4.32-4.37 (m, 1H), 4.13 (s, 1H), 3.81-3.85 (m, 1H), 3.70-3.73 (m, 1H), 3.13-3.18 (m, 1H), 2.94-2.99 (m, 1H), 2.16-2.21 (m, 2H), 1.86-2.06 (m, 7H), 1.66-1.74 (m, 1H), 1.53-1.58 (m, 2H), 1.40-1.49 (m, 3H), 1.30-1.36 (m, 1H), 1.02 (s, 3H), 0.89 (s, 3H), the compound 8b is NMR-characterized as ¹H NMR (400 MHz, DMSO-*d₆*) δ 9.75 (d, *J* = 6.8 Hz, 1H), 9.23 (d, *J* = 6.8 Hz, 1H), 7.83 (t, *J* = 5.6 Hz, 1H), 5.30-5.39 (m, 2H), 4.70 (q, *J* = 7.2 Hz, 1H), 4.38 (q, *J* = 7.2 Hz, 1H), 4.11 (s, 1H), 3.88 (dd, *J* = 10.4, 5.6 Hz, 1H), 3.73 (d, *J* = 10.4 Hz, 1H), 3.15-3.22 (m, 1H), 2.91-2.97 (m, 1H), 2.30-2.36 (m, 1H), 2.18-2.25 (m, 1H), 1.99-2.09 (m, 2H), 1.86-1.98 (m, 5H), 1.43-1.58 (m, 6H), 1.32-1.36 (m, 1H), 1.02 (s, 3H), 0.88 (s, 3H).

### Example 9

### Synthesis of N-((3S, 14S, 17aS, 18aR, 18bS, E/Z)-3-cyano-18,18-dimethyl-1,6,15-trioxo-1,2,3,4,5,6,7,8,9,10,13,14,15,17,17a,18,18a,18b-octadecyclopropyl[3,4]pyrrole[1,2-a][1,4,9]triazacycloheptene-14-yl)-2,2-trifluoroacetamide (compounds 9a and 9b)

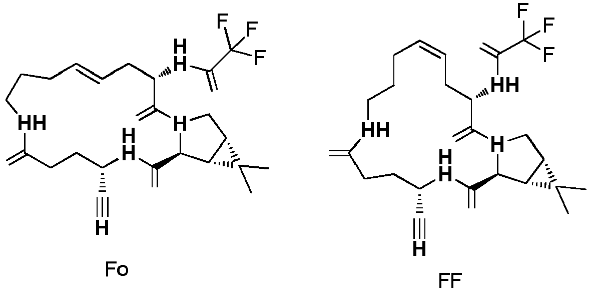

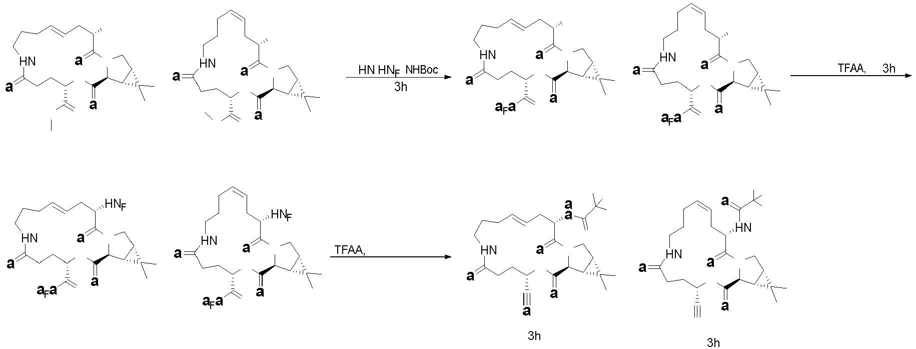

### Step 1

The compounds 7-6a and 7-6b (100 mg, 0.19 mmol) were dissolved in 7M ammonia in methanol (10 mL), to react in a sealed tube at room temperature for 24 h. The reaction solution was concentrated to remove the solvent, to obtain a crude product. The crude product was subjected to Prep-HPLC to obtain white solid compounds 9-1a (25 mg, yield: 26%) and 9-1b (10 mg, yield: 10%). LCMS (M+H)⁺ = 520.2.

### Step 2

The compounds 9-1a (25 mg, 0.05 mmol) and 9-2b (10 mg, yield: 0.02) were respectively dissolved in DCM (5 mL), and added dropwise in TFA (0.5 mL) in an ice-water bath. The reaction solution reacted at zero degrees for 3 h. The reaction solution was concentrated to remove the solvent, to obtain colorless oily compounds 9-2a (20 mg, yield: 99%) and 9-2b (8 mg, yield: 99%). LCMS (M+H)⁺ = 420.3.

### Step 10

The compounds 9-2a (20 mg, 0.05 mmol) and 9-2b (8 mg, 0.02 mmol) were respectively dissolved in THF (10 mL), and added dropwise in pyridine (16 mg, 0.2 mmol) in an ice-water bath. TFAA (13 mg, 0.06 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain white solid compounds 9a (15 mg, yield: 63%) and 9b (5 mg, yield: 53%). LCMS (M+H)⁺ = 498.1.

9a or 9b: ¹H NMR (400 MHz, DMSO) δ 9.92 (d, J = 7.7 Hz, 1H), 9.61 (d, J = 6.9 Hz, 1H), 7.71 (d, J = 7.3 Hz, 1H), 5.37 (dd, J = 13.1, 7.3 Hz, 1H), 5.10 (dd, J = 12.9, 7.2 Hz, 1H), 4.82 (dd, J = 11.3, 6.0 Hz, 1H), 4.49 (d, J = 9.1 Hz, 1H), 4.09 (s, 1H), 3.97 (dd, J = 10.6, 5.4 Hz, 1H), 3.52 (d, J = 10.5 Hz, 2H), 2.76-2.66 (m, 2H), 2.43 (d, J = 5.8 Hz, 2H), 2.13-2.07 (m, 1H), 1.98 (d, J = 5.8 Hz, 3H), 1.83 (d, J = 9.7 Hz, 1H), 1.53 (dd, J = 13.7, 8.3 Hz, 3H), 1.37 (d, J = 7.6 Hz, 1H), 1.01 (s, 3H), 0.83 (s, 3H).

9b or 9a: ¹H NMR (400 MHz, DMSO) δ 9.96 (s, 1H), 9.48 (d, J = 6.2 Hz, 1H), 7.96 (s, 1H), 5.40-5.25 (m, 2H), 4.71 (d, J = 3.8 Hz, 1H), 4.55 (dd, J = 9.3, 5.2 Hz, 1H), 4.08 (s, 1H), 3.95 (dd, J = 10.7, 5.4 Hz, 1H), 3.64 (d, J = 10.7 Hz, 1H), 2.82 (d, J = 13.2 Hz, 1H), 2.67 (s, 1H), 2.40-2.28 (m, 2H), 2.24-1.91 (m, 5H), 1.84 (s, 1H), 1.58-1.49 (m, 2H), 1.39 (t, J = 12.7 Hz, 2H), 1.01 (s, 3H), 0.83 (s, 3H).

Note: The NMR data above indicates that when the compound 9a is NMR-characterized as ¹H NMR (400 MHz, DMSO) δ 9.92 (d, J = 7.7 Hz, 1H), 9.61 (d, J = 6.9 Hz, 1H), 7.71 (d, J = 7.3 Hz, 1H), 5.37 (dd, J = 13.1, 7.3 Hz, 1H), 5.10 (dd, J = 12.9, 7.2 Hz, 1H), 4.82 (dd, J = 11.3, 6.0 Hz, 1H), 4.49 (d, J = 9.1 Hz, 1H), 4.09 (s, 1H), 3.97 (dd, J = 10.6, 5.4 Hz, 1H), 3.52 (d, J = 10.5 Hz, 2H), 2.76-2.66 (m, 2H), 2.43 (d, J = 5.8 Hz, 2H), 2.13-2.07 (m, 1H), 1.98 (d, J = 5.8 Hz, 3H), 1.83 (d, J = 9.7 Hz, 1H), 1.53 (dd, J = 13.7, 8.3 Hz, 3H), 1.37 (d, J = 7.6 Hz, 1H), 1.01 (s, 3H), 0.83 (s, 3H), the compound 9b is NMR-characterized as ¹H NMR (400 MHz, DMSO) δ 9.96 (s, 1H), 9.48 (d, J = 6.2 Hz, 1H), 7.96 (s, 1H), 5.40-5.25 (m, 2H), 4.71 (d, J = 3.8 Hz, 1H), 4.55 (dd, J = 9.3, 5.2 Hz, 1H), 4.08 (s, 1H), 3.95 (dd, J = 10.7, 5.4 Hz, 1H), 3.64 (d, J = 10.7 Hz, 1H), 2.82 (d, J = 13.2 Hz, 1H), 2.67 (s, 1H), 2.40-2.28 (m, 2H), 2.24-1.91 (m, 5H), 1.84 (s, 1H), 1.58-1.49 (m, 2H), 1.39 (t, J = 12.7 Hz, 2H), 1.01 (s, 3H), 0.83 (s, 3H); or
when the compound 9b is NMR-characterized as ¹H NMR (400 MHz, DMSO) δ 9.92 (d, J = 7.7 Hz, 1H), 9.61 (d, J = 6.9 Hz, 1H), 7.71 (d, J = 7.3 Hz, 1H), 5.37 (dd, J = 13.1, 7.3 Hz, 1H), 5.10 (dd, J = 12.9, 7.2 Hz, 1H), 4.82 (dd, J = 11.3, 6.0 Hz, 1H), 4.49 (d, J = 9.1 Hz, 1H), 4.09 (s, 1H), 3.97 (dd, J = 10.6, 5.4 Hz, 1H), 3.52 (d, J = 10.5 Hz, 2H), 2.76-2.66 (m, 2H), 2.43 (d, J = 5.8 Hz, 2H), 2.13-2.07 (m, 1H), 1.98 (d, J = 5.8 Hz, 3H), 1.83 (d, J = 9.7 Hz, 1H), 1.53 (dd, J = 13.7, 8.3 Hz, 3H), 1.37 (d, J = 7.6 Hz, 1H), 1.01 (s, 3H), 0.83 (s, 3H), the compound 9a is NMR-characterized as ¹H NMR (400 MHz, DMSO) δ 9.96 (s, 1H), 9.48 (d, J = 6.2 Hz, 1H), 7.96 (s, 1H), 5.40-5.25 (m, 2H), 4.71 (d, J = 3.8 Hz, 1H), 4.55 (dd, J = 9.3, 5.2 Hz, 1H), 4.08 (s, 1H), 3.95 (dd, J = 10.7, 5.4 Hz, 1H), 3.64 (d, J = 10.7 Hz, 1H), 2.82 (d, J = 13.2 Hz, 1H), 2.67 (s, 1H), 2.40-2.28 (m, 2H), 2.24-1.91 (m, 5H), 1.84 (s, 1H), 1.58-1.49 (m, 2H), 1.39 (t, J = 12.7 Hz, 2H), 1.01 (s, 3H), 0.83 (s, 3H).

### Example 10

### Synthesis of N-((3S, 14S, 17aR, 20aS, 20bS)-3-cyano-1,6,15-trioxodococarbodihydrocyclopentane[3,4]pyrrole[1,2-a][1,4,9]triazacyclic heptapeptide-14-yl)-2,2,2-trifluoroacetamide (compound 10)

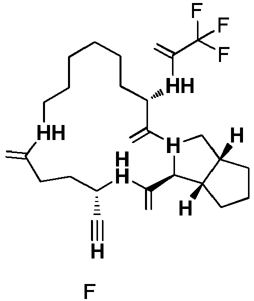

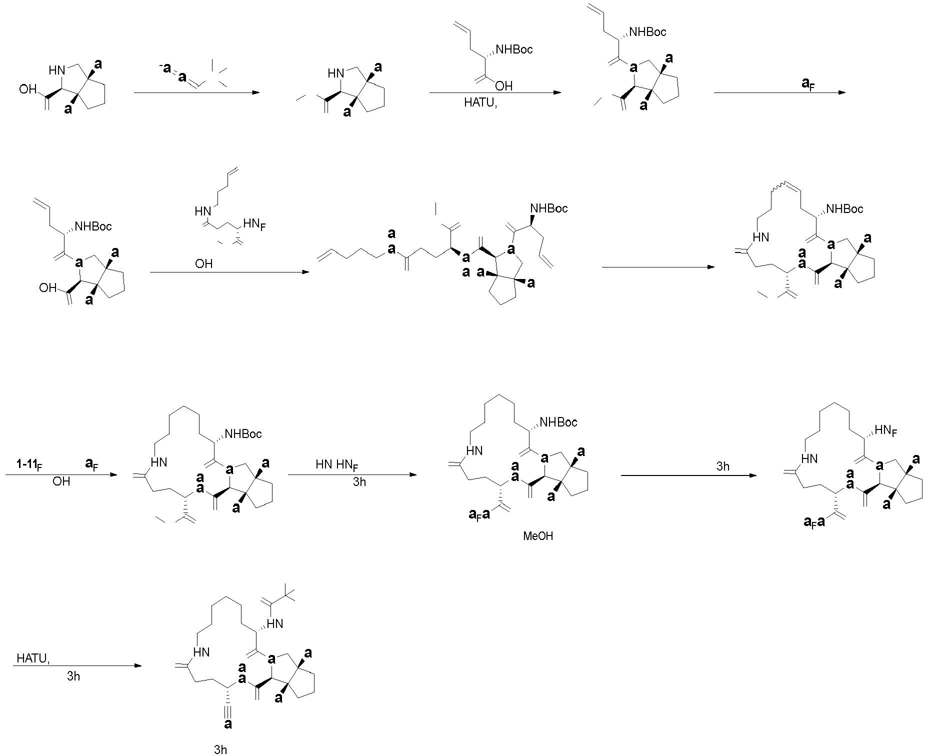

### Step 1

A compound 10-1 (220 mg, 1.42 mmol) was dissolved in a mixed solution of dichloromethane (10 mL) and methanol (1 mL), and a compound 10-2 (0.75 mL, 1.49 mmol) was added dropwise at zero degrees, to react at room temperature for 2 h. The reaction was quenched with water at zero degrees, extracted with ethyl acetate, dried with saturated brine, dried with anhydrous sodium sulfate, and concentrated to remove the solvent, to obtain a light-yellow oily compound 10-3 (150 mg, yield: 63%). LCMS (M+Na)⁺ = 170.2.

### Step 2

A compound 10-3 (150 mg, 0.89 mmol), a compound 10-4 (203 mg, 0.89 mmol), and DIEA(344 mg, 2.67 mmol) were dissolved in DMF (5 mL), and HATU (6.47 g, 0.89 mmol) was added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 10-5 (150 mg, yield: 46.2%). LCMS (M+Na)⁺ = 366.3.

### Step 3

The compound 10-5 (150 mg, 0.41 mmol) was dissolved in THF (10 mL) and added dropwise at zero degrees in LiOH (28.8 mg, 1.2 mmol) dissolved in water to react at room temperature for 2 h, adjusted with 1M hydrochloric acid to pH 4-5, extracted with ethyl acetate, dried with anhydrous sodium sulfate, and concentrated to remove the solvent, to obtain a colorless oily compound 10-6 (120 mg, yield: 83%). LCMS (M+Na)⁺ = 353.2.

### Step 4

The compound 10-6 (120 mg, 0.34 mmol) and the compound 1-4 (77 mg, 0.34 mmol) were dissolved in a mixed solvent of dichloromethane (5 mL) and DMF (5 mL), and DIEA (87 mg, 0.68 mmol), HOBT (66 mg, 0.41 mmol), and EDCI (78 mg, 0.41 mmol) were added, to react at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was purified by SGC (PE:EA = 2:1) to obtain a colorless oily compound 10-7 (170 mg, yield: 89%). LCMS (M+Na)⁺ = 563.2.

### Step 5

The compound 10-7 (170 mg, 0.30 mmol) was dissolved in dichloromethane (80 mL), and the Grubbs 2^{nd} catalyst (24 mg, 0.03 mmol) was added, to react at room temperature overnight. 1 mL of ethyl vinyl ether was added at zero degrees and stirred for 1 h for quenching. The reaction solution was filtered and concentrated to remove the solvent, to obtain a colorless oily olefin cis-trans mixture 10-8a&b (150 mg, yield: 94%). LCMS (M+H)⁺ = 535.4.

### Step 6

The compound 10-8a&b (150 mg, 0.28 mmol) was dissolved in ethanol (20 mL), PtO₂ (18 mg, 0.08 mmol) was added, and hydrogen substitution was carried out three times, to react at room temperature for 0.5 h. The reaction solution was filtered and concentrated to remove the solvent, to obtain a colorless oily compound 10-9 (120 mg, yield: 80%). LCMS (M+H)⁺ = 537.3.

### Step 7

The compound 10-9 (120 mg, 0.22 mmol) was dissolved in 7M ammonia in methanol (20 mL), to react in a sealed tube at room temperature for 24 h. The reaction solution was concentrated to remove the solvent, to obtain a colorless oily compound 10-10 (80 mg, yield: 70%). LCMS (M+H)⁺ = 522.4.

### Step 8

The compound 10-10 (40 mg, 0.08 mmol) was dissolved in DCM (5 mL), and added dropwise in TFA (0.5 mL) in an ice-water bath. The reaction solution reacted at zero degrees for 3 h. The reaction solution was concentrated to remove the solvent, to obtain a colorless oily compound 10-11 (25 mg, yield: 77%). LCMS (M+H)⁺ = 422.3.

### Step 9

The compound 10-11 (25 mg, 0.06 mmol) was dissolved in THF (10 mL), and added dropwise in pyridine (16 mg, 0.2 mmol) in an ice-water bath. TFAA (13 mg, 0.06 mmol) was added dropwise after stirring in the ice-water bath for 15 min. The reaction solution reacted at room temperature overnight. The reaction was quenched with water at zero degrees, extracted with ethyl acetate, dried with saturated brine, dried with anhydrous sodium sulfate, and concentrated to remove the solvent, to obtain a crude product. The crude product was subjected to Prep-HPLC to obtain a white solid compound 10 (8.0 mg, yield: 27%).

LCMS (M+H)⁺ = 500.1. 1H NMR (400 MHz, DMSO) δ 9.69 (d, J = 6.6 Hz, 1H), 9.12 (d, J = 7.9 Hz, 1H), 7.75 (s, 1H), 4.91 (d, J = 5.5 Hz, 1H), 4.52 (d, J = 6.7 Hz, 1H), 4.13 (d, J = 3.6 Hz, 1H), 3.82 (dd, J = 10.3, 7.9 Hz, 1H), 3.62 (dd, J = 10.5, 3.7 Hz, 1H), 2.73 (d, J = 7.6 Hz, 2H), 2.53 (s, 2H), 2.36-2.20 (m, 2H), 2.08-1.98 (m, 1H), 1.91-1.70 (m, 4H), 1.68-1.47 (m, 4H), 1.29 (ddd, J = 27.6, 12.5, 6.2 Hz, 9H).

### Examples of Activity Tests

In the following examples, the inhibitory activity against M^{pro} and pharmacokinetic properties of the compounds of the present disclosure are detected by using some of the compounds of the present disclosure as examples.

### Example A: Inhibitory Activity against M^{pro}

An objective of this experiment is to detect the in vitro inhibitory activity against M^{pro} of the compound of the present disclosure.

### Experimental materials:

GST-AVLQ-3Clpro-GP-6His (CP, Lot. No. CP20200526001);
MCA-AVLQSGFR-Lys(Dnp)-Lys-NH₂ (GL, Customized); and
384-well plate (Perkin Elmer, Cat. No. 6007279).

### Experimental steps and methods:

1. Prepare a 1x buffer solution (modified Tris buffer solution).
2. Transfer a to-be-tested compound to the assay plate with Echo's 100% DMSO solution to a final DMSO content of 0.5%.
3. Prepare a mixed protein solution: Add GST-AVLQ-3Clpro-GP-6His in a 1x assay buffer solution to support the mixed protein solution.
4. Prepare a mixed substrate solution: Add the polypeptide (MCA-AVLQSGFR-Lys(Dnp)-Lys-NH₂) in a 1x assay buffer solution to obtain the substrate solution.
5. Add 10 µL of 2× mixed protein solution in the assay plate, centrifuge for 30s, shake for 30s, and incubate the solution at room temperature for 15 min.
6. Add 10 µL of 2x mixed substrate solution in the assay plate, centrifuge for 30, and shake for 30s.
7. Read the data dynamically on Synergy (Ex320/Em405).

Table 1 shows experimental data of the inhibitory activity against M^{pro} of some of the compounds of the present disclosure.

**Table 1:**

| Compound No. | M^{pro} IC₅₀ (nM) |
|---|---|
| **1** | 254 |
| **4** | 411 |
| **6** | 379 |
| **7** | 16 |
| **8b** | 138 |

The experimental results indicate that the compound of the present disclosure has good inhibitory activity against M^{pro}.

### Example B: Pharmacokinetic Evaluation of the Compound of The Present Disclosure in Mice by Intravenous Injection or Oral Administration

An objective of this experiment is to detect the pharmacokinetic properties of the compound of the present disclosure in mice.

Experimental steps and methods are shown in the following table:

| **Category** | **Item** | **Description** |
|---|---|---|
| Animal species | CD-1 mice | Male, N = 12 for each compound, fasted overnight prior to oral administration, fed 4 hours after administration. |
| Administered formulation | IV, PO | IV&PO: provided by the client |
| Animal experiment | Group 1: IV, XX mg/kg, XX mL/kg, N = 3/time point | The dose for administration is provided by Party A. A whole blood sample (whole blood is centrifuged within 30 minutes to separate plasma) is collected |

| | | |
|---|---|---|
| | Group 2: PO, XX mg/kg, XX mL/kg, N = 3/time point | by the semi-continuous method in a tube containing the anticoagulant EDTA-K2. Design of sampling time points: A total of six time points (no overtime sampling). A total of 36 plasma samples are collected for the two groups. |
| Bioanalysis | Analytical method development | Develop an LC-MS/MS method for the determination of the concentration of a to-be-determined substance in the plasma samples. |
| | Sample testing | The 36 plasma samples are tested for one to-be-determined substance. For sample quantity <_ 12, one standard curve is used for quantification; and for sample quantity > 12, two standard curves are used for quantification and accompanied by a proper quantity of QC samples. The accuracy of at least five concentrations of each standard curve is 80%-120% of the true concentration (the accuracy of LLOQ has to be 75%-125%). |
| Data processing and reporting | | Calculate the PK parameters by using WinNonlin, and provide an English data report in Excel |

The experimental results indicate that the compound of the present disclosure has good metabolic stability in the animals to be tested and has significant advantages.

In the description of this specification, the term "an embodiment", "some embodiments", "example", "specific example", or "some examples" is intended to indicate that specific features, structures, materials, or characteristics described in conjunction with the embodiment or example are included in at least one embodiment or example of the present disclosure. In this specification, schematic expressions of the foregoing terms do not necessarily refer to the same embodiment or example. In addition, the specific features, structures, materials, or characteristics described may be combined in any one or more embodiments or examples in a suitable manner. Moreover, without contradicting each other, a person skilled in the art may combine different embodiments or examples and features of different embodiments or examples described in this specification.

Although the embodiments of the present disclosure have been shown and described above, it may be understood that the foregoing embodiments are exemplary and are not to be construed as a limitation on the present disclosure. A person of ordinary skill in the art may make changes, modifications, substitutions, and variations of the foregoing embodiments within the scope of the present disclosure.

## Claims

1. A compound, being a compound shown in formula (I) or a stereoisomer, a tautomer, a nitrogen oxide, a solvate, a metabolite, a pharmaceutically acceptable salt, or a prodrug of the compound shown in formula (I), 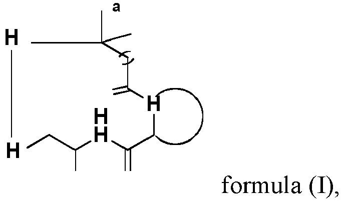
L₁ being a linker;
L₂ being -(CH₂)ₘCONR₁- or 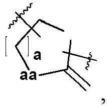 Y being C or N;
R₁ being H, F, Cl, Br, CN, NO₂, -OR^{b}, -NR^{c}R^{d}, or C₁₋₆ alkyl;
R₂ₐ and R_{2b} being each independently H, -C(=O)R^{a}, -C(=O)OR^{b}, -S(=O)₂R^{b}, - C(=O)NR^{c}R^{d}, -NC(=O)R^{c}R^{d}, -OR^{b}, -NR^{c}R^{d}, R^{b}O-C₁₋₄ alkylene, R^{d}R^{c}N-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene, C₁₋₆ alkyl, C₂₋₆ alkenyl, C₂₋₆ alkynyl, C₃₋₈ cycloalkyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, and (heteroaryl consisting of 5-10 atoms)-C₁₋₄ alkylene being each independently unsubstituted or substituted with one, two, three, or four substituents, the substituent being independently F, Cl, Br, CN, =O, -OR^{b}, -NR^{c}R^{d}, C₁₋₆ alkyl, C₁₋₆ haloalkyl, R^{b}O-C₁₋₄ alkylene, or R^{d}R^{c}N-C₁₋₄ alkylene, and R₂ₐ and R_{2b} being not all H;
ring A being optionally heterocyclyl consisting of 3-10 atoms or heteroaryl consisting of 5-10 atoms that is substituted with one or more R';
Z being cyano, aziridine, epoxypropane, trifluoromethyl ketone, aldehyde, - CH(O(C=O)R₃)SO₃Na, -CH(O(C=O)OR₃)SO₃Na, -CH(O(C=O)NHR₃)SO₃Na, - (C=O)(C=O)NHR₃, -(C=O)(P=O)(OR₃), -(C=O)COOR₃, acetoxymethyl ketone, Michael receptor, or substituted or unsubstituted heterocyclyl consisting of 3-6 atoms or heteroaryl consisting of 5-10 atoms, R₃ being H, F, Cl, Br, CN, NO₂, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, C₃₋₈ cycloalkyl-C₁₋₄ alkylene, heterocyclyl consisting of 3-12 atoms, (heterocyclyl consisting of 3-12 atoms)-C₁₋₄ alkylene, C₆₋₁₀ aryl, C₆₋₁₀ aryl-C₁₋₄ alkylene, heteroaryl consisting of 5-10 atoms, or (heteroaryl consisting of 5-10 atoms)-C₁₋₄ alkylene;
m being an integer between 1 and 6; n being 1 or 2; k being 0 or 1;
R' being independently H, F, Cl, Br, CN, NO₂, =O, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, heterocyclyl consisting of 3-12 atoms, C₆₋₁₀ aryl, or heteroaryl consisting of 5-10 atoms, C₃₋₈ cycloalkyl, heterocyclyl consisting of 3-12 atoms, C₆₋₁₀ aryl, or heteroaryl consisting of 5-10 atoms being independently unsubstituted or substituted with one, two, three, or four substituents, and the substituent being independently F, Cl, Br, CN, =O, -OR^{b}, -NR^{c}R^{d}, C₁₋₆ alkyl, C₁₋₆ haloalkyl, R^{b}O-C₁₋₄ alkylene, or R^{d}R^{c}N-C₁₋₄ alkylene; and
R^{a}, R^{b}, R^{c}, and R^{d} being each independently H, C₁₋₆ alkyl, C₁₋₆ haloalkyl, heterocyclyl consisting of 3-6 atoms, C₆₋₁₀ aryl, heteroaryl consisting of 5-10 atoms, or heterocycle consisting of 3-6 atoms that is formed by R^{c}, R^{d}, and nitrogen atoms linked to R^{c} and R^{d}, C₁₋₆ alkyl, heterocycle consisting of 3-6 atoms, C₆₋₁₀ aryl, and heteroaryl consisting of 5-10 atoms being each independently unsubstituted or substituted with one, two, three, or four substituents, and the substituent being independently F, Cl, CN, OH, NH₂, C₁₋₆ alkyl, C₁₋₆ haloalkyl, C₁₋₆ alkoxy, or C₁₋₆ alkylamino.

2. The compound according to claim 1, wherein the compound is a compound shown in formula (II) or formula (III) or a stereoisomer, a tautomer, a nitrogen oxide, a solvate, a metabolite, a pharmaceutically acceptable salt, or a prodrug of the compound shown in formula (II) or formula (III),

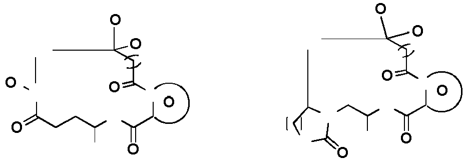

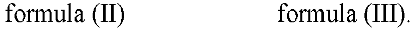

3. The compound according to claim 1, wherein the compound is a compound shown in formula (IV) or formula (V) or a stereoisomer, a tautomer, a nitrogen oxide, a solvate, a metabolite, a pharmaceutically acceptable salt, or a prodrug of the compound shown in formula (IV) or formula (V),

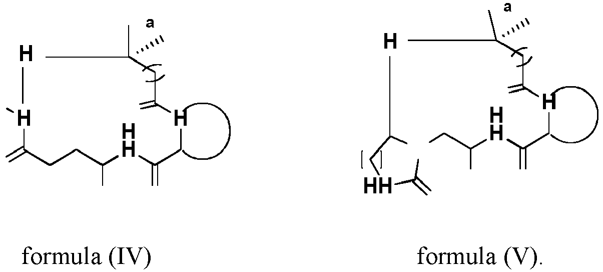

4. The compound according to claim 1, wherein the compound is a compound shown in formula (VI) or formula (VII) or a stereoisomer, a tautomer, a nitrogen oxide, a solvate, a metabolite, a pharmaceutically acceptable salt, or a prodrug of the compound shown in formula (VI) or formula (VII),

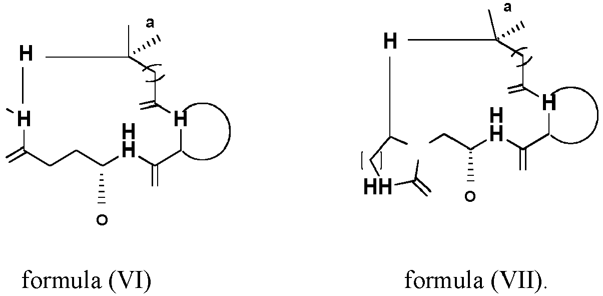

5. The compound according to claim 1, wherein the compound is a compound shown in formula (VIII) or formula (IX) or a stereoisomer, a tautomer, a nitrogen oxide, a solvate, a metabolite, a pharmaceutically acceptable salt, or a prodrug of the compound shown in formula (VIII) or formula (IX),

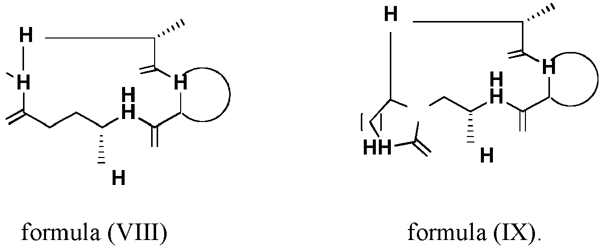

6. The compound according to claim 1, wherein the compound is a compound shown in formula (X) or formula (XI) or a stereoisomer, a tautomer, a nitrogen oxide, a solvate, a metabolite, a pharmaceutically acceptable salt, or a prodrug of the compound shown in formula (X) or formula (XI),

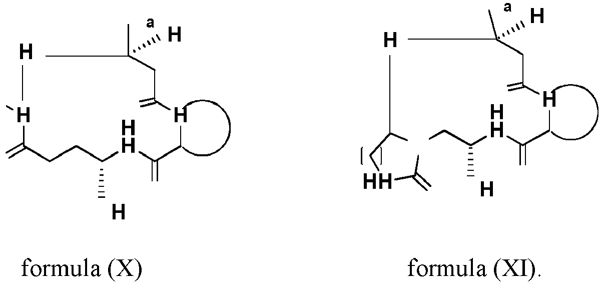

7. The compound according to any one of claims 1 to 6, wherein L₁ is a chain linker, and an atomic number of a main chain of the chain linker is 5, 6, 7, 8, 9, or 10, preferably, the atomic number of the main chain of the chain linker is 6.

8. The compound according to any one of claims 1 to 6, wherein L₁ is optionally substituted -Rₑ-, -RₑCH(OH)R_{f}-, -RₑC(=O)R_{f}-, -RₑC(=O)OR_{f}-, -RₑOC(=O)R_{f}-, -RₑC(=O)SR_{f}-, -RₑSC(=O)R_{f}-, -RₑC(=O)N(R_{g})R_{f}-, -RₑN(R_{g})C(=O)R_{f}-, -RₑS(=O)₂N(R_{g})R_{f}-, - RₑN(R_{g})S(=O)₂R_{f}-, -RₑN(R_{g})C(=O)OR_{f}-, -RₑOC(=O)N(R_{g})R_{f}-, -RₑOR_{f}-, -RₑNR_{f}-, -RₑN=R_{f}-, - Rₑ-O-O-R_{f}-, -RₑSR_{f}-, -Rₑ-S-S-R_{f}-, -Rₑ-S-S-S-R_{f}-, -Rₑ-Rₕ-R_{f}-, or -Rₑ-O-Rₕ-R_{f}-, R_{g} being -H or substituted or unsubstituted alkyl, Rₑ being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rₕ being C₃₋₈ cycloalkyl, heterocyclyl consisting of 3-12 atoms, C₆₋₁₀ aryl, or heteroaryl consisting of 5-10 atoms, and t1 and t2 being independently integers not less than 1.

9. The compound according to any one of claims 1 to 6, wherein L₁ is optionally substituted -Rₑ-, -RₑS(=O)₂N(R_{g})R_{f}-, -RₑN(R_{g})S(=O)₂R_{f}-, -RₑOR_{f}-, -RₑNR_{f}-, -RₑN=R_{f}-, -Rₑ-Rₕ-R_{f}-, or -Rₑ-O-Rₕ-R_{f}-, R_{g} being -H or substituted or unsubstituted alkyl, Rₑ being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, R_{f} being -**H**H -, C₃-C₃₀ branched alkylene, C₂-C₃₀ straight-chain alkenylene, C₃-C₃₀ branched alkenylene, C₂-C₃₀ straight-chain alkynylene, or C₃-C₃₀ branched alkynylene or bond, Rₕ being C₃₋₈ cycloalkyl, heterocyclyl consisting of 3-12 atoms, C₆₋₁₀ aryl, or heteroaryl consisting of 5-10 atoms, and t1 and t2 being independently integers not less than 1.

10. The compound according to claim 8, wherein the substituent is F, Cl, Br, CN, NO₂, =O, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, heterocyclyl consisting of 3-12 atoms, C₆₋₁₀ aryl, or heteroaryl consisting of 5-10 atoms.

11. The compound according to claim 9, wherein the substituent is F, Cl, Br, CN, NO₂, =O, -OR^{b}, -NR^{c}R^{d}, -S(=O)₂R^{b}, C₁₋₆ alkyl, C₁₋₆ haloalkyl, C₃₋₈ cycloalkyl, C₃₋₈ cycloalkenyl, heterocyclyl consisting of 3-12 atoms, C₆₋₁₀ aryl, or heteroaryl consisting of 5-10 atoms.

12. The compound according to any one of claims 1 to 4, wherein R₂ₐ and R_{2b} are each independently H, -OR^{b}, or -NC(=O)R^{c}R^{d}, and R₂ₐ and R_{2b} are not all H.

13. The compound according to any one of claims 1 to 6, wherein the ring A is optionally heterocyclyl consisting of 5-10 atoms that is substituted with one or more R'.

14. The compound according to any one of claims 1 to 4, wherein Z is cyano.

15. The compound according to claim 1, wherein m is 1.

16. The compound according to any one of claims 1 to 6, wherein R' is independently F, Cl, Br, C₁₋₆ alkyl, or C₁₋₆ haloalkyl.

17. The compound according to claim 13, wherein R' is independently F, Cl, Br, C₁₋₆ alkyl, or C₁₋₆ haloalkyl.

18. The compound according to claim 1, wherein the compound is of the following structures or is a stereoisomer, a tautomer, a nitrogen oxide, a solvate, a metabolite, a pharmaceutically acceptable salt, or a prodrug of the following structures,

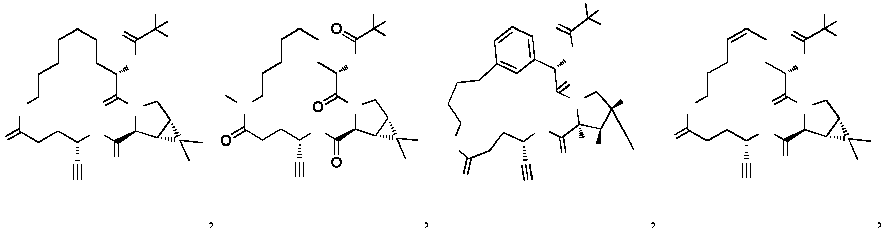

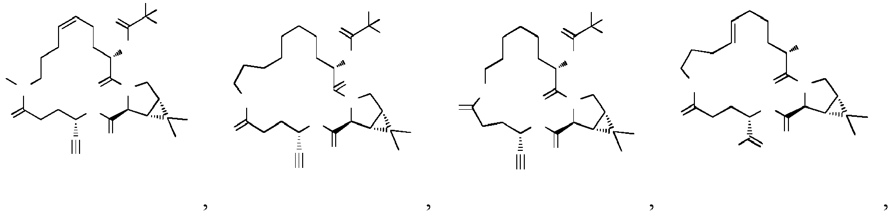

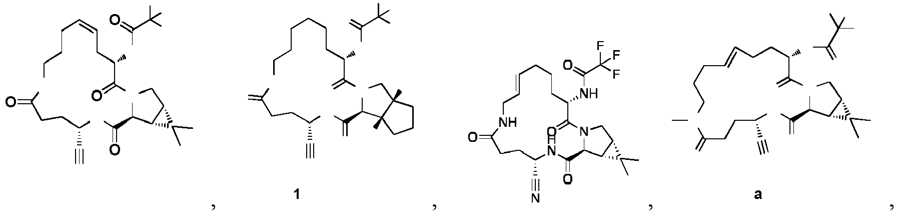

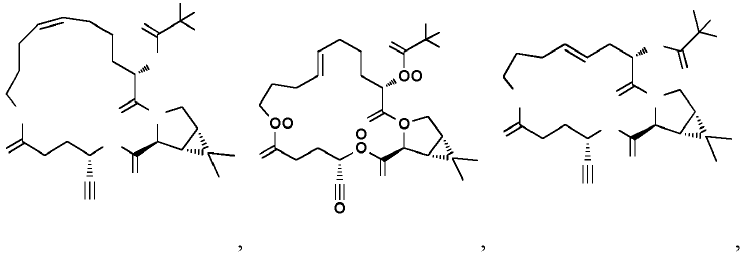

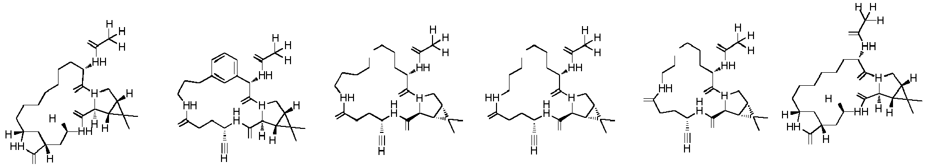

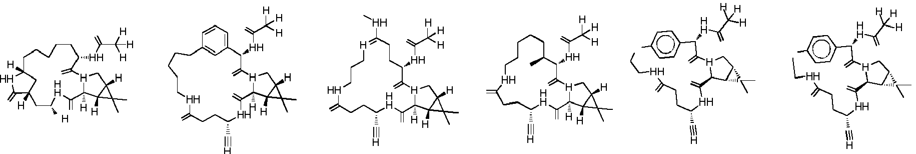

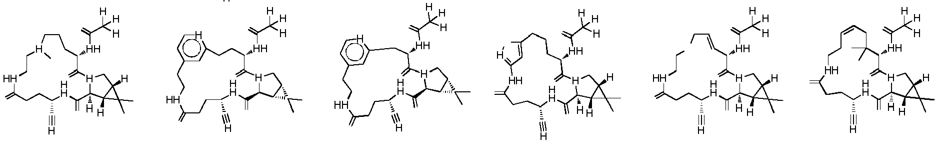

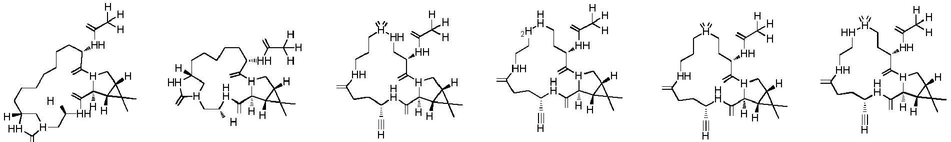

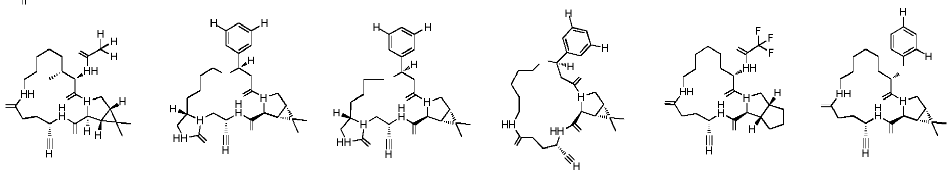

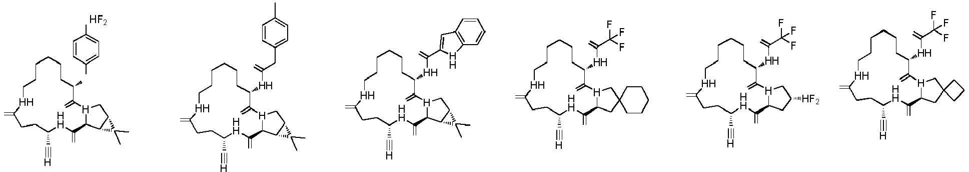

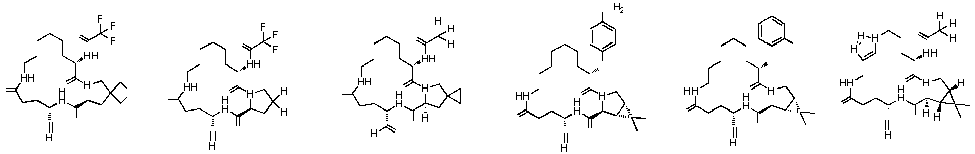

19. A pharmaceutical composition, comprising an effective amount of the compound according to any one of claims 1 to 18.

20. The pharmaceutical composition according to claim 19, further comprising a pharmaceutically acceptable carrier, an adjuvant, a medium, or a combination thereof.

21. The pharmaceutical composition according to claim 19, further comprising one or more therapeutic agents, the therapeutic agent being at least one of other drugs against coronaviruses and drugs suppressing cytokine storms.

22. Use of the compound according to any one of claims 1 to 18 or the pharmaceutical composition according to any one of claims 19 to 21 in preparation of drugs, the drugs being used for preventing coronavirus infection or treating or reducing associated diseases of patients caused by coronavirus infection.

23. The use according to claim 22, wherein the coronavirus comprises at least one of HCoV-229E, HCoV-OC43, HCoV-NL63, HCoV-HKU1, SARS-CoV, 2019-nCoV, and MERS-Cov.

24. Use of the compound according to any one of claims 1 to 18 or the pharmaceutical composition according to any one of claims 19 to 21 in preparation of drugs, the drugs being used for inhibiting M^{pro} or inhibiting coronavirus replication.

25. The compound according to any one of claims 1 to 18 or the pharmaceutical composition according to any one of claims 19 to 21, for preventing coronavirus infection or treating or reducing associated diseases of patients caused by coronavirus infection.

26. The compound according to any one of claims 1 to 18 or the pharmaceutical composition according to any one of claims 19 to 21, for preventing coronavirus infection or treating or reducing associated diseases of patients caused by coronavirus infection, wherein the coronavirus comprises at least one of HCoV-229E, HCoV-OC43, HCoV-NL63, HCoV-HKU1, SARS-CoV, 2019-nCoV, and MERS-Cov.

27. The compound according to any one of claims 1 to 18 or the pharmaceutical composition according to any one of claims 19 to 21, for inhibiting M^{pro} or inhibiting coronavirus replication.

28. A method for preventing coronavirus infection or treating or reducing associated diseases of patients caused by coronavirus infection, comprising administering to a subject a pharmaceutically acceptable dose of the compound according to any one of claims 1 to 18 or the pharmaceutical composition according to any one of claims 19 to 21.

29. The method according to claim 28, wherein the coronavirus comprises at least one of HCoV-229E, HCoV-OC43, HCoV-NL63, HCoV-HKU1, SARS-CoV, 2019-nCoV, and MERS-Cov.

30. A method for inhibiting M^{pro} or inhibiting coronavirus replication, comprising contacting M^{pro} or the coronavirus with the compound according to any one of claims 1 to 18 or the pharmaceutical composition according to any one of claims 19 to 21.
